# EUROPEAN PATENT SPECIFICATION

(11) **EP 2 402 033 B1**
(45) Date of publication and mention of the grant of the patent: **27.02.2019**
(21) Application number: 10746047.9
(22) Date of filing: 28.01.2010
(51) Int. Cl.: A61K 39/395, A61K 31/7088, A61K 45/00, A61K 48/00, A61P 35/00, A61P 35/02, A61P 43/00, G01N 33/15, G01N 33/50, C07K 16/28, C12N 15/113

(54) **CELL ADHESION INHIBITOR AND USE THEREOF**
ZELLADHÄSIONSHEMMER UND SEINE VERWENDUNG
INHIBITEUR D'ADHÉRENCE CELLULAIRE ET SON UTILISATION

(30) Priority: 24.02.2009 JP 2009041204
(43) Date of publication of application: 04.01.2012
(73) Proprietor: University of Miyazaki, Miyazaki-shi Miyazaki 889-2192 (JP)
(72) Inventor: MORISHITA, Kazuhiro, Miyazaki-gun Miyazaki 889-1692 (JP); YAMAKAWA, Norio, Miyazaki-gun Miyazaki 889-1692 (JP)
(74) Representative: Lee, Nicholas John
(86) International application number: PCT/JP2010/051109
(87) International publication number: WO 2010/098166

(56) References cited:
- WO-A1-2008/127655
- JP-A- 5 308 994
- US-B1- 6 869 795
- QIUXIA HE ET AL: "Knockdown of integrin [beta]4-induced autophagic cell death associated with P53 in A549 lung adenocarcinoma cells", FEBS JOURNAL, vol. 275, no. 22, 1 November 2008 (2008-11-01), pages 5725-5732, XP055047197, ISSN: 1742-464X, DOI: 10.1111/j.1742-4658.2008.06699.x
- B. YATSULA: "Identification of Binding Sites of EVI1 in Mammalian Cells", JOURNAL OF BIOLOGICAL CHEMISTRY, vol. 280, no. 35, 1 January 2005 (2005-01-01), pages 30712-30722, XP055047373, ISSN: 0021-9258, DOI: 10.1074/jbc.M504293200
- LIU Y. ET AL.: 'Evi1 is a survival factor which conveys resistance to both TGFbeta- and taxol- mediated cell death via PI3K/AKT' ONCOGENE vol. 25, no. 25, 2006, pages 3565 - 3575, XP055003224
- SUSUMU GOYAMA ET AL.: 'Evi-1 Is a Critical Regulator for Hematopoietic Stem Cells and Transformed Leukemic Cells' CELL STEM CELL vol. 3, 2008, pages 207 - 220, XP055046913
- V. SUNG ET AL.: '1,25-Dihydroxyvitamin D3 decreases human prostate cancer cell adhesion and migration' MOLECULAR AND CELLULAR ENDOCRINOLOGY vol. 164, no. 1-2, 2000, pages 133 - 143, XP055047195
- ZOBEIDA C. M. ET AL.: 'Integrin a6p4 Promotes Migration, Invasion through Tiaml Upregulation, and Subsequent Rac Activation' NEOPLASIA vol. 10, no. 5, 2008, pages 408 - 417, XP003031443
- T. OTA ET AL.: 'Functional suppression of integrin beta4-mediated adhesion caused by in vivo sequential selection for cancer cell intravasation' ANTICANCER RESEARCH vol. 21, no. 1A, 2001, pages 205 - 211, XP009165568
- KOBUNE M. ET AL.: 'Wnt3/RhoA/ROCK signaling pathway is involved in adhesion-mediated drug resistance of multiple myeloma in an autocrine mechanism' MOLECULAR CANCER THERAPEUTICS vol. 6, no. 6, 2007, pages 1774 - 1784, XP055067760
- V. M. FREITAS ET AL.: 'SIKVAV, a Laminin al- Derived Peptide, Interacts with Integrins and Increases Protease Activity of a Human Salivary Gland Adenoid Cystic Carcinoma Cell Line through the ERK 1/2 Signaling Pathway' THE AMERICAN JOURNAL OF PATHOLOGY vol. 171, no. 1, 2007, pages 124 - 138, XP003031444
- AMY P.N. SKUBITZ ET AL.: 'FOUR NEW CD104(p4 INTEGRIN) MABS THAT INHIBIT CELL ADHESION TO LAMININ' TISSUE ANTIGENS vol. 48, no. 4, 1996, page 356, XP003031445
- QIUXIA HE ET AL.: 'Knockdown of integrin beta4- induced autophagic cell death associated with P53 in A549 lung adenocarcinoma cells' FEBS JOURNAL vol. 275, 2008, pages 5725 - 5732, XP055047197
- X WAN ET AL.: 'Beta4 integrin promotes osteosarcoma metastasis and interacts with ezrin' ONCOGENE vol. 28, no. 38, 2009, pages 3401 - 3411, XP055067766
- JIN LIQING ET AL: "Targeting of CD44 eradicates human acute myeloid leukemic stem cells", NATURE MEDICINE, NATURE PUB. CO, vol. 12, no. 10, 1 October 2006 (2006-10-01), pages 1167-1174, XP002457833, ISSN: 1078-8956, DOI: 10.1038/NM1483
- Massimiliano Cariati ET AL: "Alpha-6 integrin is necessary for the tumourigenicity of a stem cell-like subpopulation within the MCF7 breast cancer cell line", International Journal of Cancer, vol. 122, no. 2, 12 October 2007 (2007-10-12), pages 298-304, XP055433885, US ISSN: 0020-7136, DOI: 10.1002/ijc.23103

### SEQUENCE LISTING

<110> University of Miyazaki
<120> INHIBITOR OF CELL ADHESION AND USE THEREOF
<130> F09-039-PCT
<160> 14
<170> PatentIn version 3.1
<210> 1
   <211> 4873
   <212> DNA
   <213> Homo sapiens
<400> 1
   caacatcgtg tgctgcttcg cgagaaagtc acattcggac cctttggcta gattgcttat 60
   tcatagggct tcttgactaa agcccttgga gcactgggtt tttcttgaag tatatgatct 120
   tagacgaatt ttacaatgtg aagttctgca tagatgccag tcaaccagat gttggaagct 180
   ggctcaagta cattagattc gctggctgtt atgatcagca caaccttgtt gcatgccaga 240
   taaatgatca gatattctat agagtagttg cagacattgc gccgggagag gagcttctgc 300
   tgttcatgaa gagcgaagac tatccccatg aaactatggc gccggatatc cacgaagaac 360
   ggcaatatcg ctgcgaagac tgtgaccagc tctttgaatc taaggctgaa ctagcagatc 420
   accaaaagtt tccatgcagt actcctcact cagcattttc aatggttgaa gaggactttc 480
   agcaaaaact cgaaagcgag aatgatctcc aagagataca cacgatccag gagtgtaagg 540
   aatgtgacca agtttttcct gatttgcaaa gcctggagaa acacatgctg tcacatactg 600
   aagagaggga atacaagtgt gatcagtgtc ccaaggcatt taactggaag tccaatttaa 660
   ttcgccacca gatgtcacat gacagtggaa agcactatga atgtgaaaac tgtgccaagg 720
   ttttcacgga ccctagcaac cttcagcggc acattcgctc tcagcatgtc ggtgcccggg 780
   cccatgcatg cccggagtgt ggcaaaacgt ttgccacttc gtcgggcctc aaacaacaca 840
   agcacatcca cagcagtgtg aagcccttta tctgtgaggt ctgccataaa tcctatactc 900
   agttttcaaa cctttgccgt cataagcgca tgcatgctga ttgcagaacc caaatcaagt 960
   gcaaagactg tggacaaatg ttcagcacta cgtcttcctt aaataaacac aggaggtttt 1020
   gtgagggcaa gaaccatttt gcggcaggtg gattttttgg ccaaggcatt tcacttcctg 1080
   gaaccccagc tatggataaa acgtccatgg ttaatatgag tcatgccaac ccgggccttg 1140
   ctgactattt tggcgccaat aggcatcctg ctggtcttac ctttccaaca gctcctggat 1200
   tttcttttag cttccctggt ctgtttcctt ccggcttgta ccacaggcct cctttgatac 1260
   ctgctagttc tcctgttaaa ggactatcaa gtactgaaca gacaaacaaa agtcaaagtc 1320
   ccctcatgac acatcctcag atactgccag ctacacagga tattttgaag gcactatcta 1380
   aacacccatc tgtaggggac aataagccag tggagctcca gcccgagagg tcctctgaag 1440
   agaggccctt tgagaaaatc agtgaccagt cagagagtag tgaccttgat gatgtcagta 1500
   caccaagtgg cagtgacctg gaaacaacct cgggctctga tctggaaagt gacattgaaa 1560
   gtgataaaga gaaatttaaa gaaaatggta aaatgttcaa agacaaagta agccctcttc 1620
   agaatctggc ttcaataaat aataagaaag aatacagcaa tcattccatt ttctcaccat 1680
   ctttagagga gcagactgcg gtgtcaggag ctgtgaatga ttctataaag gctattgctt 1740
   ctattgctga aaaatacttt ggttcaacag gactggtggg gctgcaagac aaaaaagttg 1800
   gagctttacc ttacccttcc atgtttcccc tcccattttt tccagcattc tctcaatcaa 1860
   tgtacccatt tcctgataga gacttgagat cgttaccttt gaaaatggaa ccccaatcac 1920
   caggtgaagt aaagaaactg cagaagggca gctctgagtc cccctttgat ctcaccacta 1980
   agcgaaagga tgagaagccc ttgactccag tcccctccaa gcctccagtg acacctgcca 2040
   caagccaaga ccagcccctg gatctaagta tgggcagtag gagtagagcc agtgggacaa 2100
   agctgactga gcctcgaaaa aaccacgtgt ttgggggaaa aaaaggaagc aacgtcgaat 2160
   caagacctgc ttcagatggt tccttgcagc atgcaagacc cactcctttc tttatggacc 2220
   ctatttacag agtagagaaa agaaaactaa ctgacccact tgaagcttta aaagagaaat 2280
   acttgaggcc ttctccagga ttcttgtttc acccacaatt ccaactgcct gatcagagaa 2340
   cttggatgtc agctattgaa aacatggcag aaaagctaga gagcttcagt gccctgaaac 2400
   ctgaggccag tgagctctta cagtcagtgc cctctatgtt caacttcagg gcgcctccca 2460
   atgccctgcc agagaacctt ctgcggaagg gaaaggagcg ctatacctgc agatactgtg 2520
   gcaagatttt tccaaggtct gcaaacctaa cacggcactt gagaacccac acaggagagc 2580
   agccttacag atgcaaatac tgtgacagat catttagcat atcttctaac ttgcaaaggc 2640
   atgttcgcaa catccacaat aaagagaagc catttaagtg tcacttatgt gataggtgtt 2700
   ttggtcaaca aaccaattta gacagacacc taaagaaaca tgagaatggg aacatgtccg 2760
   gtacagcaac atcgtcgcct cattctgaac tggaaagtac aggtgcgatt ctggatgaca 2820
   aagaagatgc ttacttcaca gaaattcgaa atttcattgg gaacagcaac catggcagcc 2880
   aatctcccag gaatgtggag gagagaatga atggcagtca ttttaaagat gaaaaggctt 2940
   tggtgaccag tcaaaattca gacttgctgg atgatgaaga agttgaagat gaggtgttgt 3000
   tagatgagga ggatgaagac aatgatatta ctggaaaaac aggaaaggaa ccagtgacaa 3060
   gtaatttaca tgaaggaaac cctgaggatg actatgaaga aaccagtgcc ctggagatga 3120
   gttgcaagac atccccagtg aggtataaag aggaagaata taaaagtgga ctttctgctc 3180
   tagatcatat aaggcacttc acagatagcc tcaaaatgag gaaaatggaa gataatcaat 3240
   attctgaagc tgagctgtct tcttttagta cttcccatgt gccagaggaa cttaagcagc 3300
   cgttacacag aaagtccaaa tcgcaggcat atgctatgat gctgtcactg tctgacaagg 3360
   agtccctcca ttctacatcc cacagttctt ccaacgtgtg gcacagtatg gccagggctg 3420
   cggcggaatc cagtgctatc cagtccataa gccacgtatg acgttatcaa ggttgaccag 3480
   agtgggacca agtccaacag tagcatggct ctttcatata ggactattta caagactgct 3540
   gagcagaatg ccttataaac ctgcagggtc actcatctaa agtctagtga ccttaaactg 3600
   aatgatttaa aaaagaaaag aaagaaaaaa gaaactattt attctcgata ttttgttttg 3660
   cacagcaaag gcagctgctg acttctggaa gatcaatcaa tgcgacttaa agtgattcag 3720
   tgaaaacaaa aaacttggtg ggctgaaggc atcttccagt ttaccccacc ttagggtatg 3780
   ggtgggtgag aagggcagtt gagatggcag cattgatatg aatgaacact ccatagaaac 3840
   tgaattctct tttgtacaag atcacctgac atgattggga acagttgctt ttaattacag 3900
   atttaatttt tttcttcgtt aaagttttat gtaatttaac cctttgaaga cagaagtagt 3960
   tggatgaaat gcacagtcaa ttattataga aactgataac agggagtact tgttccccct 4020
   tttgccttct taagtacatt gtttaaaact agggaaaaag ggtatgtgta tattgtaaac 4080
   tatggatgtt aacactcaaa gaggttaagt cagtgaagta acctattcat caccagtacc 4140
   gctgtaccac taataaattg tttgccaaat ccttgtaata acatcttaat tttagacaat 4200
   catgtcactg tttttaatgt ttattttttt gtgtgtgttg cgtgtatcat gtatttattt 4260
   gttggcaaac tattgtttgt tgattaaaat agcactgttc cagtcagcca ctactttatg 4320
   acgtctgagg cacacccctt tccgaatttc aaggaccaag gtgacccgac ctgtgtatga 4380
   gagtgccaaa tggtgtttgg cttttcttaa cattcctttt tgtttgtttg ttttgttttc 4440
   cttcttaatg aactaaatac gaatagatgc aacttagttt ttgtaatact gaaatcgatt 4500
   caattgtata aacgattata atttctttca tggaagcatg attcttctga ttaaaaactg 4560
   tactccatat tttatgctgg ttgtctgcaa gcttgtgcga tgttatgttc atgttaatcc 4620
   tatttgtaaa atgaagtgtt cccaacctta tgttaaaaga gagaagtaaa taacagactg 4680
   tattcagtta ttttgccctt tattgaggaa ccagatttgt tttctttttg tttgtaatct 4740
   cattttgaaa taatcagcaa gttgaggtac tttcttcaaa tgctttgtac aatataaact 4800
   gttatgcctt tcagtgcatt actatgggag gagcaactaa aaaataaaga cttacaaaaa 4860
   ggagtatttt taa 4873
<210> 2
   <211> 1115
   <212> PRT
   <213> Homo sapiens
<400> 2
   Met Ile Leu Asp Glu Phe Tyr Asn Val Lys Phe Cys Ile Asp Ala Ser
   1               5                   10                  15
   Gln Pro Asp Val Gly Ser Trp Leu Lys Tyr Ile Arg Phe Ala Gly Cys
               20                  25                  30
   Tyr Asp Gln His Asn Leu Val Ala Cys Gln Ile Asn Asp Gln Ile Phe
           35                  40                  45
   Tyr Arg Val Val Ala Asp Ile Ala Pro Gly Glu Glu Leu Leu Leu Phe
       50                  55                  60
   Met Lys Ser Glu Asp Tyr Pro His Glu Thr Met Ala Pro Asp Ile His
   65                  70                  75                  80
   Glu Glu Arg Gln Tyr Arg Cys Glu Asp Cys Asp Gln Leu Phe Glu Ser
                   85                  90                  95
   Lys Ala Glu Leu Ala Asp His Gln Lys Phe Pro Cys Ser Thr Pro His
               100                 105                 110
   Ser Ala Phe Ser Met Val Glu Glu Asp Phe Gln Gln Lys Leu Glu Ser
           115                 120                 125
   Glu Asn Asp Leu Gln Glu Ile His Thr Ile Gln Glu Cys Lys Glu Cys
       130                 135                 140
   Asp Gln Val Phe Pro Asp Leu Gln Ser Leu Glu Lys His Met Leu Ser
   145                 150                 155                 160
   His Thr Glu Glu Arg Glu Tyr Lys Cys Asp Gln Cys Pro Lys Ala Phe
                   165                 170                 175
   Asn Trp Lys Ser Asn Leu Ile Arg His Gln Met Ser His Asp Ser Gly
               180                 185                 190
   Lys His Tyr Glu Cys Glu Asn Cys Ala Lys Val Phe Thr Asp Pro Ser
           195                 200                 205
   Asn Leu Gln Arg His Ile Arg Ser Gln His Val Gly Ala Arg Ala His
       210                 215                 220
   Ala Cys Pro Glu Cys Gly Lys Thr Phe Ala Thr Ser Ser Gly Leu Lys
   225                 230                 235                 240
   Gln His Lys His Ile His Ser Ser Val Lys Pro Phe Ile Cys Glu Val
                   245                 250                 255
   Cys His Lys Ser Tyr Thr Gln Phe Ser Asn Leu Cys Arg His Lys Arg
               260                 265                 270
   Met His Ala Asp Cys Arg Thr Gln Ile Lys Cys Lys Asp Cys Gly Gln
           275                 280                 285
   Met Phe Ser Thr Thr Ser Ser Leu Asn Lys His Arg Arg Phe Cys Glu
       290                 295                 300
   Gly Lys Asn His Phe Ala Ala Gly Gly Phe Phe Gly Gln Gly Ile Ser
   305                 310                 315                 320
   Leu Pro Gly Thr Pro Ala Met Asp Lys Thr Ser Met Val Asn Met Ser
                   325                 330                 335
   His Ala Asn Pro Gly Leu Ala Asp Tyr Phe Gly Ala Asn Arg His Pro
               340                 345                 350
   Ala Gly Leu Thr Phe Pro Thr Ala Pro Gly Phe Ser Phe Ser Phe Pro
           355                 360                 365
   Gly Leu Phe Pro Ser Gly Leu Tyr His Arg Pro Pro Leu Ile Pro Ala
       370                 375                 380
   Ser Ser Pro Val Lys Gly Leu Ser Ser Thr Glu Gln Thr Asn Lys Ser
   385                 390                 395                 400
   Gln Ser Pro Leu Met Thr His Pro Gln Ile Leu Pro Ala Thr Gln Asp
                   405                 410                 415
   Ile Leu Lys Ala Leu Ser Lys His Pro Ser Val Gly Asp Asn Lys Pro
               420                 425                 430
   Val Glu Leu Gln Pro Glu Arg Ser Ser Glu Glu Arg Pro Phe Glu Lys
           435                 440                 445
   Ile Ser Asp Gln Ser Glu Ser Ser Asp Leu Asp Asp Val Ser Thr Pro
       450                 455                 460
   Ser Gly Ser Asp Leu Glu Thr Thr Ser Gly Ser Asp Leu Glu Ser Asp
   465                 470                 475                 480
   Ile Glu Ser Asp Lys Glu Lys Phe Lys Glu Asn Gly Lys Met Phe Lys
                   485                 490                 495
   Asp Lys Val Ser Pro Leu Gln Asn Leu Ala Ser Ile Asn Asn Lys Lys
               500                 505                 510
   Glu Tyr Ser Asn His Ser Ile Phe Ser Pro Ser Leu Glu Glu Gln Thr
           515                 520                 525
   Ala Val Ser Gly Ala Val Asn Asp Ser Ile Lys Ala Ile Ala Ser Ile
       530                 535                 540
   Ala Glu Lys Tyr Phe Gly Ser Thr Gly Leu Val Gly Leu Gln Asp Lys
   545 550 555 560
   Lys Val Gly Ala Leu Pro Tyr Pro Ser Met Phe Pro Leu Pro Phe Phe
   565 570 575
   Pro Ala Phe Ser Gln Ser Met Tyr Pro Phe Pro Asp Arg Asp Leu Arg
   580 585 590
   Ser Leu Pro Leu Lys Met Glu Pro Gln Ser Pro Gly Glu Val Lys Lys
   595 600 605
   Leu Gln Lys Gly Ser Ser Glu Ser Pro Phe Asp Leu Thr Thr Lys Arg
   610 615 620
   Lys Asp Glu Lys Pro Leu Thr Pro Val Pro Ser Lys Pro Pro Val Thr
   625 630 635 640
   Pro Ala Thr Ser Gln Asp Gln Pro Leu Asp Leu Ser Met Gly Ser Arg
   645 650 655
   Ser Arg Ala Ser Gly Thr Lys Leu Thr Glu Pro Arg Lys Asn His Val
   660 665 670
   Phe Gly Gly Lys Lys Gly Ser Asn Val Glu Ser Arg Pro Ala Ser Asp
   675 680 685
   Gly Ser Leu Gln His Ala Arg Pro Thr Pro Phe Phe Met Asp Pro Ile
   690 695 700
   Tyr Arg Val Glu Lys Arg Lys Leu Thr Asp Pro Leu Glu Ala Leu Lys
   705 710 715 720
   Glu Lys Tyr Leu Arg Pro Ser Pro Gly Phe Leu Phe His Pro Gln Phe
   725 730 735
   Gln Leu Pro Asp Gln Arg Thr Trp Met Ser Ala Ile Glu Asn Met Ala
   740 745 750
   Glu Lys Leu Glu Ser Phe Ser Ala Leu Lys Pro Glu Ala Ser Glu Leu
   755 760 765
   Leu Gln Ser Val Pro Ser Met Phe Asn Phe Arg Ala Pro Pro Asn Ala
   770 775 780
   Leu Pro Glu Asn Leu Leu Arg Lys Gly Lys Glu Arg Tyr Thr Cys Arg
   785 790 795 800
   Tyr Cys Gly Lys Ile Phe Pro Arg Ser Ala Asn Leu Thr Arg His Leu
   805 810 815
   Arg Thr His Thr Gly Glu Gln Pro Tyr Arg Cys Lys Tyr Cys Asp Arg
   820 825 830
   Ser Phe Ser Ile Ser Ser Asn Leu Gln Arg His Val Arg Asn Ile His
   835 840 845
   Asn Lys Glu Lys Pro Phe Lys Cys His Leu Cys Asp Arg Cys Phe Gly
   850 855 860
   Gln Gln Thr Asn Leu Asp Arg His Leu Lys Lys His Glu Asn Gly Asn
   865 870 875 880
   Met Ser Gly Thr Ala Thr Ser Ser Pro His Ser Glu Leu Glu Ser Thr
   885 890 895
   Gly Ala Ile Leu Asp Asp Lys Glu Asp Ala Tyr Phe Thr Glu Ile Arg
   900 905 910
   Asn Phe Ile Gly Asn Ser Asn His Gly Ser Gln Ser Pro Arg Asn Val
   915 920 925
   Glu Glu Arg Met Asn Gly Ser His Phe Lys Asp Glu Lys Ala Leu Val
   930 935 940
   Thr Ser Gln Asn Ser Asp Leu Leu Asp Asp Glu Glu Val Glu Asp Glu
   945 950 955 960
   Val Leu Leu Asp Glu Glu Asp Glu Asp Asn Asp Ile Thr Gly Lys Thr
   965 970 975
   Gly Lys Glu Pro Val Thr Ser Asn Leu His Glu Gly Asn Pro Glu Asp
   980 985 990
   Asp Tyr Glu Glu Thr Ser Ala Leu Glu Met Ser Cys Lys Thr Ser Pro
   995 1000 1005
   Val Arg Tyr Lys Glu Glu Glu Tyr Lys Ser Gly Leu Ser Ala Leu
   1010 1015 1020
   Asp His Ile Arg His Phe Thr Asp Ser Leu Lys Met Arg Lys Met
   1025 1030 1035
   Glu Asp Asn Gln Tyr Ser Glu Ala Glu Leu Ser Ser Phe Ser Thr
   1040 1045 1050
   Ser His Val Pro Glu Glu Leu Lys Gln Pro Leu His Arg Lys Ser
   1055 1060 1065
   Lys Ser Gln Ala Tyr Ala Met Met Leu Ser Leu Ser Asp Lys Glu
   1070 1075 1080
   Ser Leu His Ser Thr Ser His Ser Ser Ser Asn Val Trp His Ser
   1085 1090 1095
   Met Ala Arg Ala Ala Ala Glu Ser Ser Ala Ile Gln Ser Ile Ser
   1100 1105 1110
   His Val
   1115
<210> 3
   <211> 5680
   <212> DNA
   <213> Homo sapiens
<400> 3
   aacgggctca ttcagcggtc gcgagctgcc cgcgaggggg agcggccgga cggagagcgc      60
   gacccgtccc gggggtgggg ccgggcgcag cggcgagagg aggcgaaggt ggctgcggta     120
   gcagcagcgc ggcagcctcg gacccagccc ggagcgcagg gcggccgctg caggtccccg     180
   ctcccctccc cgtgcgtccg cccatggccg ccgccgggca gctgtgcttg ctctacctgt     240
   cggcggggct cctgtcccgg ctcggcgcag ccttcaactt ggacactcgg gaggacaacg     300
   tgatccggaa atatggagac cccgggagcc tcttcggctt ctcgctggcc atgcactggc     360
   aactgcagcc cgaggacaag cggctgttgc tcgtgggggc cccgcgggca gaagcgcttc     420
   cactgcagag agccaacaga acgggagggc tgtacagctg cgacatcacc gcccgggggc     480
   catgcacgcg gatcgagttt gataacgatg ctgaccccac gtcagaaagc aaggaagatc     540
   agtggatggg ggtcaccgtc cagagccaag gtccaggggg caaggtcgtg acatgtgctc     600
   accgatatga aaaaaggcag catgttaata cgaagcagga atcccgagac atctttgggc     660
   ggtgttatgt cctgagtcag aatctcagga ttgaagacga tatggatggg ggagattgga     720
   gcttttgtga tgggcgattg agaggccatg agaaatttgg ctcttgccag caaggtgtag     780
   cagctacttt tactaaagac tttcattaca ttgtatttgg agccccgggt acttataact     840
   ggaaagggat tgttcgtgta gagcaaaaga ataacacttt ttttgacatg aacatctttg     900
   aagatgggcc ttatgaagtt ggtggagaga ctgagcatga tgaaagtctc gttcctgttc     960
   ctgctaacag ttacttaggt ttttctttgg actcagggaa aggtattgtt tctaaagatg    1020
   agatcacttt tgtatctggt gctcccagag ccaatcacag tggagccgtg gttttgctga    1080
   agagagacat gaagtctgca catctcctcc ctgagcacat attcgatgga gaaggtctgg    1140
   cctcttcatt tggctatgat gtggcggtgg tggacctcaa caaggatggg tggcaagata    1200
   tagttattgg agccccacag tattttgata gagatggaga agttggaggt gcagtgtatg 1260
   tctacatgaa ccagcaaggc agatggaata atgtgaagcc aattcgtctt aatggaacca 1320
   aagattctat gtttggcatt gcagtaaaaa atattggaga tattaatcaa gatggctacc 1380
   cagatattgc agttggagct ccgtatgatg acttgggaaa ggtttttatc tatcatggat 1440
   ctgcaaatgg aataaatacc aaaccaacac aggttctcaa gggtatatca ccttattttg 1500
   gatattcaat tgctggaaac atggaccttg atcgaaattc ctaccctgat gttgctgttg 1560
   gttccctctc agattcagta actattttca gatcccggcc tgtgattaat attcagaaaa 1620
   ccatcacagt aactcctaac agaattgacc tccgccagaa aacagcgtgt ggggcgccta 1680
   gtgggatatg cctccaggtt aaatcctgtt ttgaatatac tgctaacccc gctggttata 1740
   atccttcaat atcaattgtg ggcacacttg aagctgaaaa agaaagaaga aaatctgggc 1800
   tatcctcaag agttcagttt cgaaaccaag gttctgagcc caaatatact caagaactaa 1860
   ctctgaagag gcagaaacag aaagtgtgca tggaggaaac cctgtggcta caggataata 1920
   tcagagataa actgcgtccc attcccataa ctgcctcagt ggagatccaa gagccaagct 1980
   ctcgtaggcg agtgaattca cttccagaag ttcttccaat tctgaattca gatgaaccca 2040
   agacagctca tattgatgtt cacttcttaa aagagggatg tggagacgac aatgtatgta 2100
   acagcaacct taaactagaa tataaatttt gcacccgaga aggaaatcaa gacaaatttt 2160
   cttatttacc aattcaaaaa ggtgtaccag aactagttct aaaagatcag aaggatattg 2220
   ctttagaaat aacagtgaca aacagccctt ccaacccaag gaatcccaca aaagatggcg 2280
   atgacgccca tgaggctaaa ctgattgcaa cgtttccaga cactttaacc tattctgcat 2340
   atagagaact gagggctttc cctgagaaac agttgagttg tgttgccaac cagaatggct 2400
   cgcaagctga ctgtgagctc ggaaatcctt ttaaaagaaa ttcaaatgtc actttttatt 2460
   tggttttaag tacaactgaa gtcacctttg acaccccaga tctggatatt aatctgaagt 2520
   tagaaacaac aagcaatcaa gataatttgg ctccaattac agctaaagca aaagtggtta 2580
   ttgaactgct tttatcggtc tcgggagttg ctaaaccttc ccaggtgtat tttggaggta 2640
   cagttgttgg cgagcaagct atgaaatctg aagatgaagt gggaagttta atagagtatg 2700
   aattcagggt aataaactta ggtaaacctc ttacaaacct cggcacagca accttgaaca 2760
   ttcagtggcc aaaagaaatt agcaatggga aatggttgct ttatttggtg aaagtagaat 2820
   ccaaaggatt ggaaaaggta acttgtgagc cacaaaagga gataaactcc ctgaacctaa 2880
   cggagtctca caactcaaga aagaaacggg aaattactga aaaacagata gatgataaca 2940
   gaaaattttc tttatttgct gaaagaaaat accagactct taactgtagc gtgaacgtga 3000
   actgtgtgaa catcagatgc ccgctgcggg ggctggacag caaggcgtct cttattttgc 3060
   gctcgaggtt atggaacagc acatttctag aggaatattc caaactgaac tacttggaca 3120
   ttctcatgcg agccttcatt gatgtgactg ctgctgccga aaatatcagg ctgccaaatg 3180
   caggcactca ggttcgagtg actgtgtttc cctcaaagac tgtagctcag tattcgggag 3240
   taccttggtg gatcatccta gtggctattc tcgctgggat cttgatgctt gctttattag 3300
   tgtttatact atggaagtgt ggattcttta aacgctctag gtacgatgac agtgttcccc 3360
   gataccatgc tgtaaggatc cggaaagaag agcgagagat caaagatgaa aagtatattg 3420
   ataaccttga aaaaaaacag tggatcacaa agtggaacga aaatgaaagc tactcatagc 3480
   gggggcctaa aaaaaaaaag cttcacagta cccaaactgc tttttccaac tcagaaattc 3540
   aatttggatt taaaagcctg ctcaatccct gaggactgat ttcagagtga ctacacacag 3600
   tacgaaccta cagttttaac tgtggatatt gttacgtagc ctaaggctcc tgttttgcac 3660
   agccaaattt aaaactgttg gaatggattt ttctttaact gccgtaattt aactttctgg 3720
   gttgccttta tttttggcgt ggctgactta catcatgtgt tggggaaggg cctgcccagt 3780
   tgcactcagg tgacatcctc cagatagtgt agctgaggag gcacctacac tcacctgcac 3840
   taacagagtg gccgtcctaa cctcgggcct gctgcgcaga cgtccatcac gttagctgtc 3900
   ccacatcaca agactatgcc attggggtag ttgtgtttca acggaaagtg ctgtcttaaa 3960
   ctaaatgtgc aatagaaggt gatgttgcca tcctaccgtc ttttcctgtt tcctagctgt 4020
   gtgaatacct gctcacgtca aatgcataca agtttcattc tccctttcac taaaacacac 4080
   aggtgcaaca gacttgaatg ctagttatac ttatttgtat atggtattta ttttttcttt   4140
   tctttacaaa ccattttgtt attgactaac aggccaaaga gtctccagtt tacccttcag   4200
   gttggtttaa tcaatcagaa ttagagcatg ggaggtcatc actttgacct aaattattta 4260
   ctgcaaaaag aaaatcttta taaatgtacc agagagagtt gttttaataa cttatctata 4320
   aactataacc tctccttcat gacagcctcc accccacaac ccaaaaggtt taagaaatag 4380
   aattataact gtaaagatgt ttatttcagg cattggatat tttttacttt agaagcctgc 4440
   ataatgtttc tggatttcat actgtaacat tcaggaattc ttggagaaaa tgggtttatt 4500
   cactgaactc tagtgcggtt tactcactgc tgcaaatact gtatattcag gacttgaaag 4560
   aaatggtgaa tgcctatggt ggatccaaac tgatccagta taagactact gaatctgcta 4620
   ccaaaacagt taatcagtga gtcgatgttc tattttttgt tttgtttcct cccctatctg 4680
   tattcccaaa aattactttg gggctaattt aacaagaact ttaaattgtg ttttaattgt 4740
   aaaaatggca gggggtggaa ttattactct atacattcaa cagagactga atagatatga 4800
   aagctgattt tttttaatta ccatgcttca caatgttaag ttatatgggg agcaacagca 4860
   aacaggtgct aatttgtttt ggatatagta taagcagtgt ctgtgttttg aaagaataga 4920
   acacagtttg tagtgccact gttgttttgg gggggctttt ttcttttcgg aaatcttaaa 4980
   ccttaagata ctaaggacgt tgttttggtt gtactttgga attcttagtc acaaaatata 5040
   ttttgtttac aaaaatttct gtaaaacagg ttataacagt gtttaaagtc tcagtttctt 5100
   gcttggggaa cttgtgtccc taatgtgttt agattgctag attgctaagg agctgatact 5160
   ttgacagtgt ttttagacct gtgttactaa aaaaaagatg aatgtcctga aaagggtgtt 5220
   gggagggtgg ttcaacaaag aaacaaagat gttatggtgt ttagatttat ggttgttaaa 5280
   aatgtcatct caagtcaagt cactggtctg tttgcatttg atacattttt gtactaacta 5340
   gcattgtaaa attatttcat gattagaaat tacctgtgga tatttgtata aaagtgtgaa 5400
   ataaattttt tataaaagtg ttcattgttt cgtaacacag cattgtatat gtgaagcaaa 5460
   ctctaaaatt ataaatgaca acctgaatta tctatttcat caaaccaaag ttcagtgttt 5520
   ttatttttgg tgtctcatgt aatctcagat cagccaaaga tactagtgcc aaagcaatgg 5580
   gattcggggt ttttttctgt tttcgctcta tgtaggtgat cctcaagtct ttcattttcc 5640
   ttctttatga ttaaaagaaa cctacaggta tttaacaacc 5680
<210> 4
   <211> 1091
   <212> PRT
   <213> Homo sapiens
<400> 4
   Met Ala Ala Ala Gly Gln Leu Cys Leu Leu Tyr Leu Ser Ala Gly Leu
   1               5                   10                  15
   Leu Ser Arg Leu Gly Ala Ala Phe Asn Leu Asp Thr Arg Glu Asp Asn
               20                  25                  30
   Val Ile Arg Lys Tyr Gly Asp Pro Gly Ser Leu Phe Gly Phe Ser Leu
           35                  40                  45
   Ala Met His Trp Gln Leu Gln Pro Glu Asp Lys Arg Leu Leu Leu Val
       50                  55                  60
   Gly Ala Pro Arg Ala Glu Ala Leu Pro Leu Gln Arg Ala Asn Arg Thr
   65                  70                  75                  80
   Gly Gly Leu Tyr Ser Cys Asp Ile Thr Ala Arg Gly Pro Cys Thr Arg
                   85                  90                  95
   Ile Glu Phe Asp Asn Asp Ala Asp Pro Thr Ser Glu Ser Lys Glu Asp
               100                 105                 110
   Gln Trp Met Gly Val Thr Val Gln Ser Gln Gly Pro Gly Gly Lys Val
           115                 120                 125
   Val Thr Cys Ala His Arg Tyr Glu Lys Arg Gln His Val Asn Thr Lys
       130                 135                 140
   Gln Glu Ser Arg Asp Ile Phe Gly Arg Cys Tyr Val Leu Ser Gln Asn
   145                 150                 155                 160
   Leu Arg Ile Glu Asp Asp Met Asp Gly Gly Asp Trp Ser Phe Cys Asp
                   165                 170                 175
   Gly Arg Leu Arg Gly His Glu Lys Phe Gly Ser Cys Gln Gln Gly Val
               180                 185                 190
   Ala Ala Thr Phe Thr Lys Asp Phe His Tyr Ile Val Phe Gly Ala Pro
           195                 200                 205
   Gly Thr Tyr Asn Trp Lys Gly Ile Val Arg Val Glu Gln Lys Asn Asn
       210                 215                 220
   Thr Phe Phe Asp Met Asn Ile Phe Glu Asp Gly Pro Tyr Glu Val Gly
   225                 230                 235                 240
   Gly Glu Thr Glu His Asp Glu Ser Leu Val Pro Val Pro Ala Asn Ser
                   245                 250                 255
   Tyr Leu Gly Phe Ser Leu Asp Ser Gly Lys Gly Ile Val Ser Lys Asp
               260                 265                 270
   Glu Ile Thr Phe Val Ser Gly Ala Pro Arg Ala Asn His Ser Gly Ala
           275                 280                 285
   Val Val Leu Leu Lys Arg Asp Met Lys Ser Ala His Leu Leu Pro Glu
       290                 295                 300
   His Ile Phe Asp Gly Glu Gly Leu Ala Ser Ser Phe Gly Tyr Asp Val
   305                 310                 315                 320
   Ala Val Val Asp Leu Asn Lys Asp Gly Trp Gln Asp Ile Val Ile Gly
                   325                 330                 335
   Ala Pro Gln Tyr Phe Asp Arg Asp Gly Glu Val Gly Gly Ala Val Tyr
               340                 345                 350
   Val Tyr Met Asn Gln Gln Gly Arg Trp Asn Asn Val Lys Pro Ile Arg
           355                 360                 365
   Leu Asn Gly Thr Lys Asp Ser Met Phe Gly Ile Ala Val Lys Asn Ile
       370                 375                 380
   Gly Asp Ile Asn Gln Asp Gly Tyr Pro Asp Ile Ala Val Gly Ala Pro
   385                 390                 395                 400
   Tyr Asp Asp Leu Gly Lys Val Phe Ile Tyr His Gly Ser Ala Asn Gly
                   405                 410                 415
   Ile Asn Thr Lys Pro Thr Gln Val Leu Lys Gly Ile Ser Pro Tyr Phe
               420                 425                 430
   Gly Tyr Ser Ile Ala Gly Asn Met Asp Leu Asp Arg Asn Ser Tyr Pro
           435                 440                 445
   Asp Val Ala Val Gly Ser Leu Ser Asp Ser Val Thr Ile Phe Arg Ser
       450                 455                 460
   Arg Pro Val Ile Asn Ile Gln Lys Thr Ile Thr Val Thr Pro Asn Arg
   465                 470                 475                 480
   Ile Asp Leu Arg Gln Lys Thr Ala Cys Gly Ala Pro Ser Gly Ile Cys
                   485                 490                 495
   Leu Gln Val Lys Ser Cys Phe Glu Tyr Thr Ala Asn Pro Ala Gly Tyr
               500                 505                 510
   Asn Pro Ser Ile Ser Ile Val Gly Thr Leu Glu Ala Glu Lys Glu Arg
           515                 520                 525
   Arg Lys Ser Gly Leu Ser Ser Arg Val Gln Phe Arg Asn Gln Gly Ser
       530                 535                 540
   Glu Pro Lys Tyr Thr Gln Glu Leu Thr Leu Lys Arg Gln Lys Gln Lys
   545                 550                 555                 560
   Val Cys Met Glu Glu Thr Leu Trp Leu Gln Asp Asn Ile Arg Asp Lys
                   565                 570                 575
   Leu Arg Pro Ile Pro Ile Thr Ala Ser Val Glu Ile Gln Glu Pro Ser
               580                 585                 590
   Ser Arg Arg Arg Val Asn Ser Leu Pro Glu Val Leu Pro Ile Leu Asn
           595                 600                 605
   Ser Asp Glu Pro Lys Thr Ala His Ile Asp Val His Phe Leu Lys Glu
       610                 615                 620
   Gly Cys Gly Asp Asp Asn Val Cys Asn Ser Asn Leu Lys Leu Glu Tyr
   625                 630                 635                 640
   Lys Phe Cys Thr Arg Glu Gly Asn Gln Asp Lys Phe Ser Tyr Leu Pro
                   645                 650                 655
   Ile Gln Lys Gly Val Pro Glu Leu Val Leu Lys Asp Gln Lys Asp Ile
               660                 665                 670
   Ala Leu Glu Ile Thr Val Thr Asn Ser Pro Ser Asn Pro Arg Asn Pro
           675                 680                 685
   Thr Lys Asp Gly Asp Asp Ala His Glu Ala Lys Leu Ile Ala Thr Phe
       690                 695                 700
   Pro Asp Thr Leu Thr Tyr Ser Ala Tyr Arg Glu Leu Arg Ala Phe Pro
   705                 710                 715                 720
   Glu Lys Gln Leu Ser Cys Val Ala Asn Gln Asn Gly Ser Gln Ala Asp
                   725                 730                 735
   Cys Glu Leu Gly Asn Pro Phe Lys Arg Asn Ser Asn Val Thr Phe Tyr
               740                 745                 750
   Leu Val Leu Ser Thr Thr Glu Val Thr Phe Asp Thr Pro Asp Leu Asp
           755                 760                 765
   Ile Asn Leu Lys Leu Glu Thr Thr Ser Asn Gln Asp Asn Leu Ala Pro
       770                 775                 780
   Ile Thr Ala Lys Ala Lys Val Val Ile Glu Leu Leu Leu Ser Val Ser
   785                 790                 795                 800
   Gly Val Ala Lys Pro Ser Gln Val Tyr Phe Gly Gly Thr Val Val Gly
                   805                 810                 815
   Glu Gln Ala Met Lys Ser Glu Asp Glu Val Gly Ser Leu Ile Glu Tyr
               820                 825                 830
   Glu Phe Arg Val Ile Asn Leu Gly Lys Pro Leu Thr Asn Leu Gly Thr
           835                 840                 845
   Ala Thr Leu Asn Ile Gln Trp Pro Lys Glu Ile Ser Asn Gly Lys Trp
       850                 855                 860
   Leu Leu Tyr Leu Val Lys Val Glu Ser Lys Gly Leu Glu Lys Val Thr
   865                 870                 875                 880
   Cys Glu Pro Gln Lys Glu Ile Asn Ser Leu Asn Leu Thr Glu Ser His
                   885                 890                 895
   Asn Ser Arg Lys Lys Arg Glu Ile Thr Glu Lys Gln Ile Asp Asp Asn
               900                 905                 910
   Arg Lys Phe Ser Leu Phe Ala Glu Arg Lys Tyr Gln Thr Leu Asn Cys
           915                 920                 925
   Ser Val Asn Val Asn Cys Val Asn Ile Arg Cys Pro Leu Arg Gly Leu
       930                 935                 940
   Asp Ser Lys Ala Ser Leu Ile Leu Arg Ser Arg Leu Trp Asn Ser Thr
   945                 950                 955                 960
   Phe Leu Glu Glu Tyr Ser Lys Leu Asn Tyr Leu Asp Ile Leu Met Arg
                   965                 970                 975
   Ala Phe Ile Asp Val Thr Ala Ala Ala Glu Asn Ile Arg Leu Pro Asn
               980                 985                 990
   Ala Gly Thr Gln Val Arg Val Thr  Val Phe Pro Ser Lys  Thr Val Ala
           995                 1000                 1005
   Gln Tyr  Ser Gly Val Pro Trp  Trp Ile Ile Leu Val  Ala Ile Leu
       1010                 1015                 1020
   Ala Gly  Ile Leu Met Leu Ala  Leu Leu Val Phe Ile  Leu Trp Lys
       1025                 1030                 1035
   Cys Gly  Phe Phe Lys Arg Ser  Arg Tyr Asp Asp Ser  Val Pro Arg
       1040                 1045                 1050
   Tyr His  Ala Val Arg Ile Arg  Lys Glu Glu Arg Glu  Ile Lys Asp
       1055                 1060                 1065
   Glu Lys  Tyr Ile Asp Asn Leu  Glu Lys Lys Gln Trp  Ile Thr Lys
       1070                 1075                 1080
   Trp Asn  Glu Asn Glu Ser Tyr  Ser
       1085                 1090
<210> 5
   <211> 5695
   <212> DNA
   <213> Homo sapiens
<400> 5
   ggaagaggat ggcagggcca cgccccagcc catgggccag gctgctcctg gcagccttga      60
   tcagcgtcag cctctctggg accttggcaa accgctgcaa gaaggcccca gtgaagagct     120
   gcacggagtg tgtccgtgtg gataaggact gcgcctactg cacagacgag atgttcaggg     180
   accggcgctg caacacccag gcggagctgc tggccgcggg ctgccagcgg gagagcatcg     240
   tggtcatgga gagcagcttc caaatcacag aggagaccca gattgacacc accctgcggc     300
   gcagccagat gtccccccaa ggcctgcggg tccgtctgcg gcccggtgag gagcggcatt     360
   ttgagctgga ggtgtttgag ccactggaga gccccgtgga cctgtacatc ctcatggact     420
   tctccaactc catgtccgat gatctggaca acctcaagaa gatggggcag aacctggctc     480
   gggtcctgag ccagctcacc agcgactaca ctattggatt tggcaagttt gtggacaaag 540
   tcagcgtccc gcagacggac atgaggcctg agaagctgaa ggagccctgg cccaacagtg 600
   accccccctt ctccttcaag aacgtcatca gcctgacaga agatgtggat gagttccgga 660
   ataaactgca gggagagcgg atctcaggca acctggatgc tcctgagggc ggcttcgatg 720
   ccatcctgca gacagctgtg tgcacgaggg acattggctg gcgcccggac agcacccacc 780
   tgctggtctt ctccaccgag tcagccttcc actatgaggc tgatggcgcc aacgtgctgg 840
   ctggcatcat gagccgcaac gatgaacggt gccacctgga caccacgggc acctacaccc 900
   agtacaggac acaggactac ccgtcggtgc ccaccctggt gcgcctgctc gccaagcaca 960
   acatcatccc catctttgct gtcaccaact actcctatag ctactacgag aagcttcaca 1020
   cctatttccc tgtctcctca ctgggggtgc tgcaggagga ctcgtccaac atcgtggagc 1080
   tgctggagga ggccttcaat cggatccgct ccaacctgga catccgggcc ctagacagcc 1140
   cccgaggcct tcggacagag gtcacctcca agatgttcca gaagacgagg actgggtcct 1200
   ttcacatccg gcggggggaa gtgggtatat accaggtgca gctgcgggcc cttgagcacg 1260
   tggatgggac gcacgtgtgc cagctgccgg aggaccagaa gggcaacatc catctgaaac 1320
   cttccttctc cgacggcctc aagatggacg cgggcatcat ctgtgatgtg tgcacctgcg 1380
   agctgcaaaa agaggtgcgg tcagctcgct gcagcttcaa cggagacttc gtgtgcggac 1440
   agtgtgtgtg cagcgagggc tggagtggcc agacctgcaa ctgctccacc ggctctctga 1500
   gtgacattca gccctgcctg cgggagggcg aggacaagcc gtgctccggc cgtggggagt 1560
   gccagtgcgg gcactgtgtg tgctacggcg aaggccgcta cgagggtcag ttctgcgagt 1620
   atgacaactt ccagtgtccc cgcacttccg ggttcctctg caatgaccga ggacgctgct 1680
   ccatgggcca gtgtgtgtgt gagcctggtt ggacaggccc aagctgtgac tgtcccctca 1740
   gcaatgccac ctgcatcgac agcaatgggg gcatctgtaa tggacgtggc cactgtgagt 1800
   gtggccgctg ccactgccac cagcagtcgc tctacacgga caccatctgc gagatcaact 1860
   actcggcgat ccacccgggc ctctgcgagg acctacgctc ctgcgtgcag tgccaggcgt 1920
   ggggcaccgg cgagaagaag gggcgcacgt gtgaggaatg caacttcaag gtcaagatgg 1980
   tggacgagct taagagagcc gaggaggtgg tggtgcgctg ctccttccgg gacgaggatg 2040
   acgactgcac ctacagctac accatggaag gtgacggcgc ccctgggccc aacagcactg 2100
   tcctggtgca caagaagaag gactgccctc cgggctcctt ctggtggctc atccccctgc 2160
   tcctcctcct cctgccgctc ctggccctgc tactgctgct atgctggaag tactgtgcct 2220
   gctgcaaggc ctgcctggca cttctcccgt gctgcaaccg aggtcacatg gtgggcttta 2280
   aggaagacca ctacatgctg cgggagaacc tgatggcctc tgaccacttg gacacgccca 2340
   tgctgcgcag cgggaacctc aagggccgtg acgtggtccg ctggaaggtc accaacaaca 2400
   tgcagcggcc tggctttgcc actcatgccg ccagcatcaa ccccacagag ctggtgccct   2460
   acgggctgtc cttgcgcctg gcccgccttt gcaccgagaa cctgctgaag cctgacactc   2520
   gggagtgcgc ccagctgcgc caggaggtgg aggagaacct gaacgaggtc tacaggcaga   2580
   tctccggtgt acacaagctc cagcagacca agttccggca gcagcccaat gccgggaaaa   2640
   agcaagacca caccattgtg gacacagtgc tgatggcgcc ccgctcggcc aagccggccc   2700
   tgctgaagct tacagagaag caggtggaac agagggcctt ccacgacctc aaggtggccc   2760
   ccggctacta caccctcact gcagaccagg acgcccgggg catggtggag ttccaggagg   2820
   gcgtggagct ggtggacgta cgggtgcccc tctttatccg gcctgaggat gacgacgaga   2880
   agcagctgct ggtggaggcc atcgacgtgc ccgcaggcac tgccaccctc ggccgccgcc   2940
   tggtaaacat caccatcatc aaggagcaag ccagagacgt ggtgtccttt gagcagcctg   3000
   agttctcggt cagccgcggg gaccaggtgg cccgcatccc tgtcatccgg cgtgtcctgg   3060
   acggcgggaa gtcccaggtc tcctaccgca cacaggatgg caccgcgcag ggcaaccggg   3120
   actacatccc cgtggagggt gagctgctgt tccagcctgg ggaggcctgg aaagagctgc   3180
   aggtgaagct cctggagctg caagaagttg actccctcct gcggggccgc caggtccgcc   3240
   gtttccacgt ccagctcagc aaccctaagt ttggggccca cctgggccag ccccactcca   3300
   ccaccatcat catcagggac ccagatgaac tggaccggag cttcacgagt cagatgttgt   3360
   catcacagcc accccctcac ggcgacctgg gcgccccgca gaaccccaat gctaaggccg   3420
   ctgggtccag gaagatccat ttcaactggc tgcccccttc tggcaagcca atggggtaca   3480
   gggtaaagta ctggattcag ggtgactccg aatccgaagc ccacctgctc gacagcaagg   3540
   tgccctcagt ggagctcacc aacctgtacc cgtattgcga ctatgagatg aaggtgtgcg   3600
   cctacggggc tcagggcgag ggaccctaca gctccctggt gtcctgccgc acccaccagg   3660
   aagtgcccag cgagccaggg cgtctggcct tcaatgtcgt ctcctccacg gtgacccagc   3720
   tgagctgggc tgagccggct gagaccaacg gtgagatcac agcctacgag gtctgctatg   3780
   gcctggtcaa cgatgacaac cgacctattg ggcccatgaa gaaagtgctg gttgacaacc   3840
   ctaagaaccg gatgctgctt attgagaacc ttcgggagtc ccagccctac cgctacacgg   3900
   tgaaggcgcg caacggggcc ggctgggggc ctgagcggga ggccatcatc aacctggcca   3960
   cccagcccaa gaggcccatg tccatcccca tcatccctga catccctatc gtggacgccc   4020
   agagcgggga ggactacgac agcttcctta tgtacagcga tgacgttcta cgctctccat   4080
   cgggcagcca gaggcccagc gtctccgatg acactgagca cctggtgaat ggccggatgg   4140
   actttgcctt cccgggcagc accaactccc tgcacaggat gaccacgacc agtgctgctg   4200
   cctatggcac ccacctgagc ccacacgtgc cccaccgcgt gctaagcaca tcctccaccc   4260
   tcacacggga ctacaactca ctgacccgct cagaacactc acactcgacc acactgccca   4320
   gggactactc caccctcacc tccgtctcct cccacggcct ccctcccatc tgggaacacg   4380
   ggaggagcag gcttccgctg tcctgggccc tggggtcccg gagtcgggct cagatgaaag 4440
   ggttcccccc ttccaggggc ccacgagact ctataatcct ggctgggagg ccagcagcgc 4500
   cctcctgggg cccagactct cgcctgactg ctggtgtgcc cgacacgccc acccgcctgg 4560
   tgttctctgc cctggggccc acatctctca gagtgagctg gcaggagccg cggtgcgagc 4620
   ggccgctgca gggctacagt gtggagtacc agctgctgaa cggcggtgag ctgcatcggc 4680
   tcaacatccc caaccctgcc cagacctcgg tggtggtgga agacctcctg cccaaccact 4740
   cctacgtgtt ccgcgtgcgg gcccagagcc aggaaggctg gggccgagag cgtgagggtg 4800
   tcatcaccat tgaatcccag gtgcacccgc agagcccact gtgtcccctg ccaggctccg 4860
   ccttcacttt gagcactccc agtgccccag gcccgctggt gttcactgcc ctgagcccag 4920
   actcgctgca gctgagctgg gagcggccac ggaggcccaa tggggatatc gtcggctacc 4980
   tggtgacctg tgagatggcc caaggaggag ggccagccac cgcattccgg gtggatggag 5040
   acagccccga gagccggctg accgtgccgg gcctcagcga gaacgtgccc tacaagttca 5100
   aggtgcaggc caggaccact gagggcttcg ggccagagcg cgagggcatc atcaccatag 5160
   agtcccagga tggaggaccc ttcccgcagc tgggcagccg tgccgggctc ttccagcacc 5220
   cgctgcaaag cgagtacagc agcatcacca ccacccacac cagcgccacc gagcccttcc 5280
   tagtggatgg gctgaccctg ggggcccagc acctggaggc aggcggctcc ctcacccggc 5340
   atgtgaccca ggagtttgtg agccggacac tgaccaccag cggaaccctt agcacccaca 5400
   tggaccaaca gttcttccaa acttgaccgc accctgcccc acccccgcca cgtcccacta 5460
   ggcgtcctcc cgactcctct cccggagcct cctcagctac tccatccttg cacccctggg 5520
   ggcccagccc acccgcatgc acagagcagg ggctaggtgt ctcctgggag gcatgaaggg 5580
   ggcaaggtcc gtcctctgtg ggcccaaacc tatttgtaac caaagagctg ggagcagcac 5640
   aaggacccag cctttgttct gcacttaata aatggttttg ctactgctaa aaaaa 5695
<210> 6
   <211> 1805
   <212> PRT
   <213> Homo sapiens
<400> 6
   Met Ala Gly Pro Arg Pro Ser Pro Trp Ala Arg Leu Leu Leu Ala Ala
   1               5                   10                  15
   Leu Ile Ser Val Ser Leu Ser Gly Thr Leu Ala Asn Arg Cys Lys Lys
               20                  25                  30
   Ala Pro Val Lys Ser Cys Thr Glu Cys Val Arg Val Asp Lys Asp Cys
           35                  40                  45
   Ala Tyr Cys Thr Asp Glu Met Phe Arg Asp Arg Arg Cys Asn Thr Gln
       50                  55                  60
   Ala Glu Leu Leu Ala Ala Gly Cys Gln Arg Glu Ser Ile Val Val Met
   65                  70                  75                  80
   Glu Ser Ser Phe Gln Ile Thr Glu Glu Thr Gln Ile Asp Thr Thr Leu
                   85                  90                  95
   Arg Arg Ser Gln Met Ser Pro Gln Gly Leu Arg Val Arg Leu Arg Pro
               100                 105                 110
   Gly Glu Glu Arg His Phe Glu Leu Glu Val Phe Glu Pro Leu Glu Ser
           115                 120                 125
   Pro Val Asp Leu Tyr Ile Leu Met Asp Phe Ser Asn Ser Met Ser Asp
       130                 135                 140
   Asp Leu Asp Asn Leu Lys Lys Met Gly Gln Asn Leu Ala Arg Val Leu
   145                 150                 155                 160
   Ser Gln Leu Thr Ser Asp Tyr Thr Ile Gly Phe Gly Lys Phe Val Asp
                   165                 170                 175
   Lys Val Ser Val Pro Gln Thr Asp Met Arg Pro Glu Lys Leu Lys Glu
               180                 185                 190
   Pro Trp Pro Asn Ser Asp Pro Pro Phe Ser Phe Lys Asn Val Ile Ser
           195                 200                 205
   Leu Thr Glu Asp Val Asp Glu Phe Arg Asn Lys Leu Gln Gly Glu Arg
       210                 215                 220
   Ile Ser Gly Asn Leu Asp Ala Pro Glu Gly Gly Phe Asp Ala Ile Leu
   225                 230                 235                 240
   Gln Thr Ala Val Cys Thr Arg Asp Ile Gly Trp Arg Pro Asp Ser Thr
                   245                 250                 255
   His Leu Leu Val Phe Ser Thr Glu Ser Ala Phe His Tyr Glu Ala Asp
               260                 265                 270
   Gly Ala Asn Val Leu Ala Gly Ile Met Ser Arg Asn Asp Glu Arg Cys
           275                 280                 285
   His Leu Asp Thr Thr Gly Thr Tyr Thr Gln Tyr Arg Thr Gln Asp Tyr
       290                 295                 300
   Pro Ser Val Pro Thr Leu Val Arg Leu Leu Ala Lys His Asn Ile Ile
   305                 310                 315                 320
   Pro Ile Phe Ala Val Thr Asn Tyr Ser Tyr Ser Tyr Tyr Glu Lys Leu
                   325                 330                 335
   His Thr Tyr Phe Pro Val Ser Ser Leu Gly Val Leu Gln Glu Asp Ser
               340                 345                 350
   Ser Asn Ile Val Glu Leu Leu Glu Glu Ala Phe Asn Arg Ile Arg Ser
           355                 360                 365
   Asn Leu Asp Ile Arg Ala Leu Asp Ser Pro Arg Gly Leu Arg Thr Glu
       370                 375                 380
   Val Thr Ser Lys Met Phe Gln Lys Thr Arg Thr Gly Ser Phe His Ile
   385                 390                 395                 400
   Arg Arg Gly Glu Val Gly Ile Tyr Gln Val Gln Leu Arg Ala Leu Glu
                   405                 410                 415
   His Val Asp Gly Thr His Val Cys Gln Leu Pro Glu Asp Gln Lys Gly
               420                 425                 430
   Asn Ile His Leu Lys Pro Ser Phe Ser Asp Gly Leu Lys Met Asp Ala
           435                 440                 445
   Gly Ile Ile Cys Asp Val Cys Thr Cys Glu Leu Gln Lys Glu Val Arg
       450                 455                 460
   Ser Ala Arg Cys Ser Phe Asn Gly Asp Phe Val Cys Gly Gln Cys Val
   465                 470                 475                 480
   Cys Ser Glu Gly Trp Ser Gly Gln Thr Cys Asn Cys Ser Thr Gly Ser
                   485                 490                 495
   Leu Ser Asp Ile Gln Pro Cys Leu Arg Glu Gly Glu Asp Lys Pro Cys
               500                 505                 510
   Ser Gly Arg Gly Glu Cys Gln Cys Gly His Cys Val Cys Tyr Gly Glu
           515                 520                 525
   Gly Arg Tyr Glu Gly Gln Phe Cys Glu Tyr Asp Asn Phe Gln Cys Pro
       530                 535                 540
   Arg Thr Ser Gly Phe Leu Cys Asn Asp Arg Gly Arg Cys Ser Met Gly
   545                 550                 555                 560
   Gln Cys Val Cys Glu Pro Gly Trp Thr Gly Pro Ser Cys Asp Cys Pro
                   565                 570                 575
   Leu Ser Asn Ala Thr Cys Ile Asp Ser Asn Gly Gly Ile Cys Asn Gly
               580                 585                 590
   Arg Gly His Cys Glu Cys Gly Arg Cys His Cys His Gln Gln Ser Leu
           595                 600                 605
   Tyr Thr Asp Thr Ile Cys Glu Ile Asn Tyr Ser Ala Ile His Pro Gly
       610                 615                 620
   Leu Cys Glu Asp Leu Arg Ser Cys Val Gln Cys Gln Ala Trp Gly Thr
   625                 630                 635                 640
   Gly Glu Lys Lys Gly Arg Thr Cys Glu Glu Cys Asn Phe Lys Val Lys
                   645                 650                 655
   Met Val Asp Glu Leu Lys Arg Ala Glu Glu Val Val Val Arg Cys Ser
               660                 665                 670
   Phe Arg Asp Glu Asp Asp Asp Cys Thr Tyr Ser Tyr Thr Met Glu Gly
           675                 680                 685
   Asp Gly Ala Pro Gly Pro Asn Ser Thr Val Leu Val His Lys Lys Lys
       690                 695                 700
   Asp Cys Pro Pro Gly Ser Phe Trp Trp Leu Ile Pro Leu Leu Leu Leu
   705                 710                 715                 720
   Leu Leu Pro Leu Leu Ala Leu Leu Leu Leu Leu Cys Trp Lys Tyr Cys
                   725                 730                 735
   Ala Cys Cys Lys Ala Cys Leu Ala Leu Leu Pro Cys Cys Asn Arg Gly
               740                 745                 750
   His Met Val Gly Phe Lys Glu Asp His Tyr Met Leu Arg Glu Asn Leu
           755                 760                 765
   Met Ala Ser Asp His Leu Asp Thr Pro Met Leu Arg Ser Gly Asn Leu
       770                 775                 780
   Lys Gly Arg Asp Val Val Arg Trp Lys Val Thr Asn Asn Met Gln Arg
   785                 790                 795                 800
   Pro Gly Phe Ala Thr His Ala Ala Ser Ile Asn Pro Thr Glu Leu Val
                   805                 810                 815
   Pro Tyr Gly Leu Ser Leu Arg Leu Ala Arg Leu Cys Thr Glu Asn Leu
               820                 825                 830
   Leu Lys Pro Asp Thr Arg Glu Cys Ala Gln Leu Arg Gln Glu Val Glu
   835 840 845
   Glu Asn Leu Asn Glu Val Tyr Arg Gln Ile Ser Gly Val His Lys Leu
   850 855 860
   Gln Gln Thr Lys Phe Arg Gln Gln Pro Asn Ala Gly Lys Lys Gln Asp
   865 870 875 880
   His Thr Ile Val Asp Thr Val Leu Met Ala Pro Arg Ser Ala Lys Pro
   885 890 895
   Ala Leu Leu Lys Leu Thr Glu Lys Gln Val Glu Gln Arg Ala Phe His
   900 905 910
   Asp Leu Lys Val Ala Pro Gly Tyr Tyr Thr Leu Thr Ala Asp Gln Asp
   915 920 925
   Ala Arg Gly Met Val Glu Phe Gln Glu Gly Val Glu Leu Val Asp Val
   930 935 940
   Arg Val Pro Leu Phe Ile Arg Pro Glu Asp Asp Asp Glu Lys Gln Leu
   945 950 955 960
   Leu Val Glu Ala Ile Asp Val Pro Ala Gly Thr Ala Thr Leu Gly Arg
   965 970 975
   Arg Leu Val Asn Ile Thr Ile Ile Lys Glu Gln Ala Arg Asp Val Val
   980 985 990
   Ser Phe Glu Gln Pro Glu Phe Ser Val Ser Arg Gly Asp Gln Val Ala
   995 1000 1005
   Arg Ile Pro Val Ile Arg Arg Val Leu Asp Gly Gly Lys Ser Gln
   1010 1015 1020
   Val Ser Tyr Arg Thr Gln Asp Gly Thr Ala Gln Gly Asn Arg Asp
   1025 1030 1035
   Tyr Ile Pro Val Glu Gly Glu Leu Leu Phe Gln Pro Gly Glu Ala
   1040 1045 1050
   Trp Lys Glu Leu Gln Val Lys Leu Leu Glu Leu Gln Glu Val Asp
   1055 1060 1065
   Ser Leu Leu Arg Gly Arg Gln Val Arg Arg Phe His Val Gln Leu
   1070 1075 1080
   Ser Asn  Pro Lys Phe Gly Ala  His Leu Gly Gln Pro  His Ser Thr
       1085                 1090                 1095
   Thr Ile  Ile Ile Arg Asp Pro  Asp Glu Leu Asp Arg  Ser Phe Thr
       1100                 1105                 1110
   Ser Gln  Met Leu Ser Ser Gln  Pro Pro Pro His Gly  Asp Leu Gly
       1115                 1120                 1125
   Ala Pro  Gln Asn Pro Asn Ala  Lys Ala Ala Gly Ser  Arg Lys Ile
       1130                 1135                 1140
   His Phe  Asn Trp Leu Pro Pro  Ser Gly Lys Pro Met  Gly Tyr Arg
       1145                 1150                 1155
   Val Lys  Tyr Trp Ile Gln Gly  Asp Ser Glu Ser Glu  Ala His Leu
       1160                 1165                 1170
   Leu Asp  Ser Lys Val Pro Ser  Val Glu Leu Thr Asn  Leu Tyr Pro
       1175                 1180                 1185
   Tyr Cys  Asp Tyr Glu Met Lys  Val Cys Ala Tyr Gly  Ala Gln Gly
       1190                 1195                 1200
   Glu Gly  Pro Tyr Ser Ser Leu  Val Ser Cys Arg Thr  His Gln Glu
       1205                 1210                 1215
   Val Pro  Ser Glu Pro Gly Arg  Leu Ala Phe Asn Val  Val Ser Ser
       1220                 1225                 1230
   Thr Val  Thr Gln Leu Ser Trp  Ala Glu Pro Ala Glu  Thr Asn Gly
       1235                 1240                 1245
   Glu Ile  Thr Ala Tyr Glu Val  Cys Tyr Gly Leu Val  Asn Asp Asp
       1250                 1255                 1260
   Asn Arg  Pro Ile Gly Pro Met  Lys Lys Val Leu Val  Asp Asn Pro
       1265                 1270                 1275
   Lys Asn  Arg Met Leu Leu Ile  Glu Asn Leu Arg Glu  Ser Gln Pro
       1280                 1285                 1290
   Tyr Arg  Tyr Thr Val Lys Ala  Arg Asn Gly Ala Gly  Trp Gly Pro
       1295                 1300                 1305
   Glu Arg  Glu Ala Ile Ile Asn  Leu Ala Thr Gln Pro  Lys Arg Pro
       1310                 1315                 1320
   Met Ser  Ile Pro Ile Ile Pro  Asp Ile Pro Ile Val  Asp Ala Gln
   1325 1330 1335
   Ser Gly Glu Asp Tyr Asp Ser Phe Leu Met Tyr Ser Asp Asp Val
   1340 1345 1350
   Leu Arg Ser Pro Ser Gly Ser Gln Arg Pro Ser Val Ser Asp Asp
   1355 1360 1365
   Thr Glu His Leu Val Asn Gly Arg Met Asp Phe Ala Phe Pro Gly
   1370 1375 1380
   Ser Thr Asn Ser Leu His Arg Met Thr Thr Thr Ser Ala Ala Ala
   1385 1390 1395
   Tyr Gly Thr His Leu Ser Pro His Val Pro His Arg Val Leu Ser
   1400 1405 1410
   Thr Ser Ser Thr Leu Thr Arg Asp Tyr Asn Ser Leu Thr Arg Ser
   1415 1420 1425
   Glu His Ser His Ser Thr Thr Leu Pro Arg Asp Tyr Ser Thr Leu
   1430 1435 1440
   Thr Ser Val Ser Ser His Gly Leu Pro Pro Ile Trp Glu His Gly
   1445 1450 1455
   Arg Ser Arg Leu Pro Leu Ser Trp Ala Leu Gly Ser Arg Ser Arg
   1460 1465 1470
   Ala Gln Met Lys Gly Phe Pro Pro Ser Arg Gly Pro Arg Asp Ser
   1475 1480 1485
   Ile Ile Leu Ala Gly Arg Pro Ala Ala Pro Ser Trp Gly Pro Asp
   1490 1495 1500
   Ser Arg Leu Thr Ala Gly Val Pro Asp Thr Pro Thr Arg Leu Val
   1505 1510 1515
   Phe Ser Ala Leu Gly Pro Thr Ser Leu Arg Val Ser Trp Gln Glu
   1520 1525 1530
   Pro Arg Cys Glu Arg Pro Leu Gln Gly Tyr Ser Val Glu Tyr Gln
   1535 1540 1545
   Leu Leu Asn Gly Gly Glu Leu His Arg Leu Asn Ile Pro Asn Pro
   1550 1555 1560
   Ala Gln Thr Ser Val Val Val Glu Asp Leu Leu Pro Asn His Ser
   1565 1570 1575
   Tyr Val  Phe Arg Val Arg Ala  Gln Ser Gln Glu Gly  Trp Gly Arg
       1580                 1585                 1590
   Glu Arg  Glu Gly Val Ile Thr  Ile Glu Ser Gln Val  His Pro Gln
       1595                 1600                 1605
   Ser Pro  Leu Cys Pro Leu Pro  Gly Ser Ala Phe Thr  Leu Ser Thr
       1610                 1615                 1620
   Pro Ser  Ala Pro Gly Pro Leu  Val Phe Thr Ala Leu  Ser Pro Asp
       1625                 1630                 1635
   Ser Leu  Gln Leu Ser Trp Glu  Arg Pro Arg Arg Pro  Asn Gly Asp
       1640                 1645                 1650
   Ile Val  Gly Tyr Leu Val Thr  Cys Glu Met Ala Gln  Gly Gly Gly
       1655                 1660                 1665
   Pro Ala  Thr Ala Phe Arg Val  Asp Gly Asp Ser Pro  Glu Ser Arg
       1670                 1675                 1680
   Leu Thr  Val Pro Gly Leu Ser  Glu Asn Val Pro Tyr  Lys Phe Lys
       1685                 1690                 1695
   Val Gln  Ala Arg Thr Thr Glu  Gly Phe Gly Pro Glu  Arg Glu Gly
       1700                 1705                 1710
   Ile Ile  Thr Ile Glu Ser Gln  Asp Gly Gly Pro Phe  Pro Gln Leu
       1715                 1720                 1725
   Gly Ser  Arg Ala Gly Leu Phe  Gln His Pro Leu Gln  Ser Glu Tyr
       1730                 1735                 1740
   Ser Ser  Ile Thr Thr Thr His  Thr Ser Ala Thr Glu  Pro Phe Leu
       1745                 1750                 1755
   Val Asp  Gly Leu Thr Leu Gly  Ala Gln His Leu Glu  Ala Gly Gly
       1760                 1765                 1770
   Ser Leu  Thr Arg His Val Thr  Gln Glu Phe Val Ser  Arg Thr Leu
       1775                 1780                 1785
   Thr Thr  Ser Gly Thr Leu Ser  Thr His Met Asp Gln  Gln Phe Phe
       1790                 1795                 1800
   Gln Thr
       1805
<210> 7
   <211> 60
   <212> RNA
   <213> Artificial
<220>
   <223> sequence forming shEVI1 with SEQ ID NO: 8
<400> 7
   gaucucucua aggcugaacu agcaguucaa gagacugcua guucagccuu agauuuuuug 60
<210> 8
   <211> 60
   <212> RNA
   <213> Artificial
<220>
   <223> sequence forming shEVI1 with SEQ ID NO: 7
<400> 8
   aauucaaaaa aucuaaggcu gaacuagcag ucucuugaac ugcuaguuca gccuuagaga 60
<210> 9
   <211> 20
   <212> DNA
   <213> Artificial
<220>
   <223> forward primer for Human EVI1
<400> 9
   cgaaagcgag aatgatctcc 20
<210> 10
   <211> 20
   <212> DNA
   <213> Artificial
<220>
   <223> reverse primer for Human EVI1
<400> 10
   ggaagacgta gtgctgaaca 20
<210> 11
   <211> 20
   <212> DNA
   <213> Artificial
<220>
   <223> forward primer for Human beta-actin
<400> 11
   aagagatggc cacggctgct 20
<210> 12
   <211> 20
   <212> DNA
   <213> Artificial
<220>
   <223> reverse primer for Human beta-actin
<400> 12
   tccttctgca tcctgtcggc 20
<210> 13
   <211> 60
   <212> RNA
   <213> Artificial
<220>
   <223> sequence forming shEVI1 with SEQ ID NO: 14
<400> 13
   gaucuccgag uugcgugggg ugugucuuca agagagacac acuccgugca gcucuuuuug 60
<210> 14
   <211> 60
   <212> RNA
   <213> Artificial
<220>
   <223> sequence forming shEVI1 with SEQ ID NO: 13
<400> 14
   aauucaaaaa gagcugcacg gagugugucu cucuugaaga cacaccccac gcaacucgga 60

## Description

### TECHNICAL FIELD

The present invention relates in general to a cell adhesion inhibitor that inhibits the adhesion of leukaemia cells, for use in the prevention or treatment of acute myelocytic leukaemia, wherein the inhibitor is used in combination with an anticancer drug to enhance sensitivity of a cancer cell to the anticancer drug, wherein the anticancer drug is Ara-C. Also disclosed herein is a diagnosis agent for cancer, containing a substance detectable expression of the above target protein or a gene (polynucleotide) encoding the relevant target protein. Still further, the present invention relates to a method for screening a substance to inhibit cell adhesion of a cancer cell.

### BACKGROUND ART

In recent years, with rapid progress of molecular biology, development of a novel type of drug called "molecularly-targeted drug" has been promoted, and several commercial products have already been sold on the market.

For example, to cite a cancer as an example, conventional anticancer drugs have been mainly those targeting a specific stage of DNA synthesis or a cell cycle. Their intensions are to exert a specific poisonous property to a cancer cell, by utilizing a property of the cancer cell that has higher proliferation activity as compared with a normal cell. Therefore, there was a problem that a cell which is not a cancer cell but has an equivalent proliferation speed as a cancer cell (for example, a juvenile hematopoietic cell in bone marrow, a mouth mucosa cell, a gastrointestinal epithelial cell, and a hair follicle cell) and the like also receives a disorder caused by conventional anticancer drugs, to generate side effect such as bone marrow suppression (leucopenia, thrombopenia), digestive system disorder, hair loss, or the like caused by them.

On the contrary, the above "molecularly-targeted drug" targets a molecule specifically (or excessively) expressing in a cancer cell, and thus exerting anticancer property by selective action to the relevant molecule. Therefore, according to cancer treatment using the molecularly-targeted drug, there is an advantage that generation of such side effects as described above can be suppressed, as compared with the case where a conventional anticancer drug is used.

As a means to develop a novel anticancer drug as the molecularly-targeted drug, expression analysis / function analysis of a gene / protein in a cancer cell / cancer tissue is effective. By such an analysis, it is considered to become possible to judge validity for a specific therapeutic agent / therapeutic method, or to search a novel drug discovery target.

AML (Acute Myelogenous Leukemia) is a kind of leukemia, which is a blood cancer, and a disease where a hematopoietic cell of a bone marrow system shows tumorigenic transformation and loses differentiation / maturation capability. The hematopoietic cell which lost differentiation / maturation capability keeps to take a form of a juvenile state, therefore it is called "blast" as well. In AML, by accumulation of this blast in bone marrow, production capability for a normal blood corpuscle decreases. As a result, a symptom such as anemia / breath shortening / palpitation / facial pallor, caused by erythropenia; or a symptom such as hemorrhage from gum / melena / hematemesis from a gastrointestinal / cerebral hemorrhage, caused by deficiency of platelets is expressed. Still further, immunity against infection decreases, and a symptom such as high fever or lassitude caused by deficiency of leukocytes becomes to be expressed.

According to the FAB (French-American-British) classification advocated in 1976, AML is defined as a state where ratio of blast occupying in a bone marrow cell is 30% or more. On the other hand, according to the WHO classification announced by the World Health Organization (WHO) as a novel classification method for leukemia including also variation of a chromosome or a gene, AML is defined as a state where ratio of blast is 20% or more.

To explain pathogenesis mechanism of leukemia, a concept of what is called "a leukemia stem cell" has been advocated in recent years. According to this concept, it is explained that pathology of leukemia expresses by functioning of only a small number of cells among hematopoietic stem cells having significantly enhanced self-replication capability as "a stem cell" for supplying a leukemia cell, and continuing to supply a leukemia cell, while repeating self-replication and limited differentiation.

In addition, it has been reported that the leukemia stem cell in AML escapes from apoptosis induced by a chemical treatment agent, by fixing in an endosteum region of bone marrow via a cell adhesion molecule, that is, acquires resistance to the chemical treatment agent (see, for example, Non-Patent Literature 1).

Conventionally, it has been known that pathology recurs caused by MRD (Minimal Residual Disease), in an ALM patient who experienced a chemical treatment method. In addition, it was clarified that, as one of the causes for acquiring drug resistance inducing such recurrence, adhesion of VLA (Very Late Antigen)-4 present on the surface of the leukemia cell to fibronectin on the surface of a bone-marrow stromal cell (osteoblast cell) was involved. Based on such knowledge, a therapeutic method / recurrence prophylactic method for AML with VLA-4 as a target has been proposed. For example, it has been proposed to inhibit adhesion between VLA-4 and fibronectin by administering a VLA-4-specific antibody (a VLA-4-neutralizing antibody), thereby treat / prevent AML (see, for example, Non-Patent Literature 2). It should be noted that VLA-4 is a hetero dimer protein composed of a α4 subunit and a β1 subunit of an integrin family, and the β1 subunit participates in adhesion to the osteoblast cell of bone marrow.

Still further, the present inventors have identified an Evil (ecotropic viral integration-1) gene, as an incorporation site of a retrovirus in bone-marrow cancer (leukemia) of mouse (see Non-Patent Literature 3).

The Evil gene is a gene composed of 3099bp (Refseq Accession No. NM_007963), isolated from a virus insertion site of mouse leukemia, as described above, and encodes a protein composed of 1032 amino acids (Evil protein) (RefSeq Accession No. NP_031908). The human homologue gene (EVI1 gene) is a gene composed of 4873bp (Refseq Accession No. NM_001105077, SEQ ID No: 1), and encodes a protein (Evil protein) composed of 1115 amino acids (Refseq Accession No. NP_001098547, SEQ ID No: 2). It has been confirmed that the Evil protein / EVI1 protein express mainly inside of a nucleus of a mouse / human hematopoietic stem cell or a leukemia cell, respectively.

Here, as for a relation between the EVI1 gene and a cancer, the present inventors have discovered that in human AML, activation of the EVI1 gene by dislocation at a long arm of No. 3 chromosome (3q26), where the EVI1 gene locates, is observed in certain cases (see Non-Patent Literature 4). After that, it has been clarified that the EVI1 gene has an important role in cell proliferation / differentiation in various types of cells, and it has also been clarified that the EVI1 gene expresses also in the hematopoietic stem cell of bone marrow and thus participates in controlling the hematopoietic stem cell (see, for example, Non-Patent Literature 5). The present inventors have clarified that this EVI1 gene participates in proliferation of the hematopoietic stem cell by controlling expression of a GATA-2 gene (see Non-Patent Literature 6). In addition, in recent years, it has been reported that the EVI1 gene is an important control factor in proliferation of the hematopoietic stem cell or transformed leukemia cell (see Non-Patent Literature 7). However, any specific method relating to treatment / prevention for cancer such as AML based on such knowledge has not been proposed. It should be noted that activation of the EVI1 gene is observed in about 20 to 30% of human AML, and such AML has been known to be intractable and has a high recurrence frequency.

### PRIOR ART LITERATURES

### NON-PATENT LITERATURES

Non-Patent Literature 1: Ishikawa et al., Nature Biotechnology 25, 1315-1321 (2007)
Non-Patent Literature 2: Matsunaga et al., Nature Medicine 9, 1158-1165 (2003)
Non-Patent Literature 3: Morishita et al., Cell 54 (6), 831-840 (1988)
Non-Patent Literature 4: Morishita et al., Proc. Natl. Acad. Sci. U.S.A. 89 (9), 3937-3941 (1992)
Non-Patent Literature 5: Phillips et al., Science 288, 1635-1640 (2000)
Non-Patent Literature 6: Yuasa et al., The EMBO Journal 24, 1976-1987 (2005)
Non-Patent Literature 7: Goyama et al., Cell Stem Cell 3 (2), 207-220 (2008)

Liu et al. disclose that a knockdown of Evil by siRNA inhibited AKT phosphorylation in HT-29 human colon cancer cells and increased their sensitivity to taxol-mediated apoptosis (Liu et al., Oncogene 25, 3565-3575 (2006)).

US 6869795 discloses antisense oligonucleotide compositons targeting mRNA transcribed by Evi-1 gene.

### SUMMARY OF THE INVENTION

### PROBLEM TO BE SOLVED BY THE INVENTION

As described above, a treatment method / recurrence prevention method for AML using VLA-4 (α4/ β1 integrin) as a target has conventionally been proposed. However, a treatment method / recurrence prevention method for AML using, as a target, the EVI1 gene or mRNA / protein which is directly encoded by the gene, genes relating to the EVI1 gene or mRNAs / proteins which is encoded by the genes has not been known. The present state is that development of a novel molecularly-targeted treatment method using a molecule other than VLA-4 as a target has been still strongly desired, in view of contribution to progress of medical treatment technology by increasing variation of more effective treatment method / recurrence prevention method with less side effects.

Accordingly, it is an object of the present invention to provide a prevention / treatment means for cancer by controlling expression / function of a target molecule different from a conventional one, as a novel method in the molecularly-targeted treatment method for cancer. In addition, it is an object of the present invention to provide a method for screening a substance having preventing / treating activity for cancer using the above target molecule.

### MEANS FOR SOLVING THE PROBLEM

The present inventors have intensively studied a way to solve the above-described problems. As a result, they have found that by suppressing expression / function of EVI1 gene or EVI1 protein, cell adhesion capability of thus suppressed cell decreases. Similarly, they have found that also by suppressing expression / function of each protein (ITGA6 protein and ITGA4 protein, respectively) composing α6 subunit and β4 subunit of integrin which are known to form a dimer, or also by suppressing expression / function of each gene (ITGA6 gene and ITGA4 gene, respectively) encoding the proteins, cell adhesion capability of thus suppressed cell also decreases. In such way, they have verified that EVI1 gene /protein or ITGA6 / ITGA4 gene / protein can be a target for prevention / treatment for cancer, and have thus completed the present invention.

That is, according to one aspect of the present invention, there is provided a cell adhesion inhibitor that inhibits the adhesion of leukaemia cells, for use in the prevention or treatment of acute myelocytic leukaemia, wherein the inhibitor is used in combination with an anticancer drug to enhance sensitivity of a cancer cell to the anticancer drug, wherein the anticancer drug is Ara-C, the inhibitor comprising:
(i) an antisense polynucleotide comprising a base sequence that is complementary to a target region of a base sequence of a polynucleotide encoding the amino acid sequence represented by SEQ ID NO: 2, or
(ii) an antisense polynucleotide comprising a base sequence that has at least 90% homology to the antisense polynucleotide of (i) for the over-lapped region thereof,
wherein the antisense polynucleotide is capable of inhibiting expression of said polynucleotide encoding the amino acid sequence represented by SEQ ID NO: 2.

Also disclosed herein is a cell adhesion inhibitor for a cancer cell, containing an antibody to a protein or a partial peptide thereof containing the same or substantially the same amino acid sequence as the amino acid sequence represented by SEQ ID No: 2, SEQ ID No: 4 or SEQ ID No: 6.

Also disclosed herein is a cell adhesion inhibitor for a cancer cell, containing an antisense polynucleotide containing a complementary or substantially complementary base sequence or a part thereof, in a base sequence of a polynucleotide encoding a protein containing the same or substantially the same amino acid sequence as the amino acid sequence represented by SEQ ID No: 2, SEQ ID No: 4 or SEQ ID No: 6.

Also disclosed herein is a cell adhesion inhibitor for a cancer cell, containing a substance to inhibit expression and/or activity of a protein containing the same or substantially the same amino acid sequence as the amino acid sequence represented by SEQ ID No: 2, SEQ ID No: 4 or SEQ ID No: 6.

Here, the above-described inhibitor is used, for example, for prevention or treatment of a cancer. In addition, the above-described inhibitor may be one to be used to improve sensitivity of a cancer cell to the above-described anticancer drug, when used in combination with another anticancer drug. The above-described cancer may be leukemia (for example, acute myelogenous leukemia).

Also disclosed herein is a method for inhibiting cell adhesion of a cancer cell, including inhibition of expression and/or activity of a protein containing the same or substantially the same amino acid sequence as the amino acid sequence represented by SEQ ID No: 2, SEQ ID No: 4 or SEQ ID No: 6.

Also disclosed herein is a method for inhibiting cell adhesion of a cancer cell, including inhibition of expression and/or activity of a polynucleotide encoding a protein containing the same or substantially the same amino acid sequence as the amino acid sequence represented by SEQ ID No: 2, SEQ ID No: 4 or SEQ ID No: 6.

Also disclosed herein is a use of a substance to inhibit expression and/or activity of a protein containing the same or substantially the same amino acid sequence as the amino acid sequence represented by SEQ ID No: 2, SEQ ID No: 4 or SEQ ID No: 6, to produce a cell adhesion inhibitor for a cancer cell.

Also disclosed herein is a use of a substance to inhibit expression and/or activity of a polynucleotide encoding a protein containing the same or substantially the same amino acid sequence as the amino acid sequence represented by SEQ ID No: 2, SEQ ID No: 4 or SEQ ID No: 6, for the production of a cell adhesion inhibitor for a cancer cell. Here, the above-described cell adhesion inhibitor may be for preventing or treating a cancer.

According to another aspect of the present invention, there is provided a method for screening a substance to inhibit cell adhesion of a cancer cell, the method comprising using a protein comprising an amino acid sequence represented by SEQ ID No: 2 to identify a substance that inhibits the activity of said protein thereby inhibiting cell adhesion of a cancer cell. In an embodiment, the protein is provided in a form of a cell producing the same. In an embodiment, said cancer is leukaemia. In an embodiment, the method is to be used for screening a substance for prevention or treatment of a cancer.

Also disclosed herein is a method for screening a substance to inhibit cell adhesion of a cancer cell, using a protein containing the same or substantially the same amino acid sequence as the amino acid sequence represented by SEQ ID No: 2, SEQ ID No: 4 or SEQ ID No: 6 or a partial peptide thereof. Here, the protein containing the same or substantially the same amino acid sequence as the amino acid sequence represented by SEQ ID No: 2, SEQ ID No: 4 or SEQ ID No: 6 or the partial peptide thereof may be provided in a form of a cell producing the same. In this case, any one selected from the group consisting of an antibody to a protein containing the same or substantially the same amino acid sequence as the amino acid sequence represented by SEQ ID No: 2, SEQ ID No: 4 or SEQ ID No: 6 or a partial peptide thereof, along with a polynucleotide encoding the protein, and a polynucleotide containing a part of the base sequence thereof may be used further. Still more, the above screening method may be used for screening a substance for prevention or treatment of a cancer.

According to the present disclosure, a prevention / treatment means for cancer can be provided, by controlling expression / function of a target molecule different from a conventional one, as a novel method in the molecularly-targeted treatment method of a cancer. In addition, a method for screening a substance having prevention / treatment activity for cancer using the above-described target molecule, can be provided.

### BRIEF DESCRIPTION OF THE DRAWINGS

Fig. 1 is a drawing showing the results of an agarose gel electrophoresis in Example 1-1.
Fig. 2 is a drawing showing the results of an agarose gel electrophoresis in Example 1-1.
Fig. 3 is a graph showing the results of cell adhesion assay in Example 1-2.
Fig. 4 is a drawing showing the results of an agarose gel electrophoresis in Reference Example 1.
Fig. 5 is a drawing showing the results of an agarose gel electrophoresis in Example 2-1.
Fig. 6 is a graph showing the results of cell adhesion assay in Example 2-2.
Fig. 7 is a graph showing the results of cell proliferation assay in Reference Example 2.
Fig. 8 is a graph showing the results of cell adhesion assay in Example 3-1.
Fig. 9 is a drawing showing the results of an agarose gel electrophoresis in Example 3-1.
Fig. 10 is a graph showing the results of cell adhesion assay in Example 3-2.
Fig. 11 is a drawing showing the results of an agarose gel electrophoresis in Reference Example 3-1.
Fig. 12 is a graph showing the results of cell adhesion assay in Reference Example 3-2.
Fig. 13 is a graph showing the results of cell proliferation assay in Reference Example 3-3.
Fig. 14 is a drawing showing the results of an agarose gel electrophoresis in Reference Example 4-1.
Fig. 15 is a drawing showing the results of an agarose gel electrophoresis in Reference Example 4-2.
Fig. 16 is a drawing showing the results of DNA microarray evaluation in Reference Example 4-3.
Fig. 17 is a drawing showing the results of DNA microarray evaluation in Reference Example 4-4.
Fig. 18 is a graph showing the results of drug resistance assay by a WST method in Reference Example 5.
Fig. 19 is a graph showing the results of drug resistance assay by a WST method in Reference Example 5.
Fig. 20 is a graph showing the results of drug resistance assay by a WST method in Example 4. Fig. 20 (a) is a graph showing the results of culture of AML1(shLuc), and Fig. 20 (b) is a graph showing the results of culture of AML1(shEVI1).
Fig. 21 is a graph showing the results of the collection of gene expression data in a bone-marrow cell of a human AML patient from gene expression bank (GEO) of NCBI in Reference Example 6. Fig. 21 (a) shows expression level data of ITGA6 gene, and Fig. 21 (b) shows expression level data of ITGA4 gene.
Fig. 22 is a graph showing the results of the collection of gene expression data in various bone marrow / peripheral blood cells in human from gene expression bank (GEO) of NCBI, in comparison with the results of expression level of ITGA6 gene, in Reference Example 7.
Fig. 23 is a graph showing the results of the collection of gene expression data in various bone marrow / peripheral blood cells in human from gene expression bank (GEO) of NCBI, in comparison with the results of expression level of ITGA4 gene, in Reference Example 7.
Fig. 24 is a drawing showing the results of an agarose gel electrophoresis in Reference Example 8.
Fig. 25 is a graph showing the measurement results by a FACS method in Example 5. Fig. 25 (a) shows the results of FACS using AML1(shLuc) cell, and Fig. 25 (b) shows the results of FACS using an AML1(shEVI1) cell.

### MODE FOR CARRYING OUT THE INVENTION

It will be outlined how the present inventors have come to complete the present invention. Firstly, the present inventors have found that, by suppressing expression of EVI1 gene, expression of integrin is suppressed in various human leukemia cells, and cell adhesion capability of the cells decrease. Specifically, it has been clarified that expression of α6 subunit and β4 subunit of integrin can be suppressed by suppressing expression of the EVI1 gene.

Here, a protein composing the α6 subunit of human integrin (ITGA6 protein) is encoded by ITGA6 gene composed of 5680 bp (RefSeq Accession No. NM_001079818, SEQ ID No: 3), and composed of 1091 amino acids (RefSeq Accession No. NP_001073286, SEQ ID No: 4). A homolog protein (Itga6 protein) in mouse is encoded by Itga6 gene composed of 4205 bp (Refseq Accession No. NM_008397), and composed of 1073 amino acids (Refseq Accession No. NP_032423).

In addition, a protein composing the β4 subunit of human integrin (ITGB4 protein) is encoded by ITGB4 gene composed of 5695 bp (RefSeq Accession No. NM_001005619, SEQ ID No: 5), and composed of 1805 amino acids (RefSeq Accession No. NP_001005619, SEQ ID No: 6). A homolog protein (Itgb4 protein) in mouse is encoded by Itgb4 gene composed of 6160 bp (RefSeq Accession No. NM_001005608), and composed of 1802 amino acids (RefSeq Accession No. NP_001005608). It should be noted that the present inventors have confirmed that the above ITGA6 gene and ITGB4 gene are expressed specifically in EVIl-positive cells.

From the above results, the present inventors set a hypothesis that ITGA6 gene / protein along with ITGB4 gene / protein could be involved in exertion of cell adhesion capability in a cancer cell such as a leukemia cell. On that basis, it was confirmed that by suppressing expression of the ITGB4 gene, or inhibiting activity of the ITGB4 protein by a neutralization antibody, cell adhesion capability of a human leukemia cell decreased, and thus the above hypothesis was verified. It should be noted that although a confirmation experiment as described above has not been carried out for the ITGA6 gene or the ITGA6 protein, it is considered with extremely high probability that the similar result as in the above ITGB4 gene / protein can be obtained also in the ITGB6 gene / protein in consideration of the fact that expression of the ITGA6 gene is suppressed by suppressing expression of the EVI1 similarly as in the ITGB4 gene, and specificity in expression of the ITGA6 gene in various cells is similar to that of ITGB4 gene,.

Here, it has been known that, in an AML cell where the EVI1 gene is expressing, expression of integrin is increased. Therefore, in view of such knowledge, the present inventors set a hypothesis that integrin could be involved in exertion of cell adhesion capability of a leukemia cell to bone marrow. On that basis, it was confirmed that by introducing the EVI1 gene to an EVI1-negative cell, cell adhesion capability of the cell increased, and in this case expressions of the ITGA6 gene and the ITGB4 gene increased as well. In combination with this, also in experiments using a clinical specimen of AML, a correlation was found between expression of the EVI1 gene and expression of the ITGA6 gene / the ITGB4 gene, and thus the above hypothesis was verified.

Based on the several items of knowledge described above, the present inventors have come to complete the present invention. Hereinafter, embodiments for carrying out the present invention will be explained in detail. However, the scope of the present invention is not limited only to the embodiments described below.

A protein to be used in accordance with the present disclosure (also called "protein of the present disclosure") is a protein containing the same or substantially the same amino acid sequence as the amino acid sequence represented by SEQ ID No: 2, SEQ ID No: 4 or SEQ ID No: 6. The protein of the present disclosure may be a protein to be isolated or purified from a cell [for example, hepatocyte, splenocyte, neurocyte, glial cell, pancreas beta cell, marrow cell, mesangial cell, Langerhans cell, epidermic cell, epithelial cell, goblet cell, endothelial cell, smooth muscles cell, fibroblast, fibrocyte, muscle cell, adipocyte, immune cell (for example, macrophage, T cell, B cell, natural killer cell, mast cell, neutrophil, basophilic leukocyte, eosinocyte, monocyte), megakaryocyte, synovial cell, chondrocyte, osteocyte, osteoblast, osteoclast, breast cell or interstitial cell, or progenitor cell, stem cell, or cancer cell of these cell, or the like], or all tissues in which these cells exist [for example, brain, each part (for example, olfactory bulb, amaygdala, cerebral basal cell, hippocampal, thalamus, hypothalamus, cerebral cortex, oblongata, cerebellar) of brain, spinal cord, pituitary, stomach, pancreas, kidney, liver, gonad, thyroid, gallbladder, marrow, adrenal, skin, muscle (for example, smooth muscles, skeletal muscles), lung, gastrointestinal (for example, large intestine, intestine), blood vessel, heart, thymus, lienal, submandibular gland, peripherals blood, prostate gland, testis, ovarian, placenta, uterus, bone, joint, adipose tissue (for example, white adipose tissue, brown adipose tissue), and the like] of human or other warm-blooded animals (for example, guinea pig, rat, mouse, chicken, rabbit, pig, sheep, cow, monkey or the like). In addition, it may be a protein chemically synthesized, or biochemically synthesized in a cell-free translation system, or a recombination protein produced from a transformant introduced with a nucleic acid having a base sequence encoding the above amino acid sequence.

Substantially the same amino acid sequence as the amino acid sequence represented by SEQ ID No: 2, SEQ ID No: 4 or SEQ ID No: 6 includes an amino acid sequence having a homology with the amino acid sequence represented by SEQ ID No: 2, SEQ ID No: 4 or SEQ ID No: 6 in about 50% or more, preferably about 60% or more, more preferably about 70% or more, further more preferably about 80% or more, particularly preferably about 90% or more, and most preferably about 95% or more, or the like. Here, "homology" means a ratio (%) of the same amino acid and similar amino acid residual groups relative to the whole overlapping amino acid residual groups, in an optimal alignment, in the case when two amino acid sequences are aligned using mathematical algorism known in the relevant technical field (preferably, said algorism is the one where introduction of a gap to either/both of the sequences can be considered for optimal alignment). The "similar amino acid" means an amino acid similar in physicochemical property, and includes an amino acid classified to the same group, for example, an aromatic amino acid (Phe, Trp, Tyr), an aliphatic amino acid (Ala, Leu, Ile, Val), a polar amino acid (Gln, Asn), a basic amino acid (Lys, Arg, His), an acidic amino acid (Glu, Asp), an amino acid having a hydroxyl group (Ser, Thr), an amino acid with small side chain (Gly, Ala, Ser, Thr, Met) or the like. It is predicted that substitution with such a similar amino acid does not bring about any change in expression type of the protein (that is, it is a preservative amino acid substitution). A specific example of the preservative amino acid substitution has been well-known in the relevant technical field, and has been described in various literatures (see, for example, Bowie et al., Science, 247: 1306-1310 (1990)).

Homology of an amino acid sequence in the present specification can be calculated using a homology calculation algorism, NCBI BLAST (National Center for Biotechnology Information Basic Local Alignment Search Tool), under the following conditions (expected value = 10; a gap is allowed; matrix = BLOSUM62; filtering = OFF). Other algorisms for determining a homology of an amino acid sequence includes, for example, an algorism described in Karlin et al., Proc. Natl. Acad. Sci. USA, 90: 5873-5877 (1993) [said algorism has been incorporated in NBLAST and XBLAST program (version 2.0) (Altschul et al., Nucleic Acids Res., 25: 3389-3402 (1997))]; an algorism described in Needleman et al., J. Mol. Biol., 48: 444-453 (1970), [said algorism has been incorporated in GAP program in GCG software package]; an algorism described in Myers and Miller, CABIOS, 4: 11-17 (1988) [said algorism has been incorporated in ALIGN program (version 2.0), which is a part of CGC sequence alignment software package]; an algorism described in Pearson et al., Proc. Natl. Acad. Sci. USA, 85: 2444-2448 (1988) [said algorism has been incorporated in FASTA program in GCG software package]; and the like, and they may be used preferably as well.

More preferably, substantially the same amino acid sequence as the amino acid sequence represented by SEQ ID No: 2, SEQ ID No: 4 or SEQ ID No: 6, is an amino acid sequence having an identity to the amino acid sequence represented by SEQ ID No: 2, SEQ ID No: 4 or SEQ ID No: 6 in about 50% or more, preferably about 60% or more, more preferably about 70% or more, further more preferably about 80% or more, particularly preferably about 90% or more, and most preferably about 95% or more.

The protein to be used in accordance with the present disclosure is a protein containing substantially the same amino acid sequence as the amino acid sequence represented by SEQ ID No: 2, SEQ ID No: 4 or SEQ ID No: 6, and having substantially the same quality of activity as a protein containing the amino acid sequence represented by SEQ ID No: 2, SEQ ID No: 4 or SEQ ID No: 6.

Substantially the same quality of activity includes, for example, ligand bonding activity, signal transduction activity, and the like. Here, "substantially the same quality" means that the property is qualitatively (for example, physiologically, or pharmacologically) the same. Therefore, preferably activity of the protein of the present disclosure is equivalent, however, a quantitative factor such as degree of these activities (for example, from about 0.01 to about 100 times, preferably from about 0.1 to about 10 times, and more preferably from 0.5 to 2 times) or molecular weight of protein, and the like may be different.

Measurement of activity such as ligand bonding activity or signal transduction activity may be performed in accordance with a known method per se. For example, it may be performed in accordance with a method used in a screening method for a compound inhibiting activity of protein or a salt thereof, to be used in accordance with the present disclosure described later, or the like.

In addition, the protein to be used in accordance with the present disclosure includes, for example, what is called mutein such as a protein containing (i) an amino acid sequence where one or two or more (for example, about 1 to 50, preferably about 1 to 30, more preferably about 1 to 10, and still more preferably several pieces (1 to 5, 4, 3 or 2)) amino acids are lost in the amino acid sequence represented by SEQ ID No: 2, SEQ ID No: 4 or SEQ ID No: 6; (ii) an amino acid sequence where one or two or more (for example, about 1 to 50, preferably about 1 to 30, more preferably about 1 to 10 and still more preferably several pieces (1 to 5, 4, 3 or 2)) amino acids are added in the amino acid sequence represented by SEQ ID No: 2, SEQ ID No: 4 or SEQ ID No: 6; (iii) an amino acid sequence where one or two or more (for example, about 1 to 50, preferably about 1 to 30, more preferably about 1 to 10 and still more preferably several pieces (1 to 5, 4, 3 or 2)) amino acids are inserted in the amino acid sequence represented by SEQ ID No: 2, SEQ ID No: 4 or SEQ ID No: 6; (iv) an amino acid sequence where one or two or more (for example, about 1 to 50, preferably about 1 to 30, more preferably about 1 to 10 and still more preferably several pieces (1 to 5, 4, 3 or 2)) amino acids are substituted with other amino acids in the amino acid sequence represented by SEQ ID No: 2, SEQ ID No: 4 or SEQ ID No: 6; or (v) an amino acid sequence in combination thereof.

In the case where an amino acid sequence is inserted, lost or substituted as described above, the inserted, lost or substituted position is not particularly limited.

A preferable example of the protein to be used in accordance with the present disclosure includes, for example, human EVI1 (Refseq Accession No. NP_001098547) containing the amino acid sequence represented by SEQ ID No: 2, or a homolog thereof in another mammal (for example, mouse homolog registered as RefSeq Accession No. NP_031989 in GenBank) ; human ITGA6 (Refseq Accession No. NP_001073286) containing the amino acid sequence represented by SEQ ID No: 4, or a homolog thereof in another mammal (for example, mouse homolog registered as RefSeq Accession No. NP_032423 in GenBank); human ITGB4 (RefSeq Accession No. NP_001005619) containing the amino acid sequence represented by SEQ ID No: 6, or a homolog thereof in another mammal (for example, mouse homolog registered as RefSeq Accession No. NP_001005608 in GenBank), or the like.

In this specification, protein and peptide are described in accordance with the customary way of peptide description that N-terminal (amino-terminal) is described at the left end, and C-terminal (carboxyl-terminal) is described at the right end. The protein to be used in accordance with the present disclosure including the protein containing the amino acid sequence represented by SEQ ID No: 2, SEQ ID No: 4, or SEQ ID No: 6, may be any of the proteins where C-terminal is carboxyl group (-COOH), carboxylate (-COO⁻), amide (-CONH₂), or ester (-COOR).

Here, when C-terminal is ester (-COOR), as R group, for example, C₁₋₆ alkyl group such as a methyl, an ethyl, a n-propyl, an isopropyl, a n-butyl group; C₃₋₈ cycloalkyl group such as a cyclopentyl, a cyclohexyl; C₆₋₁₂ aryl group such as a phenyl, an α-naphthyl; C₇₋₁₄ aralkyl group such as phenyl-C₁₋₂ alkyl group such as a benzyl, a phenethyl, or α-naphthyl-C₁₋₂ alkyl group such as an α-naphthylmethyl; a pivaroyloxymethyl group, and the like can be used.

When the protein to be used in accordance with the present disclosure contains carboxyl (or carboxylate) group in another position than the C-terminal, the one where the carboxyl group is amidated or esterified is included in the protein to be used in accordance with the present disclosure. In this case, as the ester, for example, the above-described ester of the C-terminal and the like can be used.

Furthermore, the protein to be used in accordance with the present disclosure also include the one where an amino group of N-terminal amino acid residue (for example, a methionine residue) has been protected by a protecting groups (C₁₋₆ acyl group such as C₁₋₆ alkanoyl such as, for example, a formyl group and an acetyl group); the one where a glutamine residue of N-terminal produced by in vivo cleavage has been converted to pyroglutamic acid; the one where a substituent on a side chain of an amino acid in a molecule (for example, -OH, -SH, an amino group, an imidazole group, an indole group, a guanidino group, and the like) has been protected by a suitable protecting group (for example, C₁₋₆acyl group such as C₁₋₆ alkanoyl such as a formyl group and an acetyl group); or a complex protein such as what is called, glycoprotein where a sugar chain has been bound, and the like.

The partial peptide to be used in accordance with the present disclosure, is a peptide having a partial amino-acid sequence of the above-described protein to be used in accordance with the present disclosure, and may be any type of peptide so long as it has substantially the same quality of activity as that of said protein. Here, "substantially the same quality of activity" means the same as above. In addition, measurement of "substantially the same quality of activity" can be performed in the same way as in the case of the above-described protein to be used in accordance with the present disclosure.

For example, a peptide having an amino-acid sequence containing at least 20 or more, preferably 50 or more, further preferably 70 or more, more preferably 100 or more, and most preferably 150 or more of amino-acid sequences among the amino-acid sequences constituting the protein to be used in accordance with the present disclosure, and the like can be used.

In addition, the partial peptide to be used in accordance with the present disclosure may be the one where 1 or 2 or more (preferably about 1 to 20, more preferably about 1 to 10, further preferably several (1 to 5, 4, 3 or 2 of) amino acids are deleted from the amino-acid sequence thereof; or the one where 1 or 2 or more (preferably about 1 to 20, more preferably about 1 to 10, further preferably several (1 to 5, 4, 3 or 2 of) amino acids are added to the amino-acid sequence thereof; or the one where 1 or 2 or more (preferably about 1 to 20, more preferably about 1 to 10, further preferably several (1 to 5, 4, 3 or 2 of) amino acids are inserted into the amino-acid sequence thereof; or the one where 1 or 2 or more (preferably about 1 to 20, more preferably about 1 to 10, further preferably several (1 to 5, 4, 3 or 2 of) amino acids are substituted by other amino acids.

Also, the partial peptide to be used in accordance with the present disclosure may be any type of a peptide where C-terminal is a carboxyl group (-COOH), a carboxylate (-COO⁻), an amide (-CONH₂), or an ester (-COOR). Here, when C-terminal is an ester (-COOR), R includes the same one as described above for the protein to be used in accordance with the present disclosure. When the partial peptide of the present disclosure has a carboxyl group (or carboxylate) in other position than the C-terminal, those where carboxyl group has been amidated or esterified may be included in the partial peptide of the present disclosure. As the ester group in this case, for example, the same one as the C-terminal ester group, and the like may be used.

Further, the partial peptide of the present disclosure includes, as described for the above-described protein to be used in accordance with the present disclosure, the one where an amino group of N-terminal amino acid residue (for example, a methionine residue) has been protected by a protecting group; the one where a glutamine residue of N-terminal produced by in vivo cleavage has been converted to pyroglutamic acid; the one where a substituent on a side chain of an amino acid in a molecule has been protected by a suitable protecting group; or a complex protein such as what is called, glycoprotein where a sugar chain has been bound, and the like.

The partial peptide to be used in accordance with the present disclosure can be used as an antigen for preparing an antibody.

The protein or the partial peptide thereof may be in a free form or a salt (hereinafter, the same unless otherwise stated). As such salt, a salt with a physiologically acceptable acid (for example, inorganic acid, organic acid) or base (for example, alkaline metal salt), and the like can be used, in particular, a physiologically acceptable acid addition salt is preferable. As such salt, for example, a salt with an inorganic acid (for example, hydrochloric acid, phosphoric acid, hydrobromic acid, sulfuric acid), or an organic acid (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid), and the like can be used.

The protein to be used in accordance with the present disclosure can be produced from the above-described cells or tissues of human or other warm-blooded animals by using the known purification method for protein. Specifically, the protein to be used in accordance with the present disclosure can be prepared, for example, by homogenizing tissues or cells of a mammal in the presence of a surfactant, and subjecting the resultant crude extract fraction of the tissue to chromatography such as reverse phase chromatography, ion-exchange chromatography, affinity chromatography.

The protein or the partial peptide thereof to be used in accordance with the present disclosure can be produced by a known peptide synthesis method.

As the peptide synthesis method, for example, any type of solid-phase synthesis method or liquid-phase synthesis method may be used. That is, the desired protein (peptide) can be produced by condensing a partial peptide or an amino acid which can constitute the protein or the partial peptide thereof to be used in accordance with the present disclosure and a remainder part, and when the product has a protecting group, removing the protecting group. As for the known condensation methods or removal of a protecting group, for example, the following methods described in (i) to (v) are exemplified.
(i) M. Bodanszky and M.A. Ondetti, Peptide Synthesis, Interscience Publishers, New York (1966);
(ii) Schroeder and Luebke, The Peptide, Academic Press, New York (1965) ;
(iii) Nobuo Izumiya, et al., Fundamentals and Experiments of Peptide, Marzen Co., Ltd. (1975);
(iv) Haruaki Yajima and Shunpei Sakakibara, The Course of Biochemistry Experiment 1, Chemistry of Protein IV, 205 (1977);
(v) Editorial supervisor: Haruaki Yajima, The Second Series of Development of Pharmaceuticals, Vol 14., Synthesis of Peptide, Hirokawa-Shoten Co. Ltd.

The thus obtained protein (peptide) can be purified and isolated by using a known purification method. Here, the purification method includes, for example, solvent-extraction, distillation, column-chromatography, liquid-chromatography, recrystalization, and the like.

When the protein (peptide) obtained by the above-described method is in a free form, the protein (peptide) can be converted to an appropriate salt according to a known method or a compatible method thereof, on the contrary, when the protein (peptide) is obtained as a salt, the protein (peptide) can be converted to a free form or another salt according to a known method or a compatible method thereof.

For a synthesis of the protein or the partial peptide thereof to be used in accordance with the present disclosure, or an amide form thereof, a commercially available resin for protein synthesis can commonly be used. Such resin includes, for example, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidemethyl resin, polyacrylamide resin, 4-(2',4'-dimethoxyphenyl-hydroxymethyl)phenoxy resin, 4-(2',4'-dimethoxyphenyl-Fmoc aminoethyl)phenoxy resin and the like. Using these resins, amino acids where a α-amino group and a functional group on a side chain have been appropriately protected are condensed on the resin based on the sequence of the desired protein according to the known various condensation methods. In the last step of the reaction, the protein or the partial peptide is excised from the resin, and the various protecting groups are simultaneously removed, further, the protein or the partial peptide is subjected to a reaction to form an intramolecular disulfide bond in a highly diluted solution, to obtain the desired protein or the partial peptide, or the amide form thereof.

As for the above-described condensation of the protected amino acids, various kinds of activating reagents which can be used for synthesis of protein can be used, in particular, carbodiimides is preferable. As carbodiimides, DCC, N,N'-diisopropylcarbodiimide, N-ethyl-N'-(3-dimethylaminoprolyl)carbodiimide and the like can be used. Activation by these reagents can be performed by adding the protected amino acids directly to a resin together with a racemization-reducing additive (for example, HOBt, HOOBt), or adding to a resin after activating the protected amino acids as a symmetrical acid anhydride, a HOBt-ester or a HOOBt-ester in advance.

A solvent to be used in activation of the protected amino acid or condensation with a resin can be appropriately selected from the known solvents which can be used in the protein condensation reaction. For example, acid amides such as N,N-dimethylformamide, N,N-dimethylacetamide snf N-methylpyrrolidone; halogenated hydrocarbons such as methylene chloride and chloroform; alcohols such as trifluoroethanol; sulfoxides such as dimethylsulfoxide; etheres such as pyridine, dioxane and tetrahydrofuran; nitriles such as acetonitrile and propionitrile; esters such as methyl acetate and ethyl acetate; or an appropriate mixture thereof, and the like can be used. Reaction temperature can be appropriately selected from the known range where the protein bond formation reaction can be carried out, and usually ranges from about -20 to about 50 °C. The activated amino acid derivatives are usually used in excess amount of 1.5 to 4 times. When the condensation is not sufficient from the result of test using ninhydrin reaction, sufficient condensation can be obtained by repeating the condensation reaction without removing the protecting group. When sufficient condensation cannot be obtained even by repeating the reaction, a negative effect on the subsequent reaction can be avoided by acetylating unreacted amino acids using acetic anhydride or acetylimidazole.

Protection of a functional group which should not be involved in the reaction of the raw material and a protecting group therefor, removal of the protecting group, activation of a functional group which is involved in the reaction, and the like can be appropriately selected from the known relevant groups or the known relevant means.

As a protecting group of the amino group of the raw material, for example, Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfenyl, diphenyphosphinothioyl, Fmoc, and the like are used.

Carboxyl group can be protected, for example, by alkyl esterification (for example, linear, branched or cyclic alkyl esterification such as methyl, ethyl, propyl, butyl, t-butyl, cyclopentyl, cyclohexyl, cycloheptyl, cyclooctyl, 2-adamantyl); aralkyl esterification (for example, benzyl ester, 4-nitrobenzyl ester, 4-methoxybenzyl ester, 4-chloro benzyl ester, benzhydryl esterification); phenacyl esterification, benzyloxycarbonylhydrazidation, t-butoxycarbonylhydrazidation; tritylhydrazidation, and the like.

Hydroxyl group of serine can be protected, for example, by esterification or etherification. As a group suitable for this esterification, for example, lower (C₁₋₆) alkanoyl group such as acetyl group; aroyl group such as benzoyl group; a group derived from carbonic acid such as benzyloxycarbonyl group and ethoxycarbonyl group, and the like are used. Also, a group suitable for etherification includes, for example, benzyl group, tetrahydropyranyl group, t-butyl group, and the like.

As a protecting group of phenolic hydroxyl group in tyrosine, for example, Bzl, Cl₂-Bzl, 2-nitrobenzyl, Br-Z, t-butyl, and the like are used.

As a protecting group for imidazole in histidine, for example, Tos, 4-methoxy-2,3,6-trimethylbenzenesulfonyl, DNP, benzyloxymethyl, Bum, Boc, Trt, Fmoc, and like are used.

As a removing (eliminating) method for a protecting group, for example, catalytic reduction in the hydrogen gas flow in the presence of a catalyst such as Pd-black or Pd-carbon; acid treatment with anhydrous hydrogen fluoride, methanesulfonic acid, trifluoromethanesulfonic acid, trifluoroacetic acid, or a mixed solution thereof, and the like; base treatment with diisopropylethylamine, triethylamine, piperidine, piperazine, and the like; reduction with sodium in liquid ammonia, and the like are used. The elimination reaction by the above-described acid treatment is generally carried out at a temperature of about -20°C to about 40°C, and in acid treatment, for example, addition of a cation scavenger such as, for example, anisole, phenol, thioanisol, meta-cresol, para-cresol, dimethylsulfide, 1,4-butanedithiol and 1,2-ethanedithiol is effective. Also, 2,4-dinitrophenyl group to be used as a protecting group for imidazole in hystidine can be removed by thiophenol treatment, formyl group to be used as a protecting group for indole in tryptophane can be removed by acid treatment in the presence of the above-described 1,2-ethanedithiol of 1,4-butanedithiol, as well as by alkali treatment by an aqueous dilute sodium hydroxide solution, an aqueous dilute ammonia solution.

As a raw material having an activated carboxyl group, for example, a corresponding acid anhydride, azide, activated ester [ester with alcohol (for example, pentachlorophenol, 2,4,5-trichlorophenol, 2,4-dinitrophenol, cyanomethyl alcohol, para-nitrophenol, HONB, N-hydroxysuccinimide, N-hydroxyphthalimide, HOBt)], and the like are used. As a raw material having activated amino group, for example, a corresponding phosphoric amide is used.

Another method for obtaining an amidated protein or a partial peptide thereof includes, for example, a method where firstly, α-carboxyl group of carboxyl-terminated amino acid is protected by amidation, then a peptide (a protein) chain is extended to a desired chain length on an amino group side, thereafter, a protein where only the protecting group of α-amino group at the N-terminal of said peptide chain is removed or a partial peptide thereof, and a protein where only the protecting group of carboxyl group at the C-terminal is removed or a partial peptide thereof are produced, and these proteins or peptides are condensed in the mixed solvents as described above. Details of the condensation reaction is the same as above. By purifying the protected protein or the peptide thereof obtained by the condensation, and then removing all of the protecting groups by the above-described method, the desired crude protein or peptide can be obtained. This crude protein or peptide is purified by fully using various kinds of the known purification means, and lyophilizing the main fraction, an amidated form of the desired protein or the peptide can be obtained.

As for an ester form of the protein or the peptide, for example, by condensing an α-carboxyl group of carboxyl-terminated amino acid with a desired alcohol to form an amino acid ester, then carrying out the same procedures as in the case of the amide form of the protein or the peptide, an ester form of the desired protein or the peptide can be obtained.

The partial peptide of the protein to be used in accordance with the present disclosure can be produced by cleaving the protein to be used in accordance with the present disclosure by a suitable peptidase.

Further, the protein to be used in accordance with the present disclosure or the partial peptide thereof can be also produced by culturing a transformant containing a polynucleotide encoding the protein or the peptide, and separating / purifying said protein or said partial peptide from the obtained culture. The polynucleotide encoding the protein to be used in accordance with the present disclosure or the partial peptide thereof may be DNA, RNA or DNA/RNA chimera. Preferably, the polynucleotide includes DNA. In addition, said polynucleotide may be double-stranded or single-stranded. In the case of the double-stranded chain, the polynucleotide may be a double-stranded DNA, a double-stranded RNA or a hybrid of DNA : RNA. In the case of the single-stranded chain, the polynucleotide may be a sense strand (i.e. coding strand) or an antisense strand (i.e. noncoding strand).

The polynucleotide encoding the protein to be used in accordance with the present disclosure or the partial peptide thereof includes genomic DNA, genomic DNA library, cDNA derived from all cell [for example, hepatocyte, splenocyte, neurocyte, glial cell, pancreas beta cell, marrow cell, mesangial cell, Langerhans cell, epidermic cell, epithelial cell, goblet cell, endothelial cell, smooth muscles cell, fibroblast, fibrocyte, muscle cell, adipocyte, immune cell (for example, macrophage, T cell, B cell, natural killer cell, mast cell, neutrophil, basophilic leukocyte, eosinocyte, monocyte), megakaryocyte, synovial cell, chondrocyte, osteocyte, osteoblast, osteoclast, breast cell or interstitial cell, or progenitor cell, stem cell, or cancer cell of these cells, or the like] of mammals (for example, human, bovine, monkey, horse, pig, sheep, goat, dog, cat, guinea pig, rat, mouse, rabbit hamster, etc.), or all tissues where these cells exist [for example, brain, each part (an example, olfactory bulb, amaygdala, cerebral basal cell, hippocampal, thalamus, hypothalamus, cerebral cortex, oblongata, cerebellar) of brain, spinal cord, pituitary, stomach, pancreas, kidney, liver, gonad, thyroid, gallbladder, marrow, adrenal, skin, lung, gastrointestinal (an example, large intestine, intestine), blood vessel, heart, thymus, lienal, submandibular gland, peripherals blood, prostate gland, testis, ovarian, placenta, uterus, bone, joint, adipose tissue (an example, white adipose tissue, brown adipose tissue), skeletal muscle etc.], and the like. The genomic DNA and the cDNA encoding the protein to be used in accordance with the present disclosure or the partial peptide thereof can be directly amplified by polymerase chain reaction (hereinafter, also called "PCR method") or RT-PCR method using a reverse transcriptase, using the genomic DNA fraction and the whole RNA or the mRNA fraction prepared by the above-described cell / tissue as a template. Alternatively, the genomic DNA and the cDNA encoding the protein to be used in accordance with the present disclosure or the partial peptide thereof can be cloned, respectively, from the genomic DNA library and the cDNA library prepared by introducing the genomic DNA and the whole RNA, or a fragment of the mRNA prepared from the above-described cell / tissue into an appropriate vector, by colony or plaque hybridization method, PCR method, or the like. The vector to be used in library may be any one of bacteriophage, plasmid, cosmid or phagemid.

The DNA encoding the protein to be used in accordance with the present disclosure may be, for example, a DNA which has a base sequence capable of hybridizing with a DNA having the base sequence represented by SEQ ID No: 1, SEQ ID No: 3 or SEQ ID No: 5 under highly stringent conditions, and encodes a protein which has substantially the same activity as that of a protein having the amino acid sequence represented by SEQ ID No: 2, SEQ ID No: 4 or SEQ ID No: 6.

As the DNA which can hybridize with the base sequence represented by SEQ ID No: 1, SEQ ID No: 3 or SEQ ID No: 5 under highly stringent conditions, for example, a DNA which contains a base sequence having a homology of about 50% or more, preferably about 60% or more, more preferably about 70% or more, further more preferably about 80% or more, particularly preferably about 90% or more, and most preferably about 95% or more to the base sequence represented by SEQ ID No: 1, SEQ ID No: 3 or SEQ ID No: 5, and the like can be used.

Homology of a base sequence in the present specification can be calculated by using, for example, a homology calculation algorism, NCBI BLAST (National Center for Biotechnology Information Basic Local Alignment Search Tool), under the following conditions (expected value = 10; filtering = ON; match score = 1; mismatch score = -3). As other algorithms for determining the homology of a base sequence, the above-described homology calculation algorithms for an amino acid sequence are similarly preferably exemplified.

Hybridization can be carried out by a known method per se, or a similar method, for example, the method described in Molecular Cloning 2nd ed. (J. Sambrook et al., Cold Spring Harbor Lab. Press, 1989), and the like. Also, when commercially available library is used, it can be carried out according to the method described in the attached instruction. More preferably, it can be carried out according to the highly stringent conditions.

Highly stringent conditions mean, for example, the conditions in which sodium concentration is about 19 to about 40 mM, preferably about 19 to about 20 mL, and temperature is about 50 to about 70°C, preferably about 60 to about 65°C. Particularly, the case when sodium concentration is about 19 mM, and temperature is about 65°C is preferable. As a person skilled in the art can appropriately change the salt concentration of hybridization solution, the temperature of hybridization reaction, the probe concentration, the probe length, the mismatched number, the time of hybridization reaction, the salt concentration of washing solution, the washing temperature, and the like, the desired stringency can easily be controlled.

As the DNA which encodes the protein used in accordance with the present disclosure, a DNA having the base sequence represented by SEQ ID No: 1 (Refseq Accession No. NM_001105077), or a homolog thereof in another mammal (for example, mouse homolog registered in GenBank as RefSeq Accession No. NM_007963); a DNA having the base sequence represented by SEQ ID No: 3 (Refseq Accession No. NM_001079818), or a homolog in another mammal (for example, mouse homolog registered in GenBank as RefSeq Accession No. NM_008397); a DNA having the base sequence represented by SEQ ID No: 5 (Refseq Accession No. NM_001005619), or a homolog thereof in another mammal (for example, mouse homolog registered in GenBank as RefSeq Accession No. NM_001005608), and the like are preferably exemplified.

Polynucleotide (for example, DNA) which encodes the partial peptide of the protein used in accordance with the present disclosure may be any of the one which has the base sequence encoding the above-described partial peptide of the protein used in accordance with the present disclosure. Further, it may be any of genome DNA, genome DNA library, cDNA derived from the above-described cell, tissue, cDNA library derived from the above-described cell, tissue, or synthetic DNA.

As the DNA encoding the partial peptide of the protein to be used in accordance with the present disclosure, for example, a DNA which contains a partial base sequence of the base sequence represented by SEQ ID No: 1, SEQ ID No: 3 or SEQ ID No: 5; or a base sequence capable of hybridizing with a polynucleotide containing the base sequence represented by SEQ ID No: 1, SEQ ID No: 3 or SEQ ID No: 5 under highly stringent conditions, and encodes a peptide having substantially the same quality of activity as the protein to be used in accordance with the present disclosure, and the like is used. The DNA capable of hybridizing with the base sequence represented by SEQ ID No: 1, SEQ ID No: 3 or SEQ ID No: 5 has the same meaning as above. In addition, the hybridization method and the highly stringent conditions are the same as described above.

As for means of cloning the DNA encoding the protein to be used in accordance with the present disclosure and the partial peptide thereof (hereinafter, also called "the protein of the present disclosure", in the explanations of cloning and expression of the DNA encoding them), the DNA can be selected by amplifying using a synthetic DNA primer having a part of base sequence encoding the protein of the present disclosure by PCR method, or by hybridizing with a DNA incorporated in an appropriate vector which has been labeled using a DNA fragment or a synthetic DNA encoding a part or a whole of the region of the protein of the present disclosure. The hybridization can be carried out, for example, according to the method described in Molecular Cloning 2nd ed. (J. Sambrook et al., Cold Spring Harbor Lab. Press, 1989) . Further, when a commercially available library is used, the hybridization can be carried out according to the method described in the attached instruction.

Conversion of a base sequence of DNA can be carried out by using PCR, the known kit such as, for example, Mutan (trade mark)-super Express Km (Takara Shuzo Co., Ltd), Mutan (trade mark) -K (Takara Shuzo Co., Ltd), and the like according to the known method per se or the similar method such as ODA-LA PCR method, Gapped duplex method, Kunkel method, and the like, or a compatible method thereto.

The DNA encoding the cloned protein can be used as it is, or if desired after being digested by a restriction enzyme or adding a linker, depending on the purpose. Said DNA may have ATG as a translation initiation codon at the 5'-terminal side thereof, or TAA, TGA, or TAG as a translation stop codon at the 3'-terminal side thereof. These translation initiation codon and translation stop codon can be added by using an appropriate synthetic DNA adaptor.

Expression vector of the protein of the present disclosure can be produced, for example, by cutting out the desired DNA fragment from the DNA encoding the protein of the present disclosure, and connecting said DNA fragment in the down-stream of a promoter in an appropriate expression vector.

As the vector, a plasmid derived from Bacillus coli (for example, pBR322, pBR325, pUC12, pUC13); a plasmid derived from Bacillus subtilis (for example, pUB110, pTP5, pC194); a plasmid derived from yeast (for example, pSH19, pSH15) ; a bacteriophage such as λ phage; an animal virus such as retrovirus, vaccinia virus, baculovirus; as well as pA1-11, pXT1, pRc/CMV, pRc/RSV, pcDNAI/Neo, and the like can be used.

Promoter to be used in accordance with the present disclosure may be any type of promoter, so long as it suitably corresponds to the host to be used in the gene expression. For example, when animal cell is used as a host, the promoter includes SRα promoter, SV40 promoter, LTR promoter, CMV promoter, HSV-Tk promoter, and the like.

Among them, CMV (cytomegalo virus) promoter, SRα promoter and the like are preferably used. When host is Escherichia bacteria, trp promoter, lac promoter, recA promoter, λPL promoter, lpp promoter, T7 promoter, and the like are preferable, and when host is Bacillus bacteria, SPO1 promoter, SPO2 promoter, penP promoter, and the like are preferable, and when host is yeast, PHO5 promoter, PGK promoter, GAP promoter, ADH promoter, and the like are preferable. When host is insect cell, polyhedrin promoter, P10 promoter, and the like are preferable.

As expression vector, besides those described above, if desired, the one having enhancer, splicing signal, poly-A addition signal, selective marker, SV40 replicated origin (hereinafter, also called "SV40ori"), and the like can be used. Selective marker includes, for example, dihydrofolic acid reductase (hereinafter, also called "dhfr") gene [methotrexalate(MTX) resistant], ampicillin resistant gene (hereinafter, also called "Ampr"), neomycin resistant gene (hereinafter, also called "Neor", G418 resistant), and the like. In particular, when dhfr gene is used as a selective marker by using Chinese hamster cell lacking the dhfr gene, the desired gene can be selected by the culture media containing no thymidine.

In addition, if desired, a signal sequence suitable for a host is added in the N-terminal side of the protein of the present disclosure. When host is Escherichia bacteria, PhoA-signal sequence, OmpA-signal sequence, and the like can be used; when host is Bacillus bacteria, α-amylase-signal sequence, subtilisin-signal sequence, and the like can be used, when host is yeast, MFα-signal sequence, SUC2-signal sequence, and the like can be used, when host is animal cell, an insulin-signal sequence, α-interferon-signal sequence, antibody molecule-signal sequence, and the like can be used, respectively.

By using the vector having DNA which encoding the protein of the present disclosure constructed in this way, transformant can be produced. As the host, for example, Escherichia bacteria, Bacillus bacteria, yeast, insect cell, insect, animal cell, and the like can be used.

As specific examples of Escherichia bacteria, for example, Escherichia coli K12-DH1 [(Proc. Natl. Acad. Sci. USA, 60, 160(1968)], JM103 [Nucleic Acids Research, 9, 309 (1981)], JA221 [Journal of Molecular Biology, 120, 517(1978)], HB101 [Journal of Molecular Biology, 41, 459(1969)], C600 [Genetics, 39, 440(1954)], and the like can be used.

As Bacillus bacteria, for example, Bacillus subtilis MI114 [Gene, 24, 255(1983)], 207-21 [Journal of Biochemistry, 95, 87(1984)], and the like can be used.

As yeast, for example, Saccharomyces cerevisiae AH22, AH22R-, NA87-11A, DKD-5D, 20B-12, Schizosaccharomyces pombe NCYC1913, NCYC2036, Pichia pastoris KM71, and the like can be used.

As insect cell, for example, when virus is AcNPV, cell line derived from larva of cabbage armyworm (Spodoptera frugiperda cell; Sf cell), MG1 cell derived from midgut of Trichoplusia ni, High Five TM cell derived from egg of Trichoplusia ni, cell derived from Mamestra brassicae, or cell derived from Estigmena acrea, and the like are used. When virus is BmNPV, cell line derived from silkworm (Bombyx mori N cell; BmN cell), and the like can be used. As said Sf cell, for example, Sf9 cell (ATCC CRL1711), Sf21 cell [those described above: Vaughn, J.L. et al., In Vivo, 13, 213-217(1977)], and the like can be used.

As insect, for example, larva of silkworm, and the like can be used [Maeda et al., Nature, 315 vol., 592(1985)].

As animal cell, for example, ape cell COS-1, COS-3, COS-7, Vero; Chinese hamster ovary cell (hereinafter, also called "CHO cell); dhfr gene defect CHO cell (hereinafter, also called CHO (dhfr-) cell) ; mouse L cell; mouse AtT-20; mouse myeloma cell; mouse ATDC5 cell; rat GH3; human FL cell; human 293 cell; human HeLa cell, and the like can be used.

Transformation of an Escherichia bacterium can be carried out, for example, according to the method described in Proc. Natl. Acad. Sci. USA, 69, 2110(1972) or Gene, 17, 107(1982) and the like.

Transformation of a Bacillus bacterium can be carried out, for example, according to the method described in Mol. Gen. Genet., 168, 111(1979) and the like.

Transformation of yeast can be carried out, for example, according to the method described in Meth. Enzymol., 194, 182-187(1991), Proc. Natl. Acad. Sci. USA, 75, 1929(1978), and the like.

Transformation of insect cell or insect can be carried out, for example, according to the method described in Bio/Technology, 6, 47-55(1988), and the like.

Transformation of animal cell can be carried out, for example, according to the method described in Cell Engineering additional volume 8, New Cell Engineering Experiment Protocol, 263-267(1995) (published by Shujunsha Co., Ltd.), Virology, 52, 456(1973), and the like.

Transformant transformed by the expression vector containing the DNA encoding the protein can be obtained in this way.

When a transformant where host is an Escherichia bacterium or a Bacillus bacterium is cultured, a medium to be used for culture is suitably liquid medium, in which carbon source, nitrogen source, inorganic material and other material necessary for growth of said transformant are contained. The carbon source includes, for example, glucose, dextrin, soluble starch, sucrose, and the like; the nitrogen source includes, for example, inorganic or organic materials such as ammonium salts, nitrates, corn steep liquor, peptone, casein, meat extract, soymeal, potato extracted liquid, and the like; and the inorganic materials include, for example, calcium chloride, sodium dihydrogen phosphate, magnesium chloride, and the like. In addition, yeast extract, vitamins, growth promoting factor, and the like may be added. Desirably, pH of the medium is about 5 to about 8.

As a medium to culture an Escherichia bacterium, for example, M9 medium containing glucose and casamino acid [Miller, Journal of Experiments in Molecular Genetics, 431-433, Cold Spring Harbor Laboratory, New York 1972] is preferable. Here, an agent such as, for example, 3β-indolylacrylic acid can be added, in order to facilitate the promoter to work effectively, if necessary.

When host is an Escherichia bacterium, culture is usually carried out at about 15 to about 43 °C for about 3 to about 24 hours, and if necessary, ventilation or agitation can be added.

When host is a Bacillus bacterium, culture is usually carried out at about 30 to about 40 °C for about 6 to about 24 hours, and if necessary, ventilation or agitation can be added.

When a transformant in which host is a yeast is cultured, medium includes, for example, Burkholder minimum medium (Proc. Natl. Acad. Sci. USA, 77, 4505(1980)) and SD medium containing 0.5% casamino acid (Proc. Natl. Acad. Sci. USA, 81, 5330(1984)). Preferably pH of medium is adjusted at about 5 to about 8. Culture is usually carried out at about 20 to about 35 °C for about 24 to about 72 hours, and if necessary, ventilation or agitation can be added.

When a transformant in which host is an insect cell is cultured, as a medium, Grace's Insect Medium (Nature, 195, 788 (1962)) added appropriately with an additive such as inactivated 10% bovine serum, and the like can be used. Preferably, pH of medium is adjusted at about 6.2 to about 6.4. Culture is usually carried out at about 27 °C for about 3 to about 5 days, and if necessary, ventilation (for example, 5% CO₂) or agitation is added.

When a transformant in which host is an animal cell is cultured, as a medium, for example, MEM medium containing about 5 to about 20% of fetal bovine serum [Science, 122, 501(1952)] ; DMEM medium [Virology, 8, 396(1959)]; RPMI 1640 medium [J. Am. Med. Assoc., 199, 519(1967)]; 199 medium [Proc. Soc. Biol. Med., 73, 1 (1950)], and the like can be used. Preferably, pH is adjusted at about 6 to about 8. Culture is usually carried out at about 30 to about 40°C for about 15 to about 60 hours, and if necessary, ventilation or agitation is added.

As described above, the protein of the present disclosure can be produced in a cell, in a cell membrane, or outside a cell wall of the transformant.

Separation and purification of the protein of the present disclosure from the above-described culture material can be carried out, for example, by the following method.

When the protein of the present disclosure is extracted from cultured fungus body or cell, a method where after culturing, collecting fungus body or cell by a known method, suspending in an appropriate buffer solution, and destructing the fungus body or cull by ultrasonic wave, lysozyme and/or freeze-thaw, and the like, crude extract of the protein is obtained by centrifugation or filtration, and the like are appropriately used. In the buffer solution, protein denaturant such as urea and guanidine hydrochloride, or a surfactant such as Triton X-100 (trade mark) may be contained. When the protein is excreted in the culture fluid, fungus body or cell and supernatant are separated by a known method per se after culture, and supernatant is collected.

Purification of the protein of the present disclosure contained in the culture supernatant or the extraction liquid collected in such way can be carried out by appropriately combining the known separation and purification methods by themselves. The known separation and purification methods includes a method utilizing solubility such as salting-out, solvent precipitation method, and the like; a method utilizing mainly a difference in molecular weight such as dialysis method, ultrafiltration method, gel filtration method, and SDS-polyacrylamide gel electrophoresis, and the like; a method utilizing a difference in electric charge such as ion-exchange chromatography, and the like; a method utilizing specific affinity such as affinity chromatography and the like; a method utilizing a difference in hydrophobicity such as reversed phase high-performance liquid chromatography and the like; a method utilizing a difference in isoelectric point such as isoelectric focusing; and the like.

When the protein of the present disclosure was obtained as free form, it can be converted to salt according to the known method per se or the similar method, on the contrary, when it was obtained as salt, it can be converted to free form or the other salt according to the known method per se or the similar method.

Further, by applying an appropriate protein-modifying enzyme to the protein produced by the recombinant before or after the purification, a modification can be optionally added thereto or a polypeptide thereof can be partially eliminated. As the protein-modifying enzyme, for example, trypsin, chymotrypsin, argynyl-end peptidase, protein kinase, glycosidase, and the like can be used.

Presence of the protein of the present disclosure produced in this way can be measured by enzyme immnoassay or Western Blotting using a specific antibody, and the like.

Furthermore, the protein to be used in accordance with the present disclosure or the partial peptide thereof can also be synthesized by using an RNA corresponding to a DNA encoding it as a template, through in vitro translation using a cell-free protein translation system which is composed of rabbit reticulocyte lysate, wheat germ lysate, Bacillus coli lysate, and the like. Alternatively, it can be further synthesized by using a cell-free transcription / translation system containing RNA polymerase, and by using a DNA encoding calmodulin or the partial peptide thereof as template. As for the cell-free protein (transcription /) translation system, a commercially available system can be used, or the system can be prepared by a known method per se, specifically, Bacillus coli extract can be prepared according to the method described in Pratt J.M. et al., "Transcription and Translation", Hames B.D. and Higgins S.J. edited, IRL Press, Oxford 179-209 (1984), and the like. A commercially available cell lysate includes a lysate derived from E. coli such as E. coli S30 extract system (produced by Promega Corp.), RTS 500 Rapid Translation System (produced by Roche Ltd.), and the like; a lysate derived from rabbit reticulocyte such as Rabbit Reticulocyte Lysate system (produced by Promega Corp.), and the like; and further a lysate derived from wheat germ such as PROTEIOS (trade mark) (produced by TOYOBO Co., Ltd.), and the like. Among them, the one of using the wheat germ lysate is suitable. As a method for preparing the wheat germ lysate, for example, the method described in Johnston F.B. et al., Nature, 179, 160-161(1957), or Erickson A.H. et al., Meth. Enzymol., 96, 38-50(1996), and the like can be used.

A system or an apparatus for synthesizing the protein includes a batch method (Pratt, J.M. et al. (1984) as described above); a continuous cell-free protein synthesizing system where amino acid, energy source, etc. are continuously supplied to the reaction system (Spirin A.S. et al., Science, 242, 1162-1164(1988)) ; a dialysis method (Kigawa et al., the 21st Annual Meeting of the Molecular Biology Society of Japan, WID6); or a double layer method (PROTEIOS (trade mark) Wheat germ cell-free protein synthesis core kit instruction: produced by TOYOBO Co., Ltd.), and the like. Further, a method where RNA as a template, amino acid, energy source, and the like are supplied to the synthesis reaction system as needed, and synthesis product and decomposed material are discharged as needed (JP-A-2000-333673), and the like can be used.

The antibody to the protein to be used in accordance with the present disclosure or the partial peptide thereof (hereinafter, also called "the antibody of the present disclosure") may be any one of polyclonal antibody or monoclonal antibody, so long as the antibody can recognize the protein to be used in accordance with the present disclosure or the partial peptide thereof. Isotype of the antibody is not particularly limited, but IgG, IgM or IgA is preferable, and IgG is particularly preferable.

In addition, the antibody of the present disclosure is not particularly limited, so long as it has at least complementarity determining region (CDR) to recognize specifically the target antigen and bind thereto. The antibody may include, besides a complete antibody molecule, for example, a fragment such as Fab, Fab', F(ab')₂, and the like; a conjugate molecule produced by genetic engineering such as scFv, scFv-Fc, minobody, diabody, and the like; or a derivative thereof modified by a molecule having the protein stabilizing action such as polyethylene glycol (PEG), and the like; and the like.

The antibody to the protein to be used in accordance with the present disclosure or the partial peptide thereof (hereinafter, in the explanation of antibody, these are also called "the protein of the present disclosure") can be produced according to the known production method for antibody or antiserum per se.

Hereinafter, a method for preparing an immunogen for the antibody of the present disclosure and a method for producing said antibody will be explained.

### (1) Preparation of antigen

As the antigen to be used for preparing the antibody of the present disclosure, any one of the above-described protein of the present disclosure or the partial peptide thereof, a (synthetic) peptide having one kind or 2 or more kinds of antigen-determining group same as the protein of the present disclosure, or the like can be used (hereinafter, these are simply called "the antigen of the present disclosure").

The protein of the present disclosure or the partial peptide thereof is produced, as described above, for example, by (a) preparation from a tissue of a warm-blooded animal such as human, ape, rat, mouse, chicken, and the like using a known method or a compatible method; (b) chemical synthesis by a known peptide synthesis method using peptide-synthesizer and the like; (c) culture of a tranfectant containing a DNA encoding the protein of the present disclosure or the partial peptide thereof; or (d) biochemical synthesis by using a cell-free transcription / translation system and using a nucleic acid encoding the protein of the present disclosure or the partial peptide thereof as a template.
(a) When the protein of the present disclosure is prepared from a tissue or a cell of a warm-blooded animal, after tissue or cell is homogenized, the crude fractionated material (for example, membrane fraction, soluble fraction) as it is can be used as an antigen. Alternatively, extraction is carried out using acid, surfactant, alcohol, or the like, then the protein can be purified and isolated from the resultant extraction liquid by combining salting-out, dialysis, gel-filtration, and chromatography such as reverse phase chromatography, ion-exchange chromatography and affinity chromatography. The obtained protein of the present disclosure can be used as an immunogen as it is, or a partial peptide thereof is prepared by a limited degradation using peptidase and the like, to be used as an immunogen.
(b) When the antigen of the present disclosure is chemically prepared, as said synthetic peptide, for example, the one having the same structure as that of the protein of the present disclosure purified from the natural material by using the above-described method (a), specifically, a peptide containing 1 kind or 2 or more kinds of the same amino acid sequence as the amino acid sequence composed of 3 or more, preferably 6 or more amino acids at an optional position in the amino acid sequence of said protein, and the like can be used.
(c) When the antigen of the present disclosure is produced using a transformant containing a DNA, said DNA can be produced according to a known cloning method [for example, the method described in Molecular Cloning 2nd ed. (J. Sambrook et al.. Cold Spring Harbor Lab. Press, 1989), and the like] . Said cloning method includes (1) a method where a DNA encoding said antigen is isolated from cDNA library according to a hybridization method, by using DNA-probe which is designed based on the gene sequence encoding the protein of the present disclosure; (2) a method where a DNA encoding said antigen is prepared by using a DNA-primer which is designed based on the gene sequence encoding the protein of the present disclosure, and using cDNA as a template by PCR method, and said DNA is inserted into an expression vector which is compatible with the host, and the like. The desired antigen can be obtained by culturing the transformant obtained by transforming the host with said expression vector in an appropriate medium.
(d) When the cell-free transcription / translation system is used, a method where after mRNA is synthesized using a transcription reaction liquid containing RNA polymerase which is compatible with said promoter and substrate (NTPs), and using an expression vector to which a DNA encoding the antigen prepared by the same method as in the above (c) has been inserted (for example, an expression vector where said DNA is under the control of T7, SP6 promoter, and the like) as a template, and translation reaction is carried out by using said mRNA as a template, and using a known cell-free translation system (for example, extraction liquid from Bacillus coli, rabbit reticulocyte, wheat germ and the like), and the like can be exemplified. By adjusting salt concentration appropriately, transcription reaction and translation reaction can be carried out in the same reaction liquid collectively.

As the immunogen, a complete molecule of the protein of the present disclosure or a peptide having the partial amino acid sequence thereof can be used. The partial amino acid sequence includes, for example, those composed of 3 or more, preferably 4 or more, more preferably 5 or more, and further more preferably 6 or more continuous amino acid residues. Alternatively, said amino acid sequence includes, for example, those composed of 20 or less, preferably 18 or less, more preferably 15 or less, and further more preferably 12 or less continuous amino acid residues. A part of these amino acid residues (for example, 1 to several residues) may be substituted by a replaceable group (for example, Cys residue, hydroxyl group, and the like). The peptide to be used as an immunogen has an amino acid sequence containing 1 to several of such partial amino acid sequence.

Alternatively, a cell of warm-blooded animal expressing the protein of the present disclosure itself can be directly used as the antigen of the present disclosure. As the cell of warm-blooded animal, a natural cell as described in the above item (a), or a cell transformed by the method as described in the above item (c) can be used. As the host to be used for the transcription, any one of the cell collected from human, ape, rat, mouse, hamster, chicken, and the like may be used, and HEK293, COS7, CHO-K1, NIH3T3, Balb3T3, FM3A, L929, SP2/0, P3U1, B16, P388, or the like is preferably used. The natural warm-blooded animal cell or the transformed warm-blooded animal cell expressing the protein of the present disclosure can be injected to an immunized animal in a suspended state in a culture medium to be used in tissue culture (for example, RPMI1640), or buffer solution (for example, Hanks' Balanced Salt Solution; HBSS) . As the immunization method, any method may be used so long as the method can promote production of the antibody, and intravenous injection, intraperitoneal injection, intramuscular injection, subcutaneous injection, or the like is preferably used.

As for the antigen of the present disclosure, when the antigen has an immunogenicity, insolubilized antigen can be directly immunized. However, when an antigen having a low molecular weight (for example, a molecular weight less than about 3000) and only 1 to several antigen-determining groups (i.e. the partial peptide of the protein of the present disclosure) is used, such antigen can be immunized as a complex where the antigen has been bound or adsorbed to an appropriate carrier, because the antigen is usually a hapten molecule having low immunogenicity. As the carrier, a natural or a synthetic polymer can be used. As the natural polymer, a serum albumin of a mammal such as, for example, bovine, rabbit, human, and the like; thyroglobulin of a mammal such as, for example, bovine, rabbit, and the like; ovalbumin of, for example, chicken; hemoglobin of a mammal such as, for example, bovine, rabbit, human, sheep, and the like; keyhole limpet hemocyanin (KLH); and the like can be used. The synthetic polymer includes various kinds of latexes of polymers or copolymers such as, for example, polyamino acids, polystyrenes, polyacrylics, polyvinyls, polypropylenes, and the like.

As for a mixing ratio of said carrier and the hapten, any kind of carrier may be bound or adsorbed in any ratio, so long as an antibody to the antigen bound or adsorbed to the carrier is produced efficiently. Usually a complex where the above-described natural or synthetic polymer carrier commonly used in preparing an antibody to a hapten is bound or adsorbed in a weight ratio of 0.1 to 100 of carrier based on 1 of hapten, can be used.

In addition, various condensing agents can be used for coupling of the hapten and the carrier. For example, diazonium compounds such as bisdiazotized benzidine cross-linking tyrosine, histidine and tryptophan; dialdehyde compounds such as glutaraldehyde and diisocyanate compounds such as toluene-2,4-diisocyanate cross-linking amino groups each other; dimaleimide compounds such as N,N'-o-phenylenedimaleimide cross-linking thiol groups each other; maleimide active ester compounds cross-linking amino group and thiol group; carbodiimide compounds cross-linking amino group and carboxyl group; and the like are advantageously used. Further, when amino groups are cross-linked each other, after introducing a thiol group to one amino group by reacting with an active ester reagent having dithiopyridyl group (for example, 3-(2-pyridyldithio)propionic acid N-succinimidyl (SPDP), and the like) then reducing, and introducing a maleinimide group to the other amino group using a maleinimide active ester reagent, both groups can be reacted each other.

### (2) Production of monoclonal antibody

### (a) Production of monoclonal antibody producing cell

The antigen of the present disclosure is administered to a site where the antibody can be produced of a warm-blooded animal by an administration means such as, for example, intraperitoneal infusion, intravenous infusion, hypodermic injection, endermic injection, and the like, per se or together with carrier or diluent. In the administration, a complete Freund's adjuvant or an incomplete Freund's adjuvant may be administered to enhance the antibody producibility. Administration is usually carried out once per 1 to 6 weeks, and 2 to 10 times in total. The warm-blooded animal to be used includes, for example, ape, rabbit, dog, guinea pig, mouse, rat, hamster, sheep, goat, donkey and chicken. To avoid the problem of producing anti-Ig antibody, the same kind of mammal as the target to be administered is preferably used, and to produce the monoclonal antibody, generally mouse and rat are preferably used.

Since it is ethically difficult to carry out the artificial immune sensitization for human, when the antibody of the present disclosure is intended to be administered to human, human antibody can be preferably obtained by (i) a method where the human antibody is obtained by immunizing a human antibody producing animal (for example, mouse) produced according to the method described later; (ii) a method where a chimeric antibody, a humanized antibody or a complete human antibody is produced according to the method described later; or (iii) a method where human antibody is produced by combining extracorporeal immune method and cell immortalization by virus, human-human (or mouse) hybridoma producing technology, and phage display method, and the like. Further, since the extracorporeal immune method is capable of obtaining an antigen for an antigen where antibody production is inhibited by the common immunization; antibody can be obtained with an antigen of an amount of ng to µg order; and immunization can be completed within several days; and the like, the method is preferably used even when an antibody derived from nonhuman animal is prepared, as a method for obtaining an antibody to a unstable antigen which is difficult to prepare in large amounts.

The animal cell to be used in the extracorporeal immune method includes a lymphocyte isolated from peripheral blood, spleen, lymph node, and the like of human and the above-described warm-blooded animal (preferably, mouse and rat), preferably B-lymphocyte, and the like. For example, in the case of a mouse cell or a rat cell, spleen is taken out from an about 4 to 12 week-old animal, and the spleen cell is separated, washed with an appropriate culture medium (for example, Dulbecco's modified Eagle medium (DMEM), RPMI1640 medium, ham F12 medium, and the like), and then suspended in a culture medium added with fetal bovine serum (FCS; about 5 to 20%) containing an antigen, and is cultured using a CO₂ incubator or the like for about 4 to 10 days. Antigen concentration is, for example, 0.05 to 5 µg, but is not limited thereto. Preferably thymocyte culture supernatant of an animal of the same lineage (preferably 1 to 2 week-old) is prepared according to the common method, and is added to the culture medium.

In the extracorporeal immune method of human cell, since it is difficult to obtain the thymocyte culture supernatant, preferably several kinds of cytokines such as IL-2, IL-4, IL-5, IL-6, and the like, as well as adjuvant material (for example, muramyldi peptide, and the like) if necessary, together with an antigen are added to the culture medium to carry out immunization.

When monoclonal antibody is produced, an individual or a cell population in which increase of antibody value is observed is selected from a warm-blooded animal (for example, human, mouse, rat) in which antigen is immunized or animal cells (for example, human, mouse, rat), a spleen or a lymph node is taken out after 2 to 5 days from the last immunization, or cells are recovered after culturing for 4 to 10 days after extracorporeal immunization, to isolate the antibody-producing cell, and by fusing this and myeloma cell, an antibody-producing hybridoma can be prepared. Measurement of the antibody value in serum can be carried out, for example, by reacting labeled antigen and anti-serum, and then determining activity of a labeling agent bound to the antibody.

The myeloma cell is not particularly limited so long as it can produce a hybridoma which secretes a large amount of antibody, but a myeloma cell which does not produce or secrete antibody per se is preferable, and a myeloma cell having a high cell fusion efficiency is more preferable. Also, to facilitate the selection of hybridoma, it is preferable to use a strain sensitive to HAT (hypoxanthine, aminopterin and thymidine). For example, a mouse myeloma cell includes NS-1, P3U1, SP2/0, AP-1, and the like; a rat myeloma cell includes R210.RCY3, Y3-Ag1.2.3, and the like; and a human myeloma cell includes SKO-007, GM1500-6TG-2, LICR-LON-HMy2, UC729-6 and the like.

Fusion operation can be carried out according to a known method, for example, Koehler and Milstein's method [Nature, 256, 495(1975)]. Fusion promoter includes polyethylene glycol (PEG), Sendai virus, and the like, and preferably PEG and the like are used. Molecular weight of PEG is not particularly limited, and PEG 1000 to PEG 6000 having low toxicity and comparatively low viscosity are preferable. As concentration of PEG, for example, about 10 to 80%, preferably about 30 to 50% is exemplified. As a solution for diluting PEG, serum-free medium (for example, RPMI1640), complete medium containing about 5 to 20% of serum, various buffer solutions such as phosphate buffered saline (PBS), TRIS buffer, and the like can be used. If desired, DMSO (for example, about 10 to 20%) can be added. As pH of the fusion solution, for example, about 4 to 10, preferably about 6 to 8 can be included.

Preferable ratio of a number of the antibody-producing cell (spleen cell) and a number of the myeloma cell is usually about 1:1 to 20:1, and an efficient cell fusion can be performed by incubating usually at 20 to 40°C, preferably 30 to 37°C for usually 1 to 10 minutes (for example, CO₂ incubation).

The antibody-producing cell strain can be also obtained by infecting a virus which can transform a lymphocyte to the antibody-producing cell and immortalizing said cell. Such virus includes, for example, Epstein-Barr (EB) virus and the like. Most people have been immunized because they have an infection experience with this virus as a symptomless infection of infectious mononucleosis. However, when common EB virus is used, since virus particles are produced, an appropriate purification should be carried out. As an EB system having no possibility of virus contamination, it is also preferable to use a recombinant EB virus (for example, defection in a switching gene transferring from latent infection state to lytic infection state, and the like) which is capable of immortalizing B-lymphocyte, but defective in the replicative ability for virus particle.

Since B95-8 cell derived from marmoset secretes EB virus, by using its culture suprernatant, B-lymphocyte can be easily transformed. The antibody-producing B-cell strain can be obtained by culturing this cell, for example, in a medium added with serum and penicillin / streptomycin (P/S) (for example, RPMI1640) or a serum-free medium added with cell growth factor, then separating the culture supernatant by filtration, centrifugation, or the like, and suspending antibody-producing B-lymphocyte therein in an appropriate concentration (for example, about 10⁷ cell/mL), and culturing usually at 20 to 40°C, preferably at 30 to 37°C usually for about 0.5 to 2 hours. When antibody-producing cell of human is provided as a mixed lymphocyte, since most people have T-lymphocyte exhibiting cytotoxicity to EB-virus-infected cell, it is preferable to remove T-lymphocyte in advance, for example, by forming an E-rosette with sheep red blood cell, and the like, to increase a frequency of transformation. In addition, a lymphocyte specific to the target antigen can be selected by mixing sheep red blood cell binding soluble antigen with antibody-producing B-lymphocyte, and separating a rosette by using density gradient such as Percoll and the like. Further, since antigen-specific B-lymphocyte is capped and IgG is not presented on the surface, by adding large excess of antigen, when sheep red blood cell binding anti-IgG antibody is mixed, only B-lymphocyte non-specific to antibody forms a rosette. Therefore, antigen-specific B-lymphocyte can be selected by collecting rosette non-forming layer from this mixture using density gradient such as Percoll and the like.

Human antibody secretory cell which acquired infinite proliferative capacity by transformation can be back-fused with myeloma cell of mouse or human to stably maintain secretory ability for antibody. As the myeloma cell, the same one as above can be used.

Screening and breeding of a hybridoma is usually carried out in a medium for animal cell added with HAT (hypoxanthine, aminopterin and thymidine) and containing 5 to 20% of FCS (for example, RPMI1640), or a serum-free medium added with cell growth factor. Concentrations of hypoxanthine, aminopterin and thymidine are exemplified as about 0.1 mM, about 0.4 µM and about 0.016 mM, and the like, respectively. For selection of a human-mouse hybridoma, ouabain resistance can be used. Since human cell strain is more sensitive to ouabain compared with mouse cell strain, unfused human cell can be excluded by adding about 10⁻⁷ to 10⁻³ M to the medium.

For selection of hybridoma, it is preferable to use a feeder cell or a certain kind of cell culture supernatant. As the feeder cell, an allogeneic cell species which has such a limited life span that the cell assists emersion of hybridoma but dies thereafter, and a cell which can produce growth factor useful for assisting emersion of hybridoma in a large amounts and has a reduced proliferation potential by exposure to radiation, and the like, are used. For example, as a feeder cell of mouse includes spleen cell, macrophage, blood, thymocyte, and the like, and as a feeder cell of human includes peripheral-blood mononuclear cell, and the like. The cell culture supernatant includes, for example, the primary culture supernatants of the above-described various kinds of cells, and the culture supernatants of various kinds of cell strains.

In addition, hybridoma can be selected by reacting with a fused cell by fluorescently labeling an antigen, and then separating a cell binding to the antigen using a fluorescent-activated cell sorter (FACS). In this case, since hybridoma which can produce an antibody to a target antigen can be directly selected, labor for cloning can be significantly reduced.

For cloning of hybridoma producing monoclonal antibody to the target antigen, various methods can be used.

Since aminopterin inhibits many cell functions, it is preferable to remove it from the medium as soon as possible. In the case of mouse or rat, most of myeloma cells die within 10 to 14 days, and hence aminopterin can be removed after 2 weeks from the fusion. However, human hybridoma can be maintained in a medium added with aminopterin usually for 4 to 6 weeks after the fusion. As for hypoxanthine and thymidine, it is desirable to remove after 1 week or more from the removal of aminopterin. That is, in the case of mouse cell, addition or exchange of a complete medium added with hypoxanthine and thymidine (HT) (for example, RPMI1640 added with 10% FCS) is carried out, for example, after 7 to 10 days from the fusion. Visually observable clone emerges after about 8 to 14 days from the fusion. When a diameter of clone becomes about 1 mm, measurement of an amount of antibody in the culture supernatant becomes possible.

Measurement of an amount of antibody can be carried out, for example, by a method where hybridoma culture supernatant is added to a solid phase (for example, micro plate) to which the target antigen or a derivative thereof or a partial peptide thereof (containing the partial amino acid sequence used as an antigen-determining group) is adsorbed directly or together with a carrier, subsequently anti-immunoglobulin (IgG) antibody (an antigen to IgG derived from the same kind of animal as the animal from which original antibody-producing cell is derived is used) labeled with a radioactive material (for example, ¹²⁵I, ¹³¹I, ³H, ¹⁴C); an enzyme (for example, β-galactosidase, β-glucosidase, alkaline phosphatase, peroxidase, malate dehydrogenase); a fluorescent material (for example, fluorescamine, fluorescein isothiocyanate); a luminescent material (for example, luminol, luminol derivative, luciferin, lucigenin), and the like, or protein A are added thereto, to detect an antibody to the target antigen (antigen-determining group) bound to the solid phase; or a method where hybridoma culture supernatant is added to a solid phase to which anti-IgG antibody or protein A is adsorbed, subsequently the target antigen, a derivative thereof or a partial peptide thereof labeled with the same labeling agent as those described above is added thereto, to detect an antibody to the target antigen (antigen-determining group) bound to the solid phase; or the like.

As a cloning method, limiting dilution method is usually used, but cloning using soft agar or cloning using FACS (described above) can be also used. The limiting dilution method can be carried out by the following procedures but is not limited thereto.

The amount of antibody is measured as described above, and positive wells are selected. Feeder cells appropriately selected are added to 96 wells plate in advance. Cells are sucked from antibody-positive wells, and are suspended in a complete medium (for example, 10% FCS and RMPI1640 added with P/S) so as to obtain a density of 30 cells/mL. The medium of 0.1 mL/well (3 cells/well) is added to the well plate in which feeder cells are added, and the residual cell suspension is diluted to 10 cells/mL and seeded to another well (1 cell/well), and further the residual cell suspension is diluted to 3 cells/mL and seeded to another well (0.3 cells/well). Culture is continued for about 2 to 3 weeks until visually observable clone is emerged. The amount of antibody is measured and positive wells are selected, and cloning is repeated again. In the case of human cell, since cloning is comparatively difficult, plate of 10 cells/well is additionally prepared. Monoclonal-antibody-producing hybridoma can be obtained usually by 2 times of sub-cloning, but it is desirable to repeat the cloning for several months at regularly intervals to confirm its stability.

The hybridoma can be cultured either in vitro or in vivo. In vitro culture method includes a method where the monoclonal-antibody-producing hybridoma obtained as described above is cultured by gradually scaling up from well plate while cell density is maintained, for example, at a level of about 10⁵ to 10⁶ cells/mL and FCS concentration is gradually reduced.

In vivo culture method includes, for example, a method where a mouse (a mouse having histocompatibility with parent strain of hybrodoma) is intraperitoneally injected with mineral oil to induce plasmacytoma (MOPC), and after 5 to 10 days, intraperitoneally injected with about 10⁶ to 10⁷ cells of hybridoma, and after 2 to 5 weeks, peritoneal fluid is collected under anesthesia.

### (b)Purification of monoclonal antibody

Separation and purification of the monoclonal antibody can be carried out according to a known method per se, for example, separation and purification method for immunoglobulin [for example, salting-out method, alcohol precipitation method, isoelectric precipitation method, electrophoretic method, adsorption and desorption method by ion exchanger (for example, DEAE, QEAE), ultra-centrifugal method, gel filtration method, specific purification method where only antibody is collected by active adsorbent such as antigen-binding solid phase, protein A, protein G, or the like, and dissociated to obtain antibody, and the like].

As described above, monoclonal antibody can be produced by culturing the hybridoma the method that hybridoma in vivo or in vitro of a warm-blooded animal, and collecting antibody from body fluid thereof or culture material.

When the antibody of the present disclosure is used for prevention / treatment of cancer, it is necessary to examine an extent of antitumor activity of the obtained monoclonal antibody because said antibody need to have antitumor activity. The antitumor activity can be measured by comparing growth of cancer cell, cell adhesion, apoptosis induction, and the like in the presence or absence of the antibody.

Preferably, the antibody of the present disclosure is used as a pharmaceutical agent intending to administer to human. For this reason, the antibody of the present disclosure (preferably, monoclonal antibody) is, when administered to human, an antibody which has a reduced risk of exhibiting antigenecity (specifically, complete human antibody, humanized antibody, mouse-human chimeric antibody, and the like), particularly preferably a complete human antibody. Humanized antibody and mouse-human chimeric antibody can be genetically produced according to the method described below. In addition, complete human antibody can be also produced by the above-described human-human (or mouse) hybridoma. However, in order to provide a large amount of antibody stably and at low cost, it is desirable to produce by using the human-antibody-producing animal as described below (for example, mouse) or phage display method.

### (i) Production of chimeric antibody

In this specification, "chimeric antibody" means an antibody where sequences of variable regions of H chain and L chain (V_{H} and V_{L}) are derived from a certain species of mammal, and sequences of constant regions (C_{H} and C_{L}) are derived from another species of mammal. Sequence of the variable region is preferably the one derived from an animal species by which hybridoma can be easily produced such as, for example, mouse, and the like, and sequence of the constant region is preferably the one derived from a mammal species which is the target of administration.

Production method for chimeric antibody includes, for example, the method described in the specification of USP No. 6331415, or a partially modified method thereof, and the like. Specifically, firstly, mRNA or the whole RNA is prepared according to the common method from the monoclonal-producing hybridoma (for example, mouse-mouse hybridoma) obtained as described above, to synthesize cDNA. Subsequently, a DNA encoding C_{H} and C_{L} is amplified and purified using an appropriate primer (for example, as a sense-primer, an oligo DNA containing base sequences encoding each of N-terminal sequences of V_{H} and V_{L}, and as an antisense-primer, an oligo DNA capable of hybridizing with base sequences encoding N-terminal sequences of C_{H} and C_{L} (see, for example, Bio/Technology, 9: 88-89,1991)), and using said cDNA as a template, according to the common method by PCR. By a similar method, a DNA encoding V_{H} and C_{H} is amplified and purified from RNA which is prepared from a lymphocyte of another mammal (for example, human), and the like by RT-PCR method. V_{H} and C_{H}, V_{L} and C_{L} are connected, respectively, by using a common method, and the resultant chimera H-chain DNA and chimera L-chain DNA are inserted into an appropriate expression vector (for example, a vector containing a promoter having transcriptive activity in CHO cell, COS cell, mouse myeloma cell, and the like (for example, CMV promoter, SV40 promoter, and the like)), respectively. The DNA encoding both chains may be inserted into different vectors, or may be inserted into one vector in tandem. Host cells are transformed by the obtained vector expressing chimera H-chain and chimera L-chain. The host cell includes animal cell, for example, besides the above-described mouse myeloma cell, Chinese hamster ovary (CHO) cell; COS-7 cell derived from ape; Vero cell; GHS cell derived from rat, and the like. For transformation, any method which can be applied to animal cell may be used, but preferably electroporation method, and the like can be exemplified. Chimeric monoclonal antibody can be isolated by culturing for a certain period in a medium suitable for the host cell, recovering culture supernatant, and purifying by the same method as described above. Alternatively, the chimeric monoclonal antibody can be obtained easily and in large amount by using germ line cell of an animal where transgenic technology of bovine, goat, chicken, and the like has been established, and knowhow about mass breeding as livestock (fowl) has been accumulated as a host cell, and producing transgenic animal according to a common method, from milk or egg of the resultant animal. Further, the chimeric monoclonal antibody can be also obtained in large amount by producing transgenic plant using cell of a plant where transgenic technologies of corn, rice, wheat, soybean, and the like have been established, and which is cultivated in large amount as a main crop as a host cell, and using micro-injection to protoplast, electroporation method, particle gun to intact cell method, Ti-vector method, or the like, from the resultant seeds, leaves, or the like.

Fab is obtained by decomposing the obtained chimera monoclonal antibody by papain, and F(ab')₂ is obtained by decomposing the obtained chimera monoclonal antibody by pepsin, respectively.

In addition, scFv can be obtained by connecting DNA encoding mouse V_{H} and V_{L} through an appropriate linker, for example, DNA encoding peptide containing 1 to 40 amino acids, preferably 3 to 30 amino acids, more preferably 5 to 20 amino acids (for example: [Ser-(Gly)ₘ]ₙ or [(Gly)ₘ-Ser]ₙ (m is an integer of 0 to 10, n is an integer of 1 to 5), and the like) . Further, minibody monomer can be obtained by connecting DNA encoding C_{H3} through an appropriate linker, and scFv-Fc can be obtained by connecting DNA encoding the whole length of C_{H} through an appropriate linker. Such DNA which encodes genetically modified (conjugated) antibody molecule can be expressed by microbes such as Bacillus coli and yeast under the control of an appropriate promoter, and therefore, the antibody molecules can be produced in large amount.

By inserting DNAs encoding mouse VH and VL is inserted in the down-stream of one promoter in tandem, and introduced into Bacillus coli, a dimer called as F_{V} can be formed by monocistronic gene-expression. Also, by substituting appropriate amino acids in FR of V_{H} and V_{L} by Cys using a molecular modeling, a dimer called as dsF_{V} can be formed by inter-molecular disulfide bond of both chains.

### (ii) Humanized antibody

"Humanized antibody" in the present specification means an antibody where the sequences of all regions except complementarity determining region (CDR) locating in the variable region (i.e. constant region and framework region (FR) in the variable region) are derived from human, and only the sequence of CDR is derived from another mammal species. As another mammal species, an animal species such as, for example, mouse and the like, which can easily produce hybridoma, is preferable.

Production method for humanized antibody includes, for example, the method described in USP No. 5225539, USP No. 5585089, USP No. 5693,761, and USP No. 5693762 or a partially modified method thereof, and the like. Specifically, similarly to the case of the above-described chimeric antibody, after DNAs encoding V_{H} and V_{L} derived from a mammal species other than human (for example, mouse) are isolated, sequencing is carried out using an automatic DNA sequencer (for example, the one manufactured by Biosystems Co., Ltd., and the like) according to a common method, and the obtained base sequence or the amino acid sequence estimated therefrom is analyzed by using a known antibody sequence database [for example, Kabat database (see: Kabat et al., "Sequences of Proteins of Immunological Interest", US Department of Health and Human Services, Public Health Service, edited by NIH, 5th ed. 1991) and the like], to determine CDR and FR of both chains. A base sequence where CDR code region of the base sequences encoding L-chain and H-chain of human antibody which has the similar FR sequence to the determined FR sequence [for example, human κ type L-chain subgroup I and human H-chain subgroup II or III (see: Kabat, et al.,1991 (described above)] is substituted by a base sequence encoding the determined CDR of a different species is designed, said base sequence is separated to fragments having about 20 to 40 bases, and further, a complementary sequence to said base sequence is separated to fragments having about 20 to 40 bases so as to be alternately overlapped with the above-described fragments. A DNA encoding V_{H} and V_{L} having FR derived from human and CDR derived from another mammal can be constructed by synthesizing each fragment using a DNA synthesizer, and hybridizing them according to a common method, and ligating. In order to transplant CDR derived from another mammal to V_{H} and V_{L} derived from human more quickly and efficiently, it is preferably to use site-directed mutagenesis by PCR. Such method includes, for example, the sequential CDR-transplanting method described in JP-A-5-227970, and the like. By connecting the thus obtained DNA encoding V_{H} and V_{L} with DNA encoding C_{H} and C_{L} derived from human, respectively, by the similar method to the case of the above-described chimeric antibody, and introducing to an appropriate host cell, a humanized-antibody-producing cell or a transgenic animal / plant can be obtained.

Humanized antibody can be also modified to scFv, scFv-Fc, minibody, dsFv, Fv, and the like similar to the chimeric antibody by using the gene-engineering method, and can be produced in microbes such as Bacillus coli and yeast by using an appropriate promoter.

The technology for producing humanized antibody can be applied, for example, to production of monoclonal antibody which can be preferably administered to other animal species in which technology for producing hybridoma has not still been established. For example, animals which have been widely bred as livestock (fowl) such as bovine, pig, sheep, goat, chicken, or pet animals such as dog, cat, and the like can be exemplified as a target.

### (iii) Production of complete human antibody using a human-antibody-producing animal

By introducing a functional human Ig gene to a non-human warm blooded animal where endogenetic immunoglobulin (Ig) gene has been knocked out (KO), and immunizing the animal with an antigen, a human antibody instead of an antibody derived from said animal can be produced. Therefore, by using an animal where a technology for producing hybridoma has been established such as mouse and the like, it becomes possible to obtain a complete human monoclonal antibody by the method similar to the conventional production of the mouse monoclonal antibody. Some of the human monoclonal antibodies which are produced by a human-antibody-producing mouse obtained firstly by cross-breeding a mouse to which mini-genes of H-chain and L-chain of human Ig are introduced by using the conventional transgenic (Tg) technology and a mouse where endogenetic mouse Ig gene is inactivated using the conventional KO technique (see: Immunol. Today, 17, 391-397 (1996)) have already been in clinical stage, however, the production of anti-human Ig human antibody (HAHA) has not yet been reported until now.

After that, Abgenix, Inc. [trade name: XenoMouse (see: Nat. Genet., 15, 146-156 (1997); USP No. 5939598, and the like)] and Medarex Inc. [trade name: Hu-Mab Mouse (see: Nat. Biotechnol., 14, 845-851 (1996) ; USP No. 5545806, and the like)] have produced Tg mouse to which a more larger Ig gene has been introduced by using a vector of yeast artificial chromosome (YAC), and production of human antibody having a wider repertory has become possible. However, since human Ig gene realizes a diversity thereof by a method that, for example, in the case of H-chain, the VDJ exon where about 80 kinds of V fragments, about 30 kinds of D fragments, and 6 kinds of J fragments are variously combined encodes an antigen binding site, the whole length reaches about 1.5 Mb (chromosome 14) in H-chain, about 3 Mb in κL-chain (chromosome 2), and about 1 Mb in λL-chain (chromosome 22). It is desirable to introduce the whole length of each Ig gene in order to reproduce the similar diversified antibody repertory to the case of human in another animal species. However, since a DNA which could be inserted to the conventional gene-introducing vector (plasmid, cosmid, BAC, YAC, and the like) was usually several kb to several hundred kb, it was difficult to introduce the whole length by the conventional technology for producing a transgenic animal where a cloned DNA was injected to a fertilized egg.

Tomizuka et al. (Nat. Genet., 16, 133-143(1997)) have produced a mouse having a complete length of human Ig gene by introducing a natural fragment (hCF) of human chromosome carrying Ig gene to the mouse (transferred chromosome (TC) mouse). That is, firstly, a microcell where 1 to several chromosomes or fragments thereof encapsulated by nuclear membrane is prepared, by treating a human-mouse hybrid cell which has human chromosome where chromosome 14 containing H-chain gene and chromosome 2 containing κL-chain are labeled with, for example, drug resistance marker, or the like with a spindle formation inhibitor (for example, colcemid) for about 48 hours, and then the chromosomes are introduced into a mouse ES cell by micronucleus fusion method. A hybrid ES cell which contains a chromosome having human-Ig gene or fragment thereof is selected using a medium containing an agent, and microinjected to a mouse embryo by the similar method to that in the conventional KO-mouse production. A germ line chimera is selected from the obtained chimeric mouse by a method that coat color is used as a marker, or the like, TC mouse strain (TC (hCF14)) which transfers a fragment of human chromosome 14, and TC mouse strain (TC (hCF2)) which transfers a fragment of human chromosome 2 are established. A mouse where endogenetic H-chain gene and κL-chain gene are knocked out (KO (IgH) and KO (Igκ)) by a common method is produced, and by repeating breeding of these 4 strains, a mouse strain having all of the 4 kinds of genetic modifications (double TC/KO) can be established.

By applying the same method as that for producing a usual mouse monoclonal antibody to the double TC/KO mouse produced as described above, antigen-specific human-monoclonal-antibody-producing hybridoma can be produced. However, since hCF2 containing κL-chain gene is unstable in the mouse cell, there is a problem that hybridoma-obtaining efficiency is lower comparing with the case of a usual mouse.

On the other hand, the above-described Hu-Mab mouse has about 50% of κL-chain genes, and shows diversity of κ-chain equivalent to that of the case when a complete length is contained because the gene has a structure where variable region cluster is doubled (on the other hand, since H-chain gene contains only about 10%, diversity of H-chain is low, and response to an antigen is insufficient), and is inserted into a mouse chromosome by YAC vector (Igκ-YAC), therefore, the gene is stably maintained in the mouse cell. By utilizing this advantage, by producing a mouse stably having hCF14 and Igκ-YAC (trade name, KM mouse) by breeding TC(hCF14) mouse and Hu-Mab mouse, a hybridoma-obtaining efficiency and an antigen-affinity of antibody equivalent to those of a usual mouse can be obtained.

Further, in order to reproduce more completely the diversified antibody repertory in human, it is possible to produce the human-antibody-producing animal to which λL-chain gene is further introduced. Such an animal can be obtained by producing a TC mouse (TC (hCF22)) to which the human chromosome 22 carrying λL-chain gene or a fragment thereof has been introduced by the similar method as described above, and breeding this mouse and the above-described double TC/KO mouse or KM mouse. Alternatively, the animal can be obtained, for example, by constructing a human artificial chromosome (HAC) containing H-chain gene locus and λL-chain gene locus, and introducing this into a mouse cell (Nat. Biotechnol., 18, 1086-1090 (2000)).

When the antibody of the present disclosure is used as a pharmaceutical, the antibody is preferably a monoclonal antibody, but may be polyclonal antibody. When the antibody of the present disclosure is a polyclonal antibody, since use of hybridoma is not necessary, it is possible to produce a larger amount of human antibody at low cost, by producing a human-antibody-producing animal using an animal species where the hybridoma-producing technology has not yet been established but transgenic technology has been established, preferably an ungulate such as bovine, by the same method as described above (see: for example, Nat. Biotechnol., 20, 889-894 (2002)). The obtained human polyclonal antibody can be purified by collecting blood, peritoneal fluid, breast fluid, egg, and the like of the human-antibody-producing animal, preferably breast fluid or egg, and by combining the purification technologies same as described above.

### (iv) Production of a complete human antibody by using phage display human antibody library

Another approach producing the complete human antibody is a method to use phage display. This method has only a few examples of reports on HAHA production in clinical stage, because mutation by PCR may be introduced into other place than CDR. On the other hand, the method has advantages such as no risk of virus infection among different species derived from host animal, infinite specificity of antibody (antibody to an inhibited clone or a sugar chain can be easily produced), and the like.

Production method for the phage display human antibody library includes, for example, the following methods, but is not limited thereto.

The phage to be used is not particularly limited, but usually fibrous phage (Ff bacteriophage) is preferably used. Method showing foreign protein on the surface of phage includes a method where the foreign protein is fused with any one of coat proteins of g3p, g6p to g9p to form a fused protein, and expressed and shown on said coat protein, and a method frequently used is the one where the foreign protein is fused with g3P or g8p at N-terminal side thereof. Phage display vector includes 1) a vector which exhibits all coat proteins exhibited on the phage surface as a fused protein with foreign protein by introducing foreign gene into coat protein gene of phage genome in a fused form; 2) a vector which expresses fused protein and wild type coat protein simultaneously by inserting a gene encoding fused protein separately from wild type coat protein gene; 3) a vector which produces phage particle which expresses fused protein and wild type coat protein simultaneously, by infecting helper phage having wild type coat protein gene to Bacillus coli having phagemid vector having a gene encoding fused protein; and the like. In the case of 1), when a large foreign protein is fused, infecting ability is lost, therefore, type 2) of type 3) are used to produce an antibody library.

Specific vector includes the one described by Holt et al. (Curr. Opin. Biotechnol., 11, 45-449 (2000)). For example, pCES1 (see: J. Biol. Chem., 274,18218-18230(1999)) is a Fab-expression type phagemid vector where DNA encoding κL-chain constant region in the down-stream of g3p signal peptide, DNA encoding CH3 in the down-stream of g3p signal peptide, and g3p code sequence are arranged through His-tag, c-myc tag, and amber termination (TAG). By introducing to Bacillus coli having amber mutation, Fab is exhibited on the g3p coat protein, but by expressing in HB2151 strain having no amber mutaion, a soluble Fab antibody is produced. Also, as a scFv-expression type of phagemid vector, for example, pHEN1 (J. Mol. Biol., 222, 581-597(1991)) and the like are used.

On the other hand, as a helper phage, for example, M13-K07, VCSM13 and the like are exemplified.

In addition, another phage display vector includes a vector which is designed so as to be able to exhibit antibody on the coat protein on the phage surface through a S-S bond between the introduced cysteine residues by connecting a sequence containing a codon encoding cysteine in 3' -terminal of antibody gene and in 5' -terminal of coat protein gene, respectively, and expressing both genes simultaneously and independently (not as a fused protein) (CysDisplay (trade mark) technology of Morphosys AG.), and the like.

Kind of human antibody library includes naive/non-immunized library, synthetic library, immunized library, and the like.

Naive / non-immunized library is a library obtained by obtaining V_{H} and V_{L} genes possessed by normal human by RT-PCR method, and cloning these genes to the above-described phage display vector randomly. Usually, mRNA derived from peripheral blood, marrow, tonsil, and the like of normal human is used as a template. In order to avoid a bias of V gene such as disease history, and the like, the one where only mRNA derived from IgM in which class-switch by antigen sensitization has not occurred is amplified is particularly called as naive library. A representative one includes library of CAT PLC. (see: J. Mol. Biol., 222, 581-597 (1991); Nat. Biotechnol., 14, 309-314 (19969); library of MRC, Inc. (see: Annu. Rev. Immunol., 12, 433-455 (1994)); library of Dyax Corp. (see: J. Biol. Chem., 1999 (described above) ; Proc. Natl. Acad. Sci. USA, 14, 7969-7974 (2000)), and the like.

Synthetic library is the one obtained by selecting functional specific antibody gene in the human B-cell, and substituting a part of antigen-binding region such as, for example, CDR3 of a fragment of V gene by a DNA encoding an appropriate length of random amino acid sequence to make a library. Since a library can be constructed by a combination of V_{H} and V_{L} genes which produces functional scFV or Fab from the beginning, it is said that the library is superior in expression efficiency and stability of antigen. A representative one inculudes HuCAL library of Morphosys AG. (see: J. Mol. Biol., 296, 57-86 (2000)), library of BioInvent AB. (see: Nat. Biotechnol., 18, 852 (2000)); library of Crucell Inc. (see: Proc. Natl. Acad. Sci. USA, 92, 3938 (1995); J. Immunol. Methods, 272, 219-233 (2003)), and the like.

Immunized library is the one obtained by preparing mRNA from lymphocyte collected from a human who has increased antibody value in blood to target antigen such as patients of cancer, autoimmune disease, infectious disease, and the like and a vaccinated human; or lymphocyte from a human to whom target antigen is artificially immunized by the above-described extracorporeal immune method, and the like, in the same manner as in the case of the case of the above-described naive / non-immune library, and amplifying V_{H} and V_{L} genes by RT-PCR method to make a library. Since the desired antibody genes are contained in the library from beginning, the desired antibody can be obtained even from a comparatively small size of library.

Library is better as diversity thereof becomes larger. However, practically in view of a phage number which can be handled by the following panning operation (1011 to 1013 phages) and a phage number required for isolation and amplification of clones by the usual panning, about 10⁸ to 10¹¹ clones are suitable, and a library of about 10⁸ clones can screen an antibody having a Kd value of usually 10⁻⁹ order.

A step to select an antibody to the target antigen by the phage display method is called as panning. Specifically, for example, a phage exhibiting an antigen-specific antibody is condensed by repeating about 3 to 5 times a series of operation of contacting a carrier immobilizing antigen with a phage library, removing non-binding phage by washing, eluting bonded phage from the carrier, amplifying said phage by infecting with Bacillus coli. The carrier to immobilize antigen includes various carriers used in common antigen-antibody reaction or affinity chromatography, for example, micro-plate, tube, membrane, column, bead, and the like constituted of insoluble polysaccharides such as agarose, dextran, cellulose, and the like; synthetic resins such as polystyrene, polyacrylamide, silicone, and the like; or glass, metal, and the like, and further, sensor chip of surface plasmon resonance (SPR), and the like. In the immobilization of antigen, physical adsorption may be used, and a method using chemical bond to be usually used to insolubilize, immobilize protein or enzyme may be also used. For example, biotin- (strepto) avidin system, and the like are preferably used. When endogenous ligand as the target antigen is small molecule such as peptide or the like, it is necessary to pay special attention that a part used as an antigen-determining group is not covered by binding with carrier. For washing non-bonding phage, blocking liquid such as BSA solution or the like (1 to 2 times), PBS containing surfactant such as Tween or the like (3 to 5 times) can be sequentially used. It has been reported that use of citrate buffer solution (pH5) is preferable. For eluting specific phage, acid (for example, 0.1 M hydrochloric acid) is usually used, but it is possible to elute by cleavage with specific protease (for example, a gene sequence encoding trypsin cleavage site can be introduced to the connecting part between antibody gene and coat protein gene. In this case, since wild type coat protein is exhibited on the surface of eluted phage, even though all of the coat proteins are expressed as fused protein, infection to Bacillus coli and amplification are possible); competitive elution by soluble antigen; or reduction of S-S bond (for example, in the above-described CysDisplay (trade mark), after panning, antigen-specific phage can be recovered by dissociating antibody and coat protein using a suitable reductant). When eluted by acid, after neutralized with Tris or the like, eluted phage is infected to Bacillus coli, and after incubation, the phage is recovered by a common method.

When the phage exhibiting antigen-specific antibody is concentrated by panning, these are infected to Bacillus coli, after that seeded on a plate to carry out cloning. The phage is recovered again, and antigen-binding activity is determined by the above-described antibody value determining method (for example, ELISA, RIA, FIA, and the like) or by measurement utilizing FACS or SPR.

As for isolation / purification of antibody from phage clone exhibiting the selected antigen-specific antibody, for example, when a vector where amber termination codon is introduced to the connecting part between antibody gene and coat protein gene is used as a phage display vector, since soluble antibody molecule is produced, and secreted to periplasm or culture medium by infecting said phage to Bacillus coli having no amber mutation (for example, HB2151 strain), the isolation / purification of antibody can be carried out by dissolving cell wall with lysozyme and recovering extracellular fraction using the same purification technology as described above. By introducing His-tag or c-myc tag, it is possible to purify easily using IMAC, anti-c-myc antibody column, or the like. In addition, when cleavage by specific protease is used in panning, since antibody molecule is separated from the phage surface by reacting with said protease, it is possible to purify the desired antibody by carrying out the same purification operation.

Production technology for complete human antibody by using a human-antibody-producing animal and a phage display human antibody library can be applied to produce a monoclonal antibody of other animal species, for example, animals widely bred as livestock (fowl) such as bovine, pig, sheep, goat, chicken, and the like and pet animals such as dog, cat, and the like are exemplified as a target. Since ethical problem for the artificial immune of target antigen is not severe in non-human animal, it is advantageous to use the immune library.

### (3) Production of polyclonal antibody

The polyclonal antibody of the present disclosure can be produced by a known method per se, or a compatible method thereto. For example, the polyclonal antibody can be obtained by using immunizing antigen (a protein or a peptide antigen) itself, or producing a complex of the antigen and a carrier protein, immunizing a warm-blooded animal in the same way as in the production method for the above-described monoclonal antibody, collecting an antibody-containing material to the protein of the present disclosure from said immunized animal, and isolating and purifying the antibody.

Regarding the complex of the immune antigen to be used for immunizing a warm-blooded animal and the carrier protein, any kind of carrier protein and any mixing ratio with hapten may be used to cross-link, so long as an antibody can be efficiently produced to the hapten immunized by being cross-linked to the carrier protein. For example, a method where bovine serum albumin, bovine thyroglobulin, hemocyanin, or the like is coupled in a weight ratio of about 0.1 to about 20, preferably about 1 to about 5 based on 1 of hapten, is used.

In addition, in coupling of hapten and carrier protein, various condensing agents can be used, and glutaraldehyde, carbodiimide, maleimide active ester, active ester reagent containing thiol group or dithiopyridyl group, and the like are used.

Condensation product is administered to a warm-blooded animal in a site where antibody can be produced by the product alone or together with carrier or diluent. In administration, in order to enhance antibody-producing capability, a complete Freund's adjuvant or an incomplete Freund's adjuvant may be administered. Administration is usually carried out in a level of 1 dose per about 1 to about 6 weeks, totally about 2 to about 10 doses.

Polyclonal antibody can be collected from blood, peritoneal fluid, and the like, preferably from blood of a warm-blooded animal immunized by the above-described method.

Measurement of polyclonal antibody value in the antiserum can be carried out in the same way as in the above-described measurement of antibody value in the antiserum. Isolation / purification of polyclonal antibody can be carried out according to the isolation purification method of immunoglobulin in the same way as in the isolation / purification of monoclonal antibody described above.

A polynucleotide containing a base sequence complementary to a target region of the desired polynucleotide, that is, a polynucleotide which can hybridize with the desired polynucleotide, can be said to be "antisense" to said desired polynucleotide.

The antisense polynucleotide having a complementary or substantially complementary base sequence to the base sequence of the polynucleotide encoding the protein of the present disclosure [for example, DNA (hereinafter, in explanation of antisense polynucleotide, DNA encoding the protein of the present disclosure is also called "DNA of the present disclosure")] or a part thereof may be any antisense polynucleotide, so long as the antisense polynucleotide contains a complementary or substantially complementary base sequence to the base sequence of the polynucleotide (for example, DNA) encoding the protein of the present disclosure, and has a function capable of inhibiting expression of said polynucleotide.

The substantially complementary base sequence to the base sequence of the DNA of the present disclosure means, for example, a base sequence having about 70% or more, preferably about 80% or more, more preferably about 90% or more, and most preferably about 95% or more of homology to the complementary base sequence (i.e. a complementary chain of the DNA of the present disclosure) for the over-lapped region thereof. Here, "homology" has the same meaning as in the case of the above-described DNA of the present disclosure. In particular, in all base sequences of the complementary chain of the DNA of the present disclosure, (a) in the case of an antisense polynucleotide intending translation inhibition, an antisense polynucleotide having about 70% or more, preferably about 80% or more, more preferably about 90% or more, most preferably about 95% or more of homology to the complementary chain of the base sequence of the part encoding N-terminal site of the protein of the present disclosure (for example, base sequence near the starting codon, and the like); (b) in the case of an antisense polynucleotide intending RNA decomposition by RnaseH, an antisense polynucleotide having about 70% or more, preferably about 80% or more, more preferably about 90% or more, most preferably about 95% or more of homology to the complementary chain of the DNA of the present disclosure containing intron; are suitable, respectively.

Specifically, an antisense polynucleotide containing a complementary or substantially complementary base sequence to the base sequence represented by SEQ ID No: 1, SEQ ID No: 3, or SEQ ID No: 5, or a part thereof, preferably, for example, an antisense polynucleotide containing a complementary base sequence to the base sequence represented by SEQ ID No: 1, SEQ ID No: 3, or SEQ ID No: 5, or a part thereof are exemplified.

The antisense polynucleotide containing a complementary or substantially complementary base sequence to the base sequence of the DNA of the present disclosure, and the part thereof (hereinafter, also called as "antisense polynucleotide of the present disclosure") can be designed and synthesized according to the base sequence information of DNA encoding the cloned or the determined protein of the present disclosure. Such antisense polynucleotide can inhibit replication or expression of the gene encoding the protein of the present disclosure (hereinafter, also simply called "gene of the present disclosure"). That is, the antisense polynucleotide of the present disclosure can hybridize with the RNA transformed from the gene of the present disclosure (mRNA or initial transcription product), and can inhibit synthesis of mRNA (processing) or function (translation to protein), or can regulate / control the expression of the gene of the present disclosure through an interaction with RNA associated with the gene of the present disclosure. A complementary polynucleotide to the selected sequence of the RNA associated with the gene of the present disclosure, and a polynucleotide which can specifically hybridize with the RNA associated with the gene of the present disclosure are useful to regulate / control the expression of the gene of the present disclosure in vivo and in vitro, and also useful for treatment or diagnosis of disease and the like.

Length of the target region of the antisense polynucleotide of the present disclosure is not particularly limited, so long as translation to the protein of the present disclosure is inhibited as a result of hybridization of the antisense polynucleotide. The length may be the whole sequence or a partial sequence of mRNA encoding said protein, and includes about 10 bases for shorter one and the whole sequence of mRNA or an initial transcription product for longer one. In view of easiness of synthesis and problem of antigenecity, an oligonucleotide consisting of about 10 to about 40 bases, in particular about 15 to about 30 bases is preferable, but is not limited thereto. Specifically, hair pin loop at 5'-terminal, 6-base pair repeat at 5'-terminal, non-translation region at 5'-terminal, translation-initiation codon, protein-encoding region, ORF translation-termination codon, non-translation region at 3'-terminal, palindrome region at 3' -terminal, hair pin loop at 3' -terminal, or the like of the gene of the present disclosure, and the like can be selected as a preferable antisense polynucleotide, but any region of the gene of the present disclosure can be selected as a target. For example, an intron part of said gene can be selected as a target region.

Further, the antisense polynucleotide of the present disclosure is not only the one which inhibits translation to protein by hybridizing with mRNA or an initial transcription product of the protein of the present disclosure, but may be the one which can inhibit transcription of RNA by forming a triplex by binding to the gene of the present disclosure which is a duplex DNA. Or, it may be the one which induce decomposition by RNaseH by forming a DNA:RNA hybrid.

In order to prevent the decomposition by hydrolase such as nuclease, and the like, phosphate residue (phosphate) of each nucleotide constituting antisense polynucleotide may be substituted by, for example, chemically modified phosphate residue such as phosphorothioate, methyl phosphonate, phosphorodithionate, and the like. In addition, saccharide of each nucleotide (deoxyribose) may be substituted by chemically modified saccharide structure such as 2'-O-methylation, and the like, and base part (pyrimidine, purin) may be chemically modified one. Any type of nucleotide is accepted so long as the nucleotide can hybridize with DNA having the base sequence represented by SEQ ID No: 1, SEQ ID No: 3 or SEQ ID No: 5.

The antisense polynucleotide includes, polynucleotide containing 2-deoxy-D-ribose; polynucleotide containing D-ribose; another type of polynucleotide which is N-glucoside of purin or pyrimidine base; another polymer having non-nucleotide skeleton (for example, commercially available protein nucleic acid, and synthetic sequence-specific nucleic acid polymer) ; or another polymer containing special bond (provided that said polymer contains a nucleotide having a configuration which allows base pairing or attachment of base found in DNA or RNA), and the like. These antisense polynucleotide may be double strand DNA, single strand DNA, double strand RNA, single strand RNA, DNA:RNA hybrid, and further, non-modified polynucleotide (or, non-modified oligo-nucleotide); the one having a known modification, for example, the one having a known label in the relevant field; the capped one; the methylated one; the one where one or more natural nucleotides are substituted by analogues; the one modified by intra-nucleotide, for example the one having non-charged bond (for example, methyl phosphonate, phospho-triester, phosphoramidate, carbamate, and the like); the one having charged bond or sulfur-containing bond (for example, phosphorothioate, phosphorodithioate, and the like), for example, the one having side chain such as protein (for example, nuclease, nuclease-inhibitor, toxin, antibody, signal peptide, poly-L-lysine, and the like), saccharide (for example, monosaccharide, and the like), and the like; the one having intercalated compound (for example, acridine, psoralene, and the like); the one containing chelate compound (for example, metal, radioactive metal, boron, oxidizing metal, and the like); the one containing alkylating agent; the one having modified bond (for example, α-anomer type nucleic acid, and the like). It should be noted that "nucleoside", "nucleotide" and "nucleic acid" include not only the one containing purin and pyrimidine bases, but may include the one having modified other hetero cyclic base. These modified one may be the one containing methylated purin and pyrimidine, acylated purin and pyrimidine, or other hetero cyclic ring. The modified nucleoside and the modified nucleotide may be also modified in saccharide part, for example, one or more hydroxyl group may be substituted by halogen or aliphatic group, or may be converted to a functional group such as ether, amine, or the like.

The antisense polynucleotide of the present disclosure is RNA, NA or a modified nucleic acid (RNA, DNA). Specific example of the modified nucleic acid includes sulfur derivative of nucleic acid; thiophosphate derivative; the one which has resistance to decomposition of polynucleoside amide, oligo-nucleoside amide, and the like. The antisense polynucleotide of the present disclosure can be designed, for example, as follows. That is, to make the antisense polynucleotide in the cell more stable, to increase cell permeability of the antisense polynucleotide, to increase affinity to the target sense chain, and when toxicity exists, to minimize toxicity of the antisense polynucleotide. A number of these modifications have been reported, for example, in Pharm Tech Japan, 8, 247 page, or 395 page (1992), Antisense Research and Applications, CRC Press (1993), and the like.

The antisense polynucleotide of the present disclosure may contain changed or modified saccharide, base or bond, and can be provided in a special form such as liposome and microsphere, applied to gene therapy, and provided in an added form. Those to be used in such an added form include hydrophobic one such as polycation form such as polylysine which functions so as to neutralize the charge of phosphate group skeleton, and lipid which enhances an interaction with cell membrane, or increases incorporation of nucleic acid (for example, phospholipid, cholesterol, and the like), and the like. Preferable lipid to be added includes cholesterol or derivative thereof (for example, cholesteryl chloroformate, cholic acid, and the like) . These lipids can be attached at 3'-terminal or 5'-terminal of nucleic acid, and can be attached through base, saccharide, or intra-molecular nucleoside bond. Other group includes a group for capping specifically arranged at 3'-terminal or 5'-terminal of nucleic acid to inhibit decomposition by nuclease such as exo-nuclease, RNase, and the like. Such group for capping includes, but not limited to, protective group of hydroxyl group known in the relevant field including glycols such as polyethylene glycol, tetraethylene glycol.

Ribozyme which can specifically cleave the mRNA or initial transcription product encoding the protein of the present disclosure in the code region (in the case of initial transcription product, intron part is contained) can be also included in the antisense polynucleotide of the present disclosure. "Ribozyme" means an RNA having enzymatic activity to cleave nucleic acid. Since recently it has become clear that oligo-DNA having the base sequence of active site of said enzyme has the enzyme cleavage activity as well, DNA can be included in the concept of "ribozyme" in this specificaiton, so long as the DNA has a sequence-specific nucleic acid cleavage activity. The rivozyme having the highest versatility includes self-splicing RNA found in infectious RNA such as viroid, virusoid, and the like, and hammerhead type, hairpin type, and the like are known. The hammerhead type is consists of about 40 bases and exerts enzyme activity. It is possible to cleave only target mRNA specifically by making several bases each (about 10 bases in total) at both ends adjacent to the hammerhead structure part being complimentary sequences to the desired cleavage site of mRNA. Since substrate of this type of ribozyme is only RNA, it has an additional advantage that it does not attack genome DNA. When mRNA of the protein of the present disclosure has a double-stranded structure per se, target sequence of single strand can be made, by using hybridized ribozyme connecting RNA-motif derived from virus nucleic acid which can specifically bind to RNA helicase [Proc. Natl. Acad. Sci. USA, 98 (10), 5572-5577 (2001)]. Further, when ribozyme is used in a form of expression vector containing DNA encoding it, in order to promote transfer of transcription product to cytoplasm, hybrid-ribozyme where a sequence of modified tRNA is further connected can be formed.

In this specification, a double-strand RNA composed of an oligo-RNA which is complementary to the partial sequence in the code region of mRNA of the protein of the present disclosure or initial transcription product (in the case of the initial transcription product, intron part is included), and a complementary chain thereof, that is, siRNA has been defined to be included in the antisense polynucleotide of the present disclosure.
A phenomenon so-called RNA interference (RNAi) that when short double-strand RNA is introduced into cell, mRNA complementary to the RNA is decomposed has been previously known in threadworm, insect, plant, and the like. Since it has been confirmed that this phenomenon widely occurs also in the animal cell [Nature, 411 (6836), 494-498 (2001)], this phenomenon has been widely used as an alternative technology for ribozyme. siRNA can be appropriately designed based on the base sequence information of target mRNA using commercially available software (for example, RNAi Designer; Invitrogen Corp.).

The antisense oligo-nucleotide and the ribozyme of the present disclosure can be prepared by determining target sequence of mRNA or initial transcription product based on cDNA sequence or genome DNA sequence of the gene of the present disclosure, and synthesizing a complementary sequence to this, using a commercially available DNA/RNA automatic synthesizer (manufactured by Applied Biosystems Inc., Beckman Instruments, Inc., and the like). siRNA can be prepared by synthesizing a sense chain and an antisense chain using a DNA/RNA automatic synthesizer, respectively, denaturing at about 90 to about 95°C for about 1 minute in an appropriate annealing buffer solution, then annealing at about 30 to about 70°C for about 1 to about 8 hours. Also, a longer double-stranded polynucleotide can be prepared by synthesizing complimentary oligo-nucleotide chains so as to overlap each other alternately, annealing these chains, then lygating using ligase. Alternatively, siRNA can be synthesized as RNA (shRNA) where a sense chain and an antisense chain are connected through a linker having an appropriate length (for example, about 3 to about 10 bases), and designed so as to be processed by an enzyme dicer, and the like in cell of the animal to be introduced. Further, siRNA may be formed by preparing DNA encoding sense chain and antisense chain as an expression vector under the control of Pol III system promoter such as U6 and H1 separately, or DNA encoding RNA chain connecting the above-described sense chain and antisense chain through a linker as an expression vector under the control of Pol III system promoter, and expressing in the animal cell.

Inhibition activity of the antisense polynucleotide of the present disclosure can be examined by using transformant in which the gene of the present disclosure is introduced; in-vivo and in-vitro gene expression system of the present disclosure; or in-vivo and in-vitro protein translation system of the present disclosure.

Hereinafter, use applications of the protein of the present disclosure or the partial peptide thereof (hereinafter, also called simply "protein of the present disclosure"); the DNA encoding the protein of the present disclosure or the partial peptide thereof (hereinafter, also called simply "DNA of the present disclosure"); the above-described antibody of the present disclosure; and the above-described antisense polynucleotide of the present disclosure will be described.

Since expression of the protein of the present disclosure increases in cancer tissues, the protein can be used as a disease marker. That is, the protein is useful as a marker for early diagnosis in a cancer tissue, determination of the severity of the condition, and prediction of disease progression.

Furthermore, as for the protein of the present disclosure (EVI1, ITGA6, ITGB4), cell adhesion of a cancer cell is inhibited by inhibiting the expression and/or activity thereof. Therefore, a medicament containing the antisense polynucleotide of the present disclosure, the antibody of the present disclosure, or a substance inhibiting expression and/or activity of the above-mentioned protein (for example, low molecular weight compound) can be used as a cell adhesion inhibitor of a cancer cell, therefore, prophylactic / therapeutic agent (preferably prophylactic / therapeutic agent for hematopoietics tumors, more preferably prophylactic / therapeutic agent for acute myeloid leukemia (AML)) for cancer (for example, colorectal cancer, breast cancer, lung cancer, prostate cancers, esophageal cancer, gastric cancer, liver cancer, biliary tract cancer, spleen cancer, kidney cancer, bladder cancer, uterine cancers, ovarian cancer, testicular cancer, thyroid cancer, pancreatic cancer, brain tumor, hematopoietics tumor (for example, acute myelogenous leukemia, acute promyelocytic leukemia, acute lymphoblastic leukemia, chronic myelogenous leukemia, chronic lymphatic leukemia, or the like.) or the like). In addition, when the above medicament is used together with another anticancer drug than the medicament concerned, it can also be used as an agent for improving sensitivity for a cancer cell to the anticancer drug (drug sensitivity improver) . It should be noted that, in the present specification, "cancer cell" is used as a concept including the cell oriented to turn cancerous in the future.

As described in the Examples described below, when the present inventors compared expression of ITGA6 gene / protein and ITGB4 gene / protein in human hematopoietic stem cell and human leukemia cell, it was confirmed that these genes / proteins did not express in hematopoietic stem cell, but highly expressed only in leukemia cell. Therefore, it is more preferable particularly to use ITGA6 (protein = SEQ ID No: 4, gene = SEQ ID No: 3) and ITGB4 (protein = SEQ ID No: 6, gene = SEQ ID No: 5) as a target from the viewpoint that reducing effect for adverse effect can be also obtained. This description can be also applied to the aspects concerning the antibody of the present disclosure and the antisense polynucleotide of the present disclosure described below.

The antibody (neutralizing antibody) of the present disclosure can neutralize the activity of the protein of the present disclosure. For this reason, the antibody (neutralizing antibody) of the present disclosure can be used as a cell adhesion inhibitor for a cancer cell, prophylactic / therapeutic agent for cancer (for example, colorectal cancer, breast cancer, lung cancer, prostate cancers, esophageal cancer, gastric cancer, liver cancer, biliary tract cancer, spleen cancer, kidney cancer, bladder cancer, uterine cancers, ovarian cancer, testicular cancer, thyroid cancer, pancreatic cancer, brain tumor, hematopoietics tumor, or the like) (preferably prophylactic / therapeutic agent for hematopoietics tumors (for example, acute myelogenous leukemia (AML), acute promyelocytic leukemia, acute lymphoblastic leukemia, chronic myelogenous leukemia, chronic lymphatic leukemia, or the like), and the like (preferably prophylactic / therapeutic agent for hematopoietics tumors), more preferably prophylactic / therapeutic agent for acute myeloid leukemia (AML)). In addition, the antibody (neutralizing antibody) of the present disclosure can also be used as an agent for improving sensitivity (drug sensitivity improver) of cancer cells for another anticancer drug than the antibody concerned when it is used together with the anticancer drug. It should be noted that, in the present disclosure, "neutralization" is defined as a concept including antibody-dependent cellular cytotoxicity activity (ADCC activity), complement-dependent cytotoxicity activity (CDC activity), inhibition of proliferative signal of a cancer cell (narrowly defined neutralization activity), and induction of apotosis, and the like (not limited thereto).

Since the prophylactic / therapeutic agents for the above-described diseases, cell adhesion inhibitor, drug sensitivity improver of the present disclosure containing the antibody of the present disclosure are low-toxic, these agents can be administered as a liquid agent as it is or a pharmaceutical composition of appropriate dose form to human or mammal (for example, rat, rabbit, sheep, pig, bovine, cat, dog, ape, and the like) orally or parenterally (for example, intravascular administration, subcutaneous injection, intramuscular administration, and the like).

The antibody of the present disclosure may be administered in itself, or may be administered as an appropriate pharmaceutical composition. The pharmaceutical composition to be used for administration may be a composition containing the antibody of the present disclosure and a salt thereof and pharmacologically acceptable carrier, diluent or excipient. Such pharmaceutical composition is provided in a dose form suitable for oral or parenteral administration.

As a composition for parenteral administration, for example, injectable solution, suppository, and the like are used. The injectable solution may include dose forms such as intravenous injectable solution, subcutaneous injectable solution, intramuscular injectable solution, intravenous drip injectable solution, and the like. Such injectable solution can be prepared according to the known method. As for preparation method for injectable solution, the injectable solution can be prepared, for example, by dissolving, suspending or emulsifying the above-described antibody of the present disclosure or the salt thereof in a sterilized aqueous liquid or oily liquid which is commonly used for injectable solution. As an aqueous liquid for injection, for example, saline solution, isotonic solution containing glucose and other adjuvant, and the like are used, and an appropriate solubilizing agent, for example, alcohol (for example, ethanol), polyalcohol (for example, propylene glycol, polyethylene glycol), nonionic surfactant [for example, Polysorbate 80, HCO-50 (polyoxyethylene (50 mol) adduct of hydrogenated castor oil)], and the like may be used in combination. As an oily liquid, for example, sesame oil, soybean oil, and the like are used, and as a solubilizing agent, benzyl benzoate, benzyl alcohol, and the like may be used in combination. The prepared injectable solution is preferably filled in an appropriate ampule. Suppository to be used for rectal administration may be prepared by mixing the above-described antibody or the salt thereof with the common substrate for suppository.

A composition for oral administration includes solid or liquid dose form, specifically, tablet (including sugar-coated tablet, film coating tablet), pill, granule, powder, capsule (including soft capsule), syrup, emulsion, suspension, and the like. Such composition can be produced by the known method, and carrier, diluent, excipient which are commonly used in pharmaceutical field may be contained. As a carrier and an excipient for tablet, for example, lactose, starch, sucrose, magnesium stearate are used.

The above-described parenteral or oral pharmaceutical composition is advantageously prepared in a dose form of administration unit which is adapted for active component. Such dose form of administration unit includes, for example, tablet, pill, capsule, injectable solution (ampule) and suppository. As for content of the antibody, the above-described antibody of usually 5 to 500 mg, particularly 5 to 100 mg for injectable solution, and 10 to 250 mg for other dose form per dose form of administration unit is preferably contained.

Dosage of the above-described preparation containing the antibody of the present disclosure varies depending on target to be administered, target disease, symptom, administration route, and the like, and, for example, when used for treatment / prevention of adult breast cancer, it is advantageous to administer the antibody of the present disclosure of usually about 0.01 to 20 mg/kg weight, preferably about 0.1 to 10 mg/kg weight, and further preferably about 0.1 to 5 mg/kg weight per one doze, about 1 to 5 times per day, and preferably 1 to 3 times per day by intravenous injection. In the case of other parenteral administration and oral administration, a compatible dosage thereto can be administered. When symptom is particularly serious, dosage may be increased according to the symptom.

The antibody of the present disclosure can be administered in itself, or as an appropriate pharmaceutical composition. The pharmaceutical composition to be used for the above-described administration is a composition containing the above-described antibody or the salt thereof and pharmacologically acceptable carrier, diluent or excipient. Such composition is provided in a dose form suitable for oral or parenteral administration (for example, intravascular injection, hypodermic injection, and the like).

In addition, each composition described above may contain other active component as long as the component does not cause unfavorable interaction by blending with the above antibody.

Further, the antibody of the present disclosure may be used in combination with other medicament, for example, alkylating agent (for example, cyclophosphamide, ifosfamide, and the like); metabolic antagonist (for example, methotrexate, 5-fluorouracil, and the like); anticancer antibiotics (for example, mitomycin, adriamycin, and the like); anticancer drug derived from plant (for example, vincristin, vindesine, taxol, and the like); cisplatin, carboplatin, etopoxide, irinotecan, and the like. The antibody of the present disclosure and the above-described medicaments may be administered to patient simultaneously or at different times. In such aspect, the antibody of the present disclosure can function as an agent to improve sensitivity of cancer cell to the above-described medicaments (drug sensitivity improver). However, the technical scope of the present disclosure is not limited to the aspect where such function is exerted.

### (2) Medicament containing the antisense polynucleotide

The antisense polynucleotide of the present disclosure which binds complementarily to the transcription product of the gene of the present disclosure and can inhibit expression of said gene is low-toxic, and can retard the function and action of the protein of the present disclosure or the gene of the present disclosure in vivo, and can inhibit the cell adhesion of cancer cell. For this reason, the antisense polynucleotide of the present disclosure can be used as a cell adhesion inhibitor for cancer cell, prophylactic / therapeutic agent for cancer (for example, colorectal cancer, breast cancer, lung cancer, prostate cancers, esophageal cancer, gastric cancer, liver cancer, biliary tract cancer, spleen cancer, kidney cancer, bladder cancer, uterine cancers, ovarian cancer, testicular cancer, thyroid cancer, pancreatic cancer, brain tumor, hematopoietics tumor (for example, acute myelogenous leukemia (AML), acute promyelocytic leukemia, acute lymphoblastic leukemia, chronic myelogenous leukemia, chronic lymphatic leukemia, or the like.), and the like) (preferably prophylactic / therapeutic agent for hematopoietic tumor, more preferably prophylactic / therapeutic agent for acute myelocytic leukemia (AML)). In addition, the antisense polynucleotide of the present disclosure can be used as an agent to improve sensitivity of cancer cell to the above-described anticancer drug (drug sensitivity improver) when used in combination with another anticancer drug than the antisense polynucleotide.

When the antisense polynucleotide of the present disclosure is used as the above-described prophylactic / therapeutic agent, cell adhesion inhibitor, drug sensitivity improver, and the like, the antisense polynucleotide can be formulated and can be administered according to a known method per se.

In addition, for example, the above-described antisense polynucleotide can be administered by itself or after inserting into an appropriate vector such as retrovirus vector, adenovirus vector, adenovirus associated virus vector, to human or mammal (for example, rat, rabbit, sheep, pig, bovine, cat, dog, ape, and the like) orally or parenterally. Said antisense polynucleotide is formulated by itself or in combination with physiologically acceptable carrier such as an adjuvant to promote intake, and can be administered by gene gun or catheter such as hydro-gel catheter. Alternatively, it can be locally administered into trachea by aerosolized inhalant.

Further, for the purposes of improvement of pharmacokinetics, prolongation of half-life and improvement of intracellular uptake efficiency, the above-described antisense polynucleotide is formulated (injectable solution) by itself or together with carrier such as liposome, and may be administered intravenously, subcutaneously, or the like.

Dosage of said antisense polynucleotide varies depending on target disease, target to be administered, administration route, and the like, and for example, when the antisense polynucleotide of the present disclosure is administered for treatment of breast cancer, said antisense polynucleotide of generally about 0.1 to about 100 mg per day is administered for an adult (body weight: 60 kg).

The polynucleotide containing a base sequence encoding the protein of the present disclosure or a part thereof (also called "sense polynucleotide of the present disclosure"), and the antisense polynucleotide of the present disclosure can be used as a diagnostic nucleotide probe (or primer) to examine the expression state of the gene of the present disclosure in a tissue or cell.

### (3) Screening of medicament candidate compound for disease

The protein of the present disclosure shows enhanced expression in a cancer tissue, moreover, when expression and/or activity of EVI1, ITGA6 or ITGB4 protein are inhibited, cell adhesion of a cancer cell can be inhibited. Therefore, a compound inhibiting expression and/or activity of EVI1, ITGA6 or ITGB4 protein or a salt thereof can be used as a cell adhesion inhibitor for a cancer cell, and a prophylactic / therapeutic agent for cancer (for example, colorectal cancer, breast cancer, lung cancer, prostate cancers, esophageal cancer, gastric cancer, liver cancer, biliary tract cancer, spleen cancer, kidney cancer, bladder cancer, uterine cancers, ovarian cancer, testicular cancer, thyroid cancer, pancreatic cancer, brain tumor, hematopoietics tumor (for example, acute myelogenous leukemia (AML), acute promyelocytic leukemia, acute lymphoblastic leukemia, chronic myelogenous leukemia, chronic lymphatic leukemia, or the like.), and the like) (preferably, a prophylactic / therapeutic agent for hematopoietic tumor, more preferably, a prophylactic / therapeutic agent for acute myelocytic leukemia (AML)).

Therefore, the protein of the present disclosure (EVI1, ITGA6, and ITGB4) is useful as a reagent for screening a compound which inhibits expression and/or activity of said protein or a salt thereof.

That is, according to another aspect of the present invention, there is provided a method for screening a substance to inhibit cell adhesion of a cancer cell, the method comprising using a protein comprising an amino acid sequence represented by SEQ ID No: 2 to identify a substance that inhibits the activity of said protein thereby inhibiting cell adhesion of a cancer cell.

Also disclosed is a method for screening a compound which inhibits expression and/or activity of the protein of the present disclosure or a salt thereof by using said protein.

In case of screening the compound which inhibits activity of the protein of the present disclosure or a salt thereof, said screening method is roughly divided as follows:
(a-1) a method where activity of the isolated protein of the present disclosure is compared in the presence or absence of a test substance;
(a-2) a method where a cell having capability of producing the protein of the present disclosure is cultured in the presence or absence of a test substance, and the activities of the protein of the present disclosure under both conditions are compared.

The protein of the present disclosure to be used in the screening method of the above-described (a-1) can be isolated / purified by using the production method of the above-described protein of the present disclosure or the partial peptide thereof.

A cell having the capability of producing the protein of the present disclosure to be used in the screening method of the above-described (a-2) is not particularly limited, so long as it is a cell of human or other warm-blooded animals naturally expressing it, or biological sample containing it (for example, blood, tissue, internal organ, and the like) . The cell includes, for example, human breast cancer cell strain MCF7, T47D, and the like. In the case of blood, tissue, internal organ, and the like derived from a nonhuman animal, these may be isolated from living body and cultured, or a test substance may be administered to living body, and after the elapse of a certain period of time, living samples may be isolated.

In addition, as a cell having the capability of producing the protein of the present disclosure, various transformants produced by the above-described gene-engineering procedure can be exemplified. As a host, for example, animal cells such as COS7 cell, CHO cell, HEK293 cell, and the like are preferably used.

Test substance includes, for example, protein, peptide, non-peptidic compound, synthetic compound, fermentation product, cell-extract, plant-extract, animal tissue-extract, and the like, and these substances may be a novel one or a known one.

Measurement of activity of the protein of the present disclosure in the screening method of the above-described (a-1) can be carried out, for example, by contacting (i) the protein of the present disclosure or (ii) the protein of the present disclosure and test substance with a cell of a warm-blooded animal where cell adhesion can be activated by the protein of the present disclosure, and measuring the level of cell adhesion in said cell. Also, measurement of activity of the protein of the present disclosure in the screening method of the above-described (a-2) can be carried out by measuring cell adhesion in the cell having capability of producing said protein of the present disclosure.

When the level of cell adhesion is used as an indicator, cells are suspended in an appropriate culture medium or a buffer solution, a test substance is added (or not added), further stimulus such as oxidizing stimulus (for example, addition of H₂O₂) is added, and incubation is carried out usually at 20 to 40°C, preferably at 30 to 37°C for about 6 to 72 hours, then, induction rate of apotosis (cell death) is measured.

Cell adhesion can be examined, for example, by detecting cell number in the presence / absence of the test substance. Specifically, for example, comparison of the level of cell adhesion becomes possible, by measuring residual cell number after PBS washing.

For example, in the screening method of the above-described (a-1), when activity of the protein of the present disclosure in the presence of the test substance is inhibited by about 20% or more, preferably by 30% or more, more preferably by 50% or more compared with that in the absence of the test substance, said test substance can be selected as an activity inhibiting substance for the protein of the present disclosure.

As described above, the substances which inhibit expression of the protein of the present disclosure (EVI1, ITGA6, and TGB4 proteins) are useful for inhibiting cell adhesion of a cancer cell, therefore, for prevention / treatment of cancer.

That is, according to further another aspect of the present disclosure, there is provided a method for screening a substance to inhibit cell adhesion of a cancer cell, a substance for prevention / treatment of cancer, by comparing the expression of the protein of the present disclosure in the cell having capability of producing said protein in the presence and the absence of test substance.

Expression level of the protein of the present disclosure can be measured at a transcription level by detecting mRNA thereof using polynucleotide which can hybridize with a polynucleotide encoding the protein of the present disclosure under highly stringent conditions (i.e., a polynucleotide containing a base sequence encoding the protein of the present disclosure or a part thereof (the sense nucleotide of the present disclosure), or the antisense nucleotide of the present disclosure). Alternatively, said expression level can be measured at a translation level by detecting the protein of the present disclosure using the above-described antibody of the present disclosure.

Therefore, more specifically, disclosed herein are:
(b) a method for screening a substance inhibiting cell adhesion of a cancer cell, a substance for prevention / treatment of cancer, by culturing a cell having capability of producing the protein of the present disclosure in the presence or the absence of a test substance, and measuring / comparing amounts of mRNA encoding the protein of the present disclosure in both conditions using the sense or antisense polynucleotide of the present disclosure; and
(c) a method for screening a substance inhibiting cell adhesion of a cancer cell, substance for prevention / treatment of cancer, by culturing a cell having capability of producing the protein of the present disclosure in the presence or the absence of a test substance, and measuring / comparing amounts of the protein of the present disclosure in both conditions using the antibody of the present disclosure.

In the screening methods of the above-described (b) and (c), as the cell having capability of producing the protein of the present disclosure, the same ones as used in the screening method of the above-described (a-2) are preferably used.

For example, measurement of amount of mRNA of the protein of the present disclosure, or the amount of the protein of the present disclosure can be specifically carried out as follows.
(i) A medicament (for example, TNF-α, IL-1, Fas, anticancer drug, and the like) or physico-chemical stress (for example, UV, active oxygen, ischemia, and the like) is given to a normal or a diseased-model non-human warm-blooded animal (for example, mouse, rat, rabbit, sheep, pig, bovine, cat, dog, ape, chicken, and the like), and after the elapse of a certain period of time, tissue or cell isolated from blood, or specific organ (for example, brain, liver, kidney, and the like)is obtained.

mRNA of the protein of the present disclosure contained in the obtained cell can be determined, for example, by extracting mRNA from the cell or the like by a common method, and, for example, by using a technique such as RT-PCR method, and the like, or can be determined by Northern blot analysis known per se. On the other hand, the amount of the protein of the present disclosure can be determined by using Western blot analysis, or various immunoassay methods described below in detail.
(ii) The transformant to which polynucleotide encoding the protein of the present disclosure is introduced is produced according to the above-described method, and amount of the protein of the present disclosure contained in said transformant, or mRNA encoding it can be determined / analyzed in the same way as in the above-described (i) .

Screening of a substance which changes expression level of the protein of the present disclosure can be carried out as follows:
(i) a test substance is given at a certain time before giving a chemical agent, physicochemical stress, or the like to a normal or a diseased-model non-human warm-blooded animal (30 minutes before to 24 hours before, preferably 30 minutes before to 12 hours before, more preferably 1 hour before to 6 hours before), or at certain time after that (30 minutes to 3 days after, preferably 1 hour to 2 days after, more preferably 1 hour to 24 hours after), or concurrently with the chemical agent or the physicochemical stress, and after the elapse of a certain period of time from administration (after 30 minutes to 3 days, preferably after 1 hour to 2 days, more preferably after 1 hour to 24 hours), the amount of mRNA encoding the protein of the present disclosure contained in the cell which is isolated from said animal is determined / analyzed, or
(ii) when transformant is cultured by a common method, a test substance is added in the culture medium, and after the elapse of a certain period of time of culture (after 1 day to 7 days, preferably after 1 day to 3 days, more preferably after 2 days to 3 days), the amount of mRNA of the protein of the present disclosure contained in said transformant, or the amount of the protein of the present disclosure is determined / analyzed.

The test substance includes peptide, protein, non-peptidic compound, synthetic compound, fermentation product, and the like, and these substances may be a novel one or a known one.

Method of determining amount of the protein of the present disclosure in the screening method of the above-described (c) specifically includes, for example,:
(i) a method where the protein of the present disclosure in the sample liquid is determined by reacting the antibody of the present disclosure with the sample liquid and the labeled protein of the present disclosure competitively, and detecting the labeled protein of the present disclosure bound to said antibody;
(ii) a method where the protein of the present disclosure in the sample liquid is determined by reacting the sample liquid with the antibody of the present disclosure immobilized on the carrier and the labeled another antibody of the present disclosure concurrently or sequentially, after that measuring amount (activity) of the labeling agent on the immobilized carrier; and the like.

In the determining method of the above-described (ii), the two kinds of the antibodies are desirably the ones which recognize different parts of the protein of the present disclosure. For example, when one antibody is the one which recognizes the N-terminal of the protein of the present disclosure, as the other antibody, the one which reacts with C-terminal of the protein of the present disclosure can be used.

As the labeling agent to be used in the measuring method using labeling substance, for example, radioactive isotope, emzyme, fluorescent substance, luminescent substance, and the like are used. As the radioactive isotope, for example, [¹²⁵I], [¹³¹I], [³H], [¹⁴C], and the like are used. As the above-described enzyme, the one which is stable and has a high specific activity is preferable. For example, β-galactosidase, β-glucosidase, alkaline phosphatase, peroxidase, malate dehydrogenase, and the like are used. As the fluorescent substance, for example, fluorescamine, fluorescein isothiocyanate, and the like are used. As the luminescent substance, for example, luminol, luminol derivative, luciferin, lucigenin, and the like are used. Further, bioin-(strept) avidin system can be used for binding the antibody or the antigen and the labeling agent.

As the sample liquid, when the protein of the present disclosure is localized in a cell, cell-broken liquid obtained by suspending cell in an appropriate buffer solution, and then breaking cell through ultrasonication, freezing and thawing, and the like; and when the protein of the present disclosure is secreted outside a cell, cell culture supernatant can be used, respectively. If necessary, the protein of the present disclosure may be determined after separation / purification from the cell-broken liquid or the culture supernatant. In addition, an intact cell may be used as sample, as long as detection of the labeling agent is possible.

The determining method of the protein of the present disclosure using the antibody of the present disclosure is not particularly limited, and any type of method may be used so long as the measuring method is the one where amount of antibody, antigen or antibody-antigen complex corresponding to the amount of antigen in the sample liquid is detected by chemical or physical means, and amount of the protein is calculated from standard curve which is made using a standard liquid containing known amount of antigen.

For example, nephelometry, competition method, immunometric assay and sandwich method are suitably used. From the viewpoints of sensitivity and specificity, it is preferable to use, for example, sandwich method described below.

In immobilizing antigen or antibody, physical adsorption may be used, also, chemical bond which is commonly used for insolubilizing / immobilizing protein, enzyme, or the like may be used. The carrier includes insoluble polysaccharides such as agarose, dextran, cellulose, and the like; synthetic resins such as polystyrene, polyacrylamide, silicone; glass; and the like.

In the sandwich method, the protein of the present disclosure in a sample liquid can be determined, by reacting the immobilized antibody of the present disclosure with sample liquid (first reaction), further reacting with the labeled another antibody of the present disclosure (second reaction), after that, measuring amount or activity of the labeling agent on the immobilized carrier. The first reaction and the second reaction may be carried out in a reverse order, or concurrently, or with an interval. The labeling agent and immobilizing method are similar to those described above. In addition, in immunoassay by the sandwich method, the antibody to be used for solid-phased antibody or labeled antibody is not necessarily one kind, a mixture of two kinds or more of antibodies may be used for the purpose to improve measuring sensitivity or the like.

The antibody of the present disclosure can be used in the measuring system other than the sandwich method, for example, competition method, immunometric assay, or nephelometry, and the like.

In the competitive method, the protein of the present disclosure in a sample liquid is determined, by reacting antibody with the protein of the present disclosure in a sample liquid and the labeled protein of the present disclosure competitively, after that, separating unreacted labeled antigen (F) and labeled antigen (B) bound to antibody (B/F separation), and measuring amount of label of either B or F. In this reaction method, liquid phase method where soluble antibody is used as an antibody, and B/F separation is carried out by using polyethylene glycol, secondary antibody to the above-described antibody (primary antibody), and the like; and solid-phased method where solid-phased antibody is used as a primary antibody (direct method), or soluble antibody is used for primary antibody and solid-phased antibody is used for secondary antibody (indirect method); are used.

In the immunometric assay, amount of antigen in a sample liquid is determined, by reacting the protein of the present disclosure in a sample liquid and the solid-phased protein of the present disclosure with a certain amount of labeled antibody competitively, then separating solid phase and liquid phase, or reacting the protein of the present disclosure in a sample liquid with excess amount of labeled antibody, subsequently adding the solid-phased protein of the present disclosure to bind unreacted labeled antibody to the solid phase, then separating solid phase and liquid phase, after that, measuring amount of label of either phase.

In addition, in the nephelometry, amount of insoluble precipitate resulted from the antigen-antibody reaction in a gel or in a solution is determined. Even when amount of the protein of the present disclosure in sample liquid is small and only small amount of precipitate can be obtained, laser-nephelometry utilizing laser scattering, and the like are preferably used.

In applying individual immunological measuring method to the determination method of the present disclosure, setting of special conditions, operation, and the like are not necessary. A measurement system of the protein of the present disclosure may be constructed based on the usual conditions and procedures in each method added with usual technical considerations of those skilled in the art. Details of these general technical means may be referred to reviews, published books, and the like.

For example, Hiroshi Irie, "Radio-Immunoassay" (Kodansha Ltd., 1974); Hiroshi Irie, "Second Series of Radio-Immunoassay" (Kodansha Ltd., 1979); Eiji Ishikawa, et al., "Enzyme Immunoassay" (Igakushoin Ltd., 1978); Eiji Ishikawa, et al., "Enzyme Immunoassay" (2nd ed.) (Igakushoin Ltd., 1982); Eiji Ishikawa, et al., "Enzyme Immunoassay" (3rd ed.) (Igakushoin Ltd., 1987); "Methods in ENZYMOLOGY" Vol. 70 (Immunochemical Techniques (Part A)); Ibid. Vol. 73 (Immunochemical Techniques (Part B)); Ibid. Vol. 74; (Immunochemical Techniques (Part C)); Ibid. Vol.84 (Immunochemical Techniques (Part D: Selected Immunoassays)); Ibid. Vol. 92 (Immunochemical Techniques (Part E: Monoclonal Antibodies and General Immunoassay Methods)); Ibid. Vol. 121 (Immunochemical Techniques (Part I: Hybridoma Technology and Monoclonal Antibodies)) (Hereinbefore, edited by Academic Press Co., Ltd), and the like can be referred to.

Hereinbefore, by using the antibody of the present disclosure, production amount of the protein of the present disclosure in cell can be sensitively determined.

For example, in the screening method of the above-described (b) and (c), expression level of the protein of the present disclosure(EVI1, ITGA6 and ITGB4) under the presence of test substance (amount of mRNA, or amount of protein) comparing with the case on the absence of test substance, is inhibited by more than about 20%, preferably more than about 30%, more preferably more than about 50%, said test substance can be selected as expression-inhibiting material of the protein of the present disclosure.

Screening kit of the present disclosure contains the protein or its partial peptide of the present disclosure (hereinafter, called as "the protein of the present disclosure"). The protein of the present disclosure may be isolated, purified by using any of the above-described method, or may be provided as a cell form which produces it (a cell of a warm-blooded animal) as described above.

The screening kit of the present disclosure can additionally contain the above-described antibody of the present disclosure or sense or antisense polynucleotide of the present disclosure to measure the expression level of said protein in the cell which produces the protein of the present disclosure.

Said screening kit can optionally contain a reaction buffer solution, a blocking solution, a washing buffer solution, a labeling reagent, a label-detecting reagent, and the like in addition to the above-described ones.

The expression and/or activity inhibiting substance (may be free form or a salt form) of the protein of the present disclosure obtained by the screening method or the screening kit of the present disclosure is useful for a low-toxic and safe medicament as a cell adhesion inhibitor of a cancer cell and a prophylactic / therapeutic agent for cancer (for example, colorectal cancer, breast cancer, lung cancer, prostate cancers, esophageal cancer, gastric cancer, liver cancer, biliary tract cancer, spleen cancer, kidney cancer, bladder cancer, uterine cancers, ovarian cancer, testicular cancer, thyroid cancer, pancreatic cancer, brain tumor, hematopoietics tumor (for example, acute myelogenous leukemia (AML), acute promyelocytic leukemia, acute lymphoblastic leukemia, chronic myelogenous leukemia, chronic lymphatic leukemia, or the like.), and the like) (preferably a prophylactic / therapeutic agent for hematopoietic tumor, and more preferably a prophylactic / therapeutic agent for acute myelocytic leukemia(AML)) .

When the compound or a salt thereof obtained by using the screening method or the screening kit of the present disclosure is used as the above-described prophylactic / therapeutic agent, the compound can be formulated according to a common means.

For example, composition for oral administration includes solid or liquid dose form, specifically, tablet (including sugar-coated tablet, film coating tablet), pill, granule, powder, capsule (including soft capsule), syrup, emulsion, suspension, and the like. Such composition can be produced by the known method per se, and contains carrier, diluent or excipient commonly used in pharmaceutical field. As carrier, excipient for tablet, for example, lactose, starch, sucrose, magnesium stearate, and the like are used.

As the composition for parenteral administration, for example, injectable solution, suppository, and the like are used, and injectable solution includes dose forms such as intravenous injectable solution, hypodermic injectable solution, intracutaneous injectable solution, intramuscular injectable solution, intravenous drip injectable solution, intraarticular injectable solution, and the like. Such injectable solution can be prepared according to the known method per se, for example, by dissolving, suspending or emulsifying the above-described compound or the salt thereof in a sterilized aqueous or oily liquid to be usually used for injectable solution. As the aqueous liquid for injection, for example, saline solution, isotonic solution containing glucose and other adjuvant, and the like are used. An appropriate solubilizing agent, for example, alcohol (for example, ethanol), polyalcohol (for example, propylene glycol, polyethylene glycol), nonionic surfactant [for example, Polysorbate 80, HCO-50 (polyoxyethylene (50 mol) adduct of hydrogenated castor oil)], and the like may be used in combination. As the oily liquid, for example, sesame oil, soybean oil, and the like are used, and benzyl benzoate, benzyl alcohol, and the like may be used in combination as a solubilizing agent. The prepared injectable solution is usually filled in an appropriate ampule. Suppository used for rectal administration is prepared by mixing the above-described compound or the salt thereof with a common substrate for suppository.

The above-described pharmaceutical composition for oral or parenteral administration is advantageously prepared in a dose form of administration unit which is adapted for dosage of active component. As such dose form of administration unit, tablet, pill, capsule, injectable solution (ampule), suppository, and the like are exemplified. As content of the above-described compound, usually 5 to 500 mg, in particular, 5 to 100 mg for injectable solution, and 10 to 250 mg for other dose form per each dose form of administration unit is preferably contained.

It should be noted that each of the above-described compositions may contain other active component, as long as any unfavorable interaction is not caused by the blending with the above-described compound.

Since the preparation obtained in such way is safe and low-toxic, it can be administered orally or parenterally, for example, to human or warm-blooded animal (for example, mouse, rat, rabbit, sheep, pig, bovine, horse, chicken, cat, dog, ape, chimpanzee, and the like).

Dosage of said compound or the salt thereof varies depending on its function, target disease, target to be administered, administration route, and the like, but, for example, when an expression and/or activity inhibiting substance of the protein of the present disclosure is administered orally for the purpose of treating breast cancer, generally in an adult (provided that body weight is 60 kg), said compound or the salt thereof of about 0.1 to about 100 mg, preferably about 1.0 to about 50 mg, more preferably about 1.0 to about 20 mg per day is administered. In the case of parenteral administration, single dosage of said inhibiting substance varies depending on target to be administered, target disease, and the like, but, for example, when an expression and/or activity inhibiting substance of the protein of the present disclosure is administered in a form of injectable solution to an adult (provided that body weight is 60 kg), for the purpose of treating breast cancer, usually said compound or the salt thereof of about 0.01 to about 30 mg, preferably about 0.1 to about 20 mg, more preferably about 0.1 to about 10 mg per day is advantageously administered to the cancer-affected part by injection. In the case of other animal, a converted amount based on body weight of 60 kg can be also administered.

### (4) Diagnostic agent for cancer

The antibody of the present disclosure can specifically recognize the protein of the present disclosure. For this reason, said antibody can be used for determination of the protein of the present disclosure in a test liquid. Therefore, in the screening method of expression-inhibiting substance for the protein of the present disclosure using the above-described antibody of the present disclosure, by carrying out immunoassay using biological sample (for example, blood, plasma, urine, biopsy, and the like) collected from the test warm-blooded animal instead of the cell having capability of producing the protein of the present disclosure, extent of expression of the protein of the present disclosure in the body of said animal can be examined, and eventually the antibody of the present disclosure can be used for diagnosis of cancer. As a result of immunoassay, when increase of the protein of the present disclosure is detected in said sample, it is possible to make a diagnosis that cancer (for example, colorectal cancer, breast cancer, lung cancer, prostate cancers, esophageal cancer, gastric cancer, liver cancer, biliary tract cancer, spleen cancer, kidney cancer, bladder cancer, uterine cancers, ovarian cancer, testicular cancer, thyroid cancer, pancreatic cancer, brain tumor, hematopoietics tumor (in particular, AML), or the like.) has been developed or is likely to be developed in future.

Similarly, the above-described sense or antisense polynucleotide of the present disclosure can detect abnormality in DNA or mRNA (gene abnormality) encoding the protein of the present disclosure in human or other warm-blooded animal (for example, rat, mouse, hamster, rabbit, sheep, goat, pig, bovine, horse, cat, dog, ape, chimpanzee, chicken, and the like) by using as a probe or a primer. For this reason, said polynucleotide is useful, for example, as a gene diagnosis agent for amplification of said DNA or excess expression of mRNA, in particular, a diagnosis agent for cancer. The polynucleotide encoding the protein of the present disclosure or the corresponding antisense polynucleotide is not particularly limited, so long as it has a necessary length as a probe or a primer (for example, about 15 bases or more).

The above-described gene diagnosis using the sense or antisense polynucleotide of the present disclosure can be carried out, for example, by northern hybridization method, quantitative RT-PCR method, PCR-SSCP method, allele-specific PCR method, PCR-SSOP method, DGGE method, RNase protection method, PCR-RFLP method, and the like known per se.

For example, when increased expression of the protein of the present disclosure is detected, as a result of diagnosis by northern hybridization method or quantitative RT-PCR method on RNA fraction extracted from a cell of the test warm-blooded animal, it is possible to make a diagnosis that cancer (for example, colorectal cancer, breast cancer, lung cancer, prostate cancers, esophageal cancer, gastric cancer, liver cancer, biliary tract cancer, spleen cancer, kidney cancer, bladder cancer, uterine cancers, ovarian cancer, testicular cancer, thyroid cancer, pancreatic cancer, brain tumor, hematopoietics tumor (in particular, AML), or the like.) has been developed or is likely to be developed in future.

### (5) Medicament containing the protein of the present disclosure

Since the protein of the present disclosure expresses excessively in a cancer cell, the protein of the present disclosure can be used as a cancer vaccine to activate immune system of a cancer patient.

For example, so-called adoptive immunotherapy where a strong, antigen-presenting cell (for example, dendritic cell) is cultured in the presence of the protein of the present disclosure, and after phagocytosis of said protein, the cell is returned into the body of the patient, and the like can be preferably applied. The dendritic cell returned into the body can kill a cancer cell, by inducing and activating cancer antigen-specific cytotoxic T cells.

In addition, the protein of the present disclosure can be safely administered to mammal (for example, human, ape, mouse, rat, rabbit, pig) as a vaccine preparation for prevention or treatment of, for example, cancer (for example, colorectal cancer, breast cancer, lung cancer, prostate cancers, esophageal cancer, gastric cancer, liver cancer, biliary tract cancer, spleen cancer, kidney cancer, bladder cancer, uterine cancers, ovarian cancer, testicular cancer, thyroid cancer, pancreatic cancer, brain tumor, hematopoietics tumor (in particular, AML), or the like.).

Said vaccine preparation contains usually the protein of the present disclosure and a physiologically acceptable carrier. The carrier includes, for example, liquid carrier such as water, salt solution (including physiological saline), buffer solution (for example, phosphate buffer solution), alcohols (for example, ethanol).

The vaccine preparation can be prepared according to the production method for the common vaccine preparation.

Usually, the protein of the present disclosure is dissolved or suspended in a physiologically acceptable carrier. Alternatively, the protein of the present disclosure and the physiologically acceptable carrier may be separately prepared, which may be mixed before use.

In vaccine preparation, in addition to the protein of the present disclosure and the physiologically acceptable carrier, adjuvant (for example, aluminum hydroxide gel, serum albumin, and the like), preservative (for example, thimerosal, and the like), soothing agent (for example, glucose, benzyl alcohol, and the like), and the like may be contained.

When used as a vaccine preparation, the protein of the present disclosure may be used as an active form, but said protein may be denatured to increase antigenecity. Denaturing of the protein of the present disclosure is carried out usually by heat treatment, treatment with protein denaturant (for example, formalin, guanidine hydrochloride, urea).

The obtained vaccine preparation is low-toxic, and may be administered usually as an injectable solution, for example, by hypodermic injection, endodermic injection, intramuscular injection, or may be locally administered to cluster of cancer cells or adjacent part thereto.

Dosage of the protein of the present disclosure varies depending on, for example, target disease, target to be administered, administration route, and the like, but, for example, when the protein of the present disclosure is administered to an adult (body weight: 60 kg) affected by cancer subcutaneously as an injectable solution, dosage is usually about 0.1 to 300 mg, and preferably about 100 to 300 mg per single dose. Administration frequency of the vaccine preparation may be 1 time, but said vaccine preparation can be administered 2 to 4 times with intervals of about 2 weeks to about 6 months to increase the amount of antibody production,.

### (6) DNA-transferring animal

Disclosed herein is a non-human mammal having the DNA encoding the foreign protein of the present disclosure (hereinafter, also called "foreign DNA of the present disclosure), or the mutated DNA (also called "foreign mutated DNA of the present disclosure").

That is, disclosed herein are the followings:
(1) a non-human mammal having the foreign DNA of the present disclosure or the mutated DNA thereof;
(2) the animal according to the above item (1), wherein the non-human mammal is a rodent;
(3) the animal according to the above item (2), wherein the rodent is a mouse or a rat; and
(4) a recombinant vector containing the foreign DNA of the present disclosure or the mutated DNA thereof, which can be expressed in a mammal.

The non-human mammal having the foreign DNA of the present disclosure or the mutated DNA thereof (hereinafter, also called "DNA-transferred animal of the present disclosure") can be produced by transferring the desired DNA to germinal cell including unfertilized egg, fertilized egg, sperm and initial cell thereof, preferably in the stage of embryo development in development of non-human mammal (more preferably, in the stage of single cell or fertilized egg cell, and generally before 8-cell stage), by calcium phosphate method, electrical pulse method, ripofection method, coagulation method, micro-injection method, particle gun method, DEAE-dextran method, and the like. In addition, the desired foreign DNA of the present disclosure can be used in cell culture, tissue culture, and the like, by transferring the desired foreign DNA of the present disclosure to somatic cell, living organ, tissue cell, and the like by said DNA-transfer method, and further, by fusing these cells with the above-described germinal cell by the known cell fusion method per se, the DNA-transferred animal of the present disclosure can be produced.

As the non-human mammal, for example, bovine, pig, sheep, goat, rabbit, dog, cat, guinea pig, hamster, mouse, rat, and the like are used. Among them, in view of production of a disease state animal model line, rodent where ontogenetic and biological cycles are comparatively short and breeding is easy, in particular, mouse (for example, pure line such as C57BL/6 strain, DBA2 strain, and the like, crossline such as B6C3F1 strain, BDF1 strain, B6D2F1 strain, BALB/c strain, ICR strain, and the like), or rat (for example, Wistar, SD, and the like) is preferable.

As "mammal" in the recombinant vector which can be expressed in mammal, besides the above-described non-human mammals, human and the like are exemplified.

The foreign DNA of the present disclosure is not the DNA of the present disclosure essentially possessed by non-human mammal, but the DNA of the present disclosure which is once isolated / extracted from mammal.

As the mutated DNA of the present disclosure, a DNA where a variation (for example, mutation, and the like) has occurred in the base sequence of the original DNA of the present disclosure, specifically, a DNA where addition or defect of base, substitution by other base, and the like has occurred is used, and abnormal DNA is also included.

Said abnormal DNA means a DNA which expresses the abnormal DNA of the present disclosure, for example, a DNA which expresses a protein inhibiting the function of the normal protein of the present disclosure, and the like are used.

The foreign DNA of the present disclosure may be the one derived from either mammal of the same species to or different species from the target animal. When the DNA of the present disclosure is transferred to target animal, it is generally advantageous to use the DNA as a DNA-construct where said DNA is bound to a promoter which can express said DNA in animal cell, at the down-stream thereof. For example, when human DNA of the present disclosure is transferred, said DNA is bound to various promoter at the down-stream thereof to form a DNA-construct (for example, vector, and the like). Here, the promoter can express DNA derived from various kinds of mammals (for example, rabbit, dog, cat, guinea pig, hamster, rat, mouse, and the like) having the DNA of the present disclosure having high homology to the human DNA. By microinjection of the DNA-construct to fertilized egg of target mammal, for example, fertilized egg of mouse, DNA-transferred mammal which can highly express the DNA of the present disclosure can be produced.

As the expression vector of the protein of the present disclosure, bacteriophage such as plasmid derived from Bacillus coli, plasmid derived from Bacillus subtilis, plasmid derived from yeast, λ phage; retrovirus such as a Moloney leukemia virus; animal virus such as vaccinia virus, or baculo virus, and the like are used. Among them, plasmid derived from Bacillus coli, plasmid derived from Bacillus subtilis or plasmid derived from yeast is preferably used.

As the above-described promoter which regulates expression of DNA, for example, promoters such as (i) DNA promoter derived from virus (for example, simian virus, cytomegalovirus, Moloney leukemia virus, Jc virus, breast cancer virus, poliovirus, and the like) ; (ii) promoter derived from various mammals (human, rabbit, dog, cat, guinea pig, hamster, rat, mouse, and the like); for example, albumin, insulin II, uroplakin II, elastase, erythropoietin, endothelin, muscular creatine kinase, glial fibrillary acidic protein, glutathione-s-transferase, platelet-derived growth factor β, keratin K1, K10 and K14, collagen I-type, and II-type, cyclic AMP dependent protein kinase βI-subunit, dystrophin, tartrate-resistant alkali phophatase, atrial natriuretic factor, endothelium receptor tyrosine kinase (generally abbreviated as "Tie 2"), sodium potassium adenosine 3-phosphorylated enzyme (Na,K-ATPase), neurofilament light chain, metallothionein I and IIA, metalloproteinase 1-tissue inhibitor, MHC class I antigen(H-2L), H-ras, renin, dopamine β-hydroxylated enzyme, thyroid peroxidase (TPO), peptide chain elongation factor 1α (EF-1α), β-actin, α- and β-myosin heavy chain, myosin light chain 1 and 2, myelin fundamental protein, thyroglobulin, Thy-1, immunoglobulin, H-chain variable part (VNP), serum amyloid P-component, myoglobin, troponin C, smooth muscle α-actin, pre-pro-enkephalin A, vasopressin, and the like are used. Among them, cytomegalovirus promoter which can highly express throughout the body, promoter of human peptide chain elongation factor 1α (EF-1α), human and chicken β-actin promoter, and the like are preferable.

The above-described vector preferably contain a sequence (generally, called "terminator") which terminate the transcription of the desired mRNA in the DNA-transferred mammal. For example, each of DNA sequences derived from virus and various mammals can be used, and preferably SV40 terminator of simian virus and the like are used.

In addition, for the purpose of expressing more highly the desired foreign DNA, splicing signal, enhancer region, a part of intron of eukaryotic DNA, and the like of each DNA can be connected at 5' up-stream of promoter region, between promoter region and translation region, or at 3' down-stream of translation region depending on the purpose.

Translation region of the normal protein of the present disclosure can be obtained as a whole or a part of genome DNA from DNA derived from liver, kidney, thyroid gland cell, fibroblast derived from human or various mammals (for example, rabbit, dog, cat, guinea pig, hamster, rat, mouse, and the like), and commercially available various genome DNA libraries; or from complementary DNA prepared from RNA derived from liver, kidney, thyroid gland cell and fibroblast as a raw material by a known method. Also, the abnormal foreign DNA can be produced by mutating a translation region of the normal protein obtained from the above-described cell or tissue by point mutagenesis method.

Said translation region can be produced by connecting at down-stream of the above-described promoter, and if necessary, at up-stream of the transcription-termination site as a DNA construct which can express in the transferred animal, by a usual DNA-engineering procedure.

Transference of the foreign DNA of the present disclosure in the stage of amphicytula ensures that the foreign DNA can exist in all of germinal cells and body cells of the target mammal. Existence of the foreign DNA of the present disclosure in the germinal cell of the produced animal after the transference of DNA means that all of the progenies of the produced animal maintain the foreign DNA of the present disclosure in all of germinal cells and body cells. Progeny of this kind of animal inheriting the foreign DNA of the present disclosure contains the foreign DNA of the present disclosure in all of germinal cells and body cells thereof.

Non-human mammals to which the foreign normal DNA of the present disclosure is transferred can be sub-bred in a common breeding environment as an animal containing said DNA, after confirming that the foreign DNA is stably kept by cross-breeding.

Transference of the foreign DNA of the present disclosure in the stage of amphicytula ensures that the foreign DNA can excessively exist in all of germinal cells and body cells of the target mammal. Excessive existence of the foreign DNA of the present disclosure in the germinal cell of the produced animal after the transference of DNA means that all of the progenies of the produced animal excessively maintain the foreign DNA of the present disclosure in all of germinal cells and body cells. Progeny of this kind of animal inheriting the foreign DNA of the present disclosure excessively contains the foreign DNA of the present disclosure in all of germinal cells and body cells thereof.

Homozygote animal which contains the introduced DNA in both of homologous chromosomes is obtained, and by cross-breeding male and female of this animal, sub-breeding of this animal can be done so that all progenies excessively contain said DNA.

In non-human mammal having the normal DNA of the present disclosure, the normal DNA of the present disclosure is highly expressed. Therefore, the non-human mammal has a possibility to develop finally hyper-function syndrome of the protein of the present disclosure due to promoted function of the endogenous normal DNA, and can be utilized as a disease state model animal thereof. For example, it is possible to carry out clarification of disease mechanism about hyper-function syndrome of the protein of the present disclosure and diseases related to the protein of the present disclosure and investigation of treatment method of these diseases, by using the normal DNA-transferred animal of the present disclosure.

In addition, since the mammal to which the foreign normal DNA of the present disclosure is transferred has a symptom of the increased free protein of the present disclosure, the mammal can be utilized for screening test of a prophylactic / therapeutic agent for the disease related to the protein of the present disclosure, for example, a prophylactic / therapeutic agent for cancer (for example, colorectal cancer, breast cancer, lung cancer, prostate cancers, esophageal cancer, gastric cancer, liver cancer, biliary tract cancer, spleen cancer, kidney cancer, bladder cancer, uterine cancers, ovarian cancer, testicular cancer, thyroid cancer, pancreatic cancer, brain tumor, hematopoietics tumor (in particular, AML), or the like.).

On the other hand, non-human mammal having the foreign abnormal DNA of the present disclosure can be sub-bred in a common breeding environment as an animal containing said DNA, after confirming that the foreign DNA is stably kept by cross-breeding. Further, the animal can be used as a raw material, by incorporating the desired foreign DNA to the above-described plasmid. The DNA-construct with a promoter can be produced by a common DNA-engineering method.
Transference of the abnormal DNA of the present disclosure in the stage of amphicytula ensures that the abnormal DNA can exist in all of germinal cells and body cells of the target mammal. Existence of the abnormal DNA of the present disclosure in the germinal cell of the produced animal after the transference of DNA means that all of the progenies of the produced animal maintain the abnormal DNA of the present disclosure in all of germinal cells and body cells. Progeny of this kind of animal inheriting the foreign DNA of the present disclosure contains the abnormal DNA of the present disclosure in all of germinal cells and body cells thereof. Homozygote animal which contains the introduced DNA in both of homologous chromosomes is obtained, and by cross-breeding male and female of this animal, sub-breeding of this animal can be done so that all progenies contain said DNA.

In non-human mammal having the abnormal DNA of the present disclosure, the abnormal DNA of the present disclosure is highly expressed. Therefore, the non-human mammal can finally become functionally-inactive insensitivity syndrome of the protein of the present disclosure due to inhibition of the function of the endogenous normal DNA, and can be utilized as a disease state model animal thereof. For example, it is possible to carry out clarification of disease mechanism about functionally-inactive insensitivity syndrome of the protein of the present disclosure and investigation of treatment method of this disease, by using the abnormal DNA-transferred animal of the present disclosure.

In addition, as specific availability, the animal which highly expresses the abnormal DNA of the present disclosure can become a model to clarify the functional inhibition (dominant negative action) to the normal protein by the abnormal protein of the present disclosure in the functionally-inactive insensitivity syndrome of the protein of the present disclosure.

Further, the mammal to which the foreign abnormal DNA of the present disclosure is transferred can be also utilized for screening test of a prophylactic / therapeutic agent for the functionally-inactive insensitivity syndrome of the protein of the present disclosure, for example, a prophylactic / therapeutic agent for cancer (for example, colorectal cancer, breast cancer, lung cancer, prostate cancers, esophageal cancer, gastric cancer, liver cancer, biliary tract cancer, spleen cancer, kidney cancer, bladder cancer, uterine cancers, ovarian cancer, testicular cancer, thyroid cancer, pancreatic cancer, brain tumor, hematopoietics tumor (in particular, AML), or the like.).

Also, as other applicability of the above-described 2 kinds of DNA-transferred animals, for example, the followings are considered:
(i) use as a cell source for tissue culture;
(ii) analysis of relationship with a peptide which is specifically expressed or activated by the protein of the present disclosure through direct analysis of DNA or RNA in the tissue of the DNA-transferred animal of the present disclosure, or analysis of peptide tissue expressed by the DNA;
(iii) investigation of the function of a cell from a tissue which is generally difficult to culture, by culturing a cell of a tissue having DNA by a standard tissue culture technology, then using this;
(iv) screening of a medicament which enhance cell function by using the cell according to the above-described item (iii); and
(v) isolation / purification of the mutated protein of the present disclosure and production of the antibody thereof; and the like.

Further, by using the DNA-transferred animal of the present disclosure, clinical symptom of the disease related to the protein of the present disclosure including the functionally-inactive insensitivity syndrome, and the like can be examined. Also, more detailed pathological findings on each organ of a disease model related to the protein of the present disclosure can be obtained, and thus the animal can contribute to development of a novel treatment method, and further to investigation and treatment of secondary condition by said disease.

Also, it is possible to obtain a free DNA-transferred cell by taking out each organ from the DNA-transferring animal of the present disclosure, slicing, and after that using an enzyme such as trypsin, and carry out culture thereof or systematization of the cultured cell. Further, it is possible to specify the cell producing the protein of the present disclosure, investigate relationship with cell adhesion and the like, or signal transfer mechanism in these cells, and examine their abnormality. Thus, the animal becomes an effective study material for clarifying the protein of the present disclosure and the action thereof.

Moreover, it becomes possible to provide an effective and rapid screening method using the above-described examination method and determination method, in order to promote development of treatment agent for diseases relating to the protein of the present disclosure including functionally-inactive insensitivity syndrome of the protein of the present disclosure by using the DNA-transferred animal of the present disclosure. Also, it is possible to examine / develop a DNA-treatment method for diseases relating to the protein of the present disclosure, by using the DNA-transferred animal of the present disclosure or the foreign DNA expression vector of the present disclosure.

### (7) Knockout animal

Disclosed herein is an embryonic stem cell of non-human mammal where the DNA of the present disclosure has been inactivated, and a DNA expression incomplete non-human mammal of the present disclosure.

That is, disclosed herein is:
(1) an embryonic stem cell of non-human mammal wherein the DNA of the present disclosure has been inactivated;
(2) the embryonic stem cell according to the item (1), wherein said DNA has been inactivated by introducing reporter gene (for example, β-galactosidase gene derived from Bacillus coli);
(3) the embryonic stem cell according to the item (1), which is resistant to neomycin;
(4) the embryonic stem cell according to the item (1), wherein the non-human mammal is a rodent;
(5) the embryonic stem cell according to the item (4), wherein the rodent is a mouse;
(6) a DNA expression incomplete non-human mammal of the present disclosure, wherein the DNA of the present disclosure has been inactivated;
(7) the non-human mammal according to the item (6), whrein said DNA has been inactivated by introducing a reporter gene (for example, β-galactosidase gene derived from Bacillus coli), and said reporter gene can express under the control of a promoter to the DNA of the present disclosure;
(8) the non-human mammal according to the item (6), wherein the non-human mammal is a rodent;
(9) the non-human mammal according to the item (8), wherein the rodent is a mouse; and
(10) a method for screening a compound promoting or inhibiting activity of a promoter to the DNA of the present disclosure, by administrating a test compound to the animal according to the item (7), and detecting expression of the reporter gene.

The embryonic stem cell of non-human mammal wherein the DNA of the present disclosure has been inactivated means an embryonic stem cell of non-human mammal (hereinafter, also called "ES cell") where the DNA has substantially no expressional potency (hereinafter, also called "knockout DNA of the present disclosure", by inhibiting expressional potency of the DNA or substantially losing activity of the protein of the present disclosure encoded by said DNA by adding artificial variation to the DNA of the present disclosure possessed by said non-human mammal.

As the non-human mammal, the same one as described above is used.

The method for adding artificial variation to the DNA of the present disclosure can be carried out, for example, by deleting a part of the whole of said DNA sequence by a gene-engineering procedure, or inserting or substituting other DNA. The knockout DNA of the present disclosure may be produced by these variations, for example, by shifting reading frame of codon or destructing function of promoter or exon.

As a specific example of the method for obtaining the embryonic stem cell of non-human mammal wherein the DNA of the present disclosure has been inactivated (hereinafter, also called "ES cell where the DNA of the present disclosure has been inactivated" or "knockout ES cell of the present disclosure disclosure"), the cell can be obtained, for example, by isolating the DNA of the present disclosure possessed by the target non-human mammal, introducing the DNA-chain (hereinafter, also called "targeting vector") having a DNA sequence constructed so as to destroy a gene as a result by destroying the function of exon by inserting to the exon part thereof a drug-resistant gene represented by neomycin-resistant gene and hygromycin-resistant gene, or a reporter gene represented by lacZ (β-galactosidase gene), cat (chloramphenicol acetyltransferase gene), and the like, or making it impossible to synthesize a complete mRNA by inserting into an intron part between exons a DNA sequence (for example, poly-A-addition signal, and the like) to terminate the transcription of the gene, into a chromosome of said animal, for example, by homologous recombination method, analyzing the obtained ES cell by southern hybridization analysis using the DNA sequence on the DNA of the present disclosure or in the neighborhood thereof as a probe, or PCR method using DNA sequence on the targeting vector and a DNA sequence in the adjacent region other than the DNA of the present disclosure used for producing the targeting vector as a primer, and selecting the knockout cell of the present disclosure.

In addition, as the original ES cell inactivating the DNA of the present disclosure by homologous recombination method, and the like, for example, the one described above which has been already established may be used, or a newly established one according to the known Evans and Kaufman method may be used. For example, in the case of mouse ES cell which is commonly used at present, immunologic background is not clear. For the purpose of obtaining an ES cell of which line is pure and genetic background is immunologically clear, instead of the above mouse ES cell, for example, an ES cell established by using C57BL/6 mouse, BDF1 mouse (F1 of C57BL/6 and DBA/2) where poor egg collection number of C57BL/6 has been improved by cross-breeding with DBA/2, and the like can be preferably used. Besides the merits that BDF1 mouse has greater egg collection number and egg thereof is hard, since BDF1 mouse has the background of C57BL/6 mouse, ES cell obtained using this can be advantageously used in view of that the ES cell can change genetic background thereof to C57BL/6 mouse by back-crossing when disease model mouse is produced.

Further, when ES cell is established, blastocyst of 3,5 days after fertilization is generally used, but except this, it is possible to obtain many early embryos more efficiently by collecting eight-cell embryo and using after culturing this until blastocyst.

Also, any one of female or male ES cell may be used, but generally male ES cell is advantageous to produce germ line chimera. Further, to reduce labor of the complicated culture, it is desirable to discriminate female or male as early as possible.

As an example of method for determining female or male of ES cell, for example, a method where gene in the sex-determining region on Y chromosome is amplified and detected by PCR method can be exemplified. Conventionally about 106 cells were required for karyotype analysis, whereas about 1 colony level of ES cell number (about 50 cells) is enough by using this method, therefore, it is possible that first selection of ES cell in the initial stage of culture is carried out by sex-determination, labor in the initial stage of culture can be drastically reduced by making it possible to select male cell early.

In addition, second selection can be carried out, for example, by identification of chromosome number by G-banding method, and the like. Chromosome number of the obtained ES cell is desirably 100% of normal number. When this rate is difficult to obtain because of physical operation in establishing stage, and the like, it is desirable to carry out the cloning again in normal cell (for example, cell having 2n = 40 of chromosome number in mouse) after knocking out the gene of ES cell.

As for the embryonic stem cell strain obtained by such a method, productivity thereof is usually excellent, but capability of ontogeny tends to be lost, therefore, careful subculture is needed. For example, the embryonic stem cell is cultured by a method where the cell is cultured on an appropriate feeder cell such as STO fibroblast, in the presence of LIF (1∼10000 U/ml), in a carbon dioxide incubator (preferably, 5% of carbonic acid, 95% of air, or 5% of oxygen, 5% of carbon dioxide, 90% of air), at about 37 °C, and the like. When sub-cultured, for example, a method where the cell is unicellularized by treatment with trypsin / EDTA solution (usually, 0.001 to 0.5% of trypsin / 0.1 to 5mM of EDTA, preferably about 0.1% of trypsin / 1mM of EDTA), and then seeding on a newly prepared feeder cell, and the like are employed. Such sub-culture is usually carried out every 1 to 3 days, and on this occasion, cell is observed, and when morphologically abnormal cell is observed, it is desirable to abandon this cultured cell.

The ES cell can be differentiated to various types of cells such as occipitofrontalis muscle, visceral muscle, heart muscle, and the like, by carrying out monolayer culture under appropriate conditions until reaching high density, or carry out floating culture until forming cell clump [M. J. Evans and M. H. Kaufman, Nature, Vol. 292, page 154, 1981; G. R. Martin, Proc. Natl. Acad. Sci. U.S.A. Vol. 78, page 7634, 1981; T. C. Doetschman et al., Journal of embryology and experimental morphology, Vol. 87, page 27, 1985]. DNA expression incomplete cell of the present disclosure obtained by differentiating ES cell of the present disclosure is useful in in vitro cytological investigation of the protein of the present disclosure.

The DNA expression incomplete non-human mammal of the present disclosure can be distinguished from normal animal by measuring amount of mRNA of said animal by the known method, and comparing indirectly expression level thereof.

As said non-human mammal, same one as described above is used.

As for the DNA expression incomplete non-human mammal of the present disclosure, the DNA of the present disclosure can be knocked out, for example, by introducing the targeting vector produced as described above into embryonic stem cell of mouse or egg cell of mouse, and carrying out gene homologous recombination where a DNA sequence of the DNA of the present disclosure inactivated by the introduction in targeting vector is replaced to the DNA of the present disclosure on the chromosome of embryonic stem cell of mouse or egg cell of mouse.

The cell where the DNA of the present disclosure has been knocked out can be determined by southern hybridization analysis using the DNA sequence on the DNA of the present disclosure or in the neighborhood thereof as a probe, or by analysis by PCR method using DNA sequence on the targeting vector and a DNA sequence in the adjacent region other than the DNA of the present disclosure derived from mouse used for producing the targeting vector as a primer. When non-human mammal embryonic stem cell is used, cell strain where DNA of the present disclosure has been inactivated by gene-homologous recombination is cloned, the cell is injected at an appropriate time, for example, at 8-cell stage of non-human mammal embryo or blastocyst, the produced chimera embryo is transplanted to the uterus of said non-human mammal which becomes pseudopregnancy. The produced animal is a chimera animal which is composed of both cells of normal DNA locus of the present disclosure, and artificially mutated DNA locus of the present disclosure.

When a part of germ cell of said chimera animal has mutated DNA locus of the present disclosure, from the population obtained by crossbreeding this chimera individual and normal individual, an individual where all tissues are composed of cells having artificially mutated DNA locus of the present disclosure can be obtained, for example, by selecting by coat color judgment, and the like. The individual obtained in this way is usually hetero-expression incomplete individual of the protein of the present disclosure. The hetero-expression incomplete individuals are cross-bred each other, and homo-expression incomplete individual of the protein of the present disclosure can be obtained from these progenies thereof.

When egg cell is used, for example, by injecting DNA solution to egg cell nucleus by micro-injection method, a transgenic non-human mammal where targeting vector is introduced to chromosome can be obtained. Comparing these transgenic nonhuman mammals, the one having mutated DNA locus of the present disclosure by gene homologous recombination can be obtained by selection.

The individual where DNA of the present disclosure has been knocked out in this way can be sub-bred in usual breeding environment, after confirming that said DNA has been knocked out in animal individual obtained by crossbreeding.

Further, obtaining and maintaining of germ line may be followed by common method. That is, by cross-breeding female and male animals having said inactivated DNA, homozygote animal having said inactivated DNA in both of homologous chromosomes can be obtained. The obtained homozygote animal can be effectively obtained by breeding under such condition that 1 of normal individual and plural of homozygote based on mother animal. By cross-breeding female and male of heterozygote animals, homozygote and heterozygote animals having said inactivated DNA are sub-bred.

The non-human mammal embryonic stem cell where the DNA of the present disclosure is inactivated is very useful for producing a DNA expression incomplete non-human mammal of the present disclosure.

In addition, since in the DNA expression incomplete non-human mammal of the present disclosure, various biological activities induced by the protein of the present disclosure are deleted, this mammal can be a model of the disease caused by inactivated biological activity of the protein of the present disclosure, and is useful for investing cause of these diseases and study of treatment method.
(9a) Screening method for a compound having treatment / prevention effect for diseases caused by deletion, damage, or the like of DNA of the present disclosure

The DNA expression incomplete non-human mammal of the present disclosure can be used for screening of a compound having treatment / prevention effect for diseases caused by deletion, damage, or the like of DNA of the present disclosure.

That is, according to the present disclosure, there is provided a screening method for a compound having treatment / prevention effect for diseases (for example, cancer and the like) caused by deletion, damage, or the like of DNA of the present disclosure or the salt thereof, by administering a test compound to the DNA expression incomplete non-human mammal of the present disclosure, and observing / measuring change of said animal.

As the DNA expression incomplete non-human mammal of the present disclosure to be used in said screening method, the same one as described above can be exemplified.

The test compound includes, for example, peptide, protein, non-peptide compound, synthetic compound, fermentation product, cell extract, plant extract, animal tissue extract, blood plasma, and the like. These compounds may be a novel compound, or a known compound.

Specifically, treatment / prevention effect of the test compound can be tested, by treating the DNA expression incomplete non-human mammal of the present disclosure with a test compound, comparing with untreated control animal using change in each organ, tissue, symptom of disease of said animal as an index.

As the method for treating test animal with a test compound, for example, oral administration, intravenous injection, and the like are used, and can be appropriately selected according to the symptoms of test animal, property of test compound, and the like. Moreover, administration amount of test compound can be appropriately selected according to administration method, property of test compound, and the like.

For example, when a compound having treatment and prevention effects for cancer (for example, colorectal cancer, breast cancer, lung cancer, prostate cancers, esophageal cancer, gastric cancer, liver cancer, biliary tract cancer, spleen cancer, kidney cancer, bladder cancer, uterine cancers, ovarian cancer, testicular cancer, thyroid cancer, pancreatic cancer, brain tumor, hematopoietics tumor, or the like.), the test compound is administered to the DNA expression incomplete non-human mammal of the present disclosure, difference in degree of cancer development and difference in healing degree of cancer are observed in the above-described tissues with time comparing with non-administration group of test compound.

When a test compound is administered to a test animal in said screening method, said test compound can be selected as a compound having treatment and prevention effects for the above-described disease when the above-described disease symptom of said test animal is improved by about 10% or more, preferably about 30% or more, more preferably about 50% or more.

The compound obtained by using said screening method is a compound selected from the above-described test compounds. Since the compound has treatment / prevention effect for the disease caused by deletion or damage of the protein of the present disclosure, it can be used as a medicament such as a safe and low-toxic prophylactic / therapeutic agent, and the like for said disease. Further, the compound derived from the compound obtained by the above-described screening can similarly be used.

The compound obtained by said screening method may be in a form of salt, and as a salt of said compound, a salt formed with physiologically acceptable acid (for example, inorganic acid, organic acid, and the like), or base (for example, alkali metal, and the like), and the like are used, in particular, physiologically acceptable acid addition salt is preferable. As such salts, for example, salt with inorganic acid (for example, hydrochloric acid, phosphoric acid, hydrobromic acid, sulfuric acid, and the like), salt with organic acid (for example, acetic acid, formic acid, propionic acid, fumaric acid, maleic acid, succinic acid, tartaric acid, citric acid, malic acid, oxalic acid, benzoic acid, methane sulfonic acid, benzene sulfonic acid, and the like), and the like are used.

Medicament containing the compound obtained by said screening method or the salt thereof can be produced similarly to the medicament containing the above-described protein of the present disclosure.

Since a preparation obtained in this manner is safe and low-toxic, it can be administered, for example, to human or mammal (for example, rat, mouse, guinea pig, rabbit, sheep, pig, bovine, house, cat, dog, ape, and the like).

Dosage of said compound or the salt thereof varies depending on target disease, target to be administered, administration route, and the like, but, for example, when said compound is administered orally, generally in an adult of breast cancer patient (provided that body weight is 60 kg), said compound of about 0.1 to about 100 mg, preferably about 1.0 to about 50 mg, more preferably about 1.0 to about 20 mg per day is administered. In the case of parenteral administration, single dosage of said compound varied depending on target to be administered, target disease, and the like, but, for example, in the case of administration to an adult of breast cancer patient (provided that body weight is 60 kg) in the form of injectable solution, it is advantageous to administer said compound of about 0.01 to about 30 mg, preferably about 0.1 to about 20 mg, more preferably about 0.1 to about 10 mg per day by intravenous injection. In the case of other animal, an amount which is converted based on 60 kg of body weight can be administered.
(9b) Screening method for a compound promoting or inhibiting the activity of promoter to the DNA of the present disclosure

According to the present disclosure, there is provided a screening method for a compound promoting or inhibiting the activity of promoter to the DNA of the present disclosure or the salt thereof, by administering a test compound to the DNA expression incomplete non-human mammal, and detecting expression of reporter gene.

In the above-described screening method, as the DNA expression incomplete non-human mammal of the present disclosure, among the above-described DNA expression incomplete non-human mammals of the present disclosure, the mammal where the DNA of the present disclosure is inactivated by introducing reporter gene, and said reporter gene can be expressed under the control of a promoter to the DNA of the present disclosure is used.

As the test compound, the same one as described above is exemplified.

As the reporter gene, the same one as described above is used, and β-galactosidase gene (lacZ), soluble alkaliphosphatase gene or luciferase gene, and the like are preferable.

In the DNA expression incomplete non-human mammal of the present disclosure where the DNA of the present disclosure is substituted by reporter gene, since the reporter gene exists under the control of the promoter to the DNA of the present disclosure, activity of the promoter can be detected by tracing expression of the material encoded by the reporter gene.

For example, when a part of DNA region encoding the protein of the present disclosure is substituted by β-galactosidase gene (lacZ), β-galactosidase expresses instead of the protein of the present disclosure in the tissues where the protein of the present disclosure should be originally expressed. Therefore, for example, by staining using a reagent which becomes a substrate of β-galactosidase such as 5-bromo-4-chloro-3-indolyl-β-galactopyranoside (X-gal), expression state of the protein of the present disclosure in living body of animal can easily be observed. Specifically, the protein of the present disclosure deficient mouse or the tissue fragment thereof is immobilized with glutaraldehyde, or the like, and after washing with phosphate buffered saline (PBS), reacted in a staining liquid containing X-gal at room temperature or at about 37°C for about 30 minutes to 1 hour. After that, tissue specimen is washed with 1 mM EDTA / PBS solution to stop β-galactosidase reaction, coloring can be observed. Also, according to the common method, mRNA encoding lacZ may be detected.

The compound obtained by the above-described method or the salt thereof is a compound selected from the above-described test compounds, which promotes or inhibits activity of the promoter to the DNA of the present disclosure.

The compound obtained by said screening method may be in a form of salt, and as a salt of said compound, salt formed with physiologically acceptable acid (for example, inorganic acid, and the like), or base (for example, alkali metal, and the like), and the like are used. In particular, physiologically acceptable acid addition salt is preferable. As such salt, for example, salt with inorganic acid (for example, hydrochloric acid, phosphoric acid, hydrobromic acid, sulfuric acid, and the like), or salt with organic acid (for example, acetic acid, formic acid, propionic acid, fumaric acid, maleic acid, succinic acid, tartaric acid, citric acid, malic acid, oxalic acid, benzoic acid, methane sulfonic acid, benzene sulfonic acid, and the like), and the like are used.

Since the compound inhibiting activity of the promoter to the DNA of the present disclosure or the salt thereof can inhibit expression of the protein of the present disclosure, or can inhibit function of said protein, the compound is useful as a prophylactic / therapeutic agent for cancer (for example, a large bowel cancer, a breast cancer, a lung cancer, a prostate cancer, an esophageal cancer, a gastric cancer, a liver cancer, a biliary tract cancer, a spleen cancer, a kidney cancer, a bladder cancer, an uterine cancer, an ovarian cancer, a testicular cancer, a thyroid cancer, a pancreatic cancer, a brain tumor, a hematopoietic tumor(particularly AML), and the like).

Further, a compound derived from the compound obtained by the above-described screening can be similarly used.

A medicament containing the compound obtained by said screening method or the salt thereof can be produced in the same way as in the medicament containing the above-described protein of the present disclosure or the salt thereof.

Since the preparation obtained by such method is stable and low-toxic, it can be administered, for example, to human or mammal (for example, rat, mouse, guinea pig, rabbit, sheep, pig, bovine, horse, cat, dog, ape, and the like).

Dosage of said compound or the salt thereof varies depending on target disease, target to be administered, administration route, and the like, but, for example, when the compound inhibiting activity of the promoter to the DNA of the present disclosure is administered orally, generally for an adult of breast cancer patient (provided that body weight is 60 kg), said compound of about 0.1 to about 100 mg, preferably about 1.0 to about 50 mg, more preferably about 1.0 to about 20 mg per day is administered. In the case of parenteral administration, single dosage of said compound varies depending on target to be administered, target disease, and the like, but, for example, in the case of administering a compound inhibiting activity of the promoter to the DNA of the present disclosure to an adult of breast cancer patient (provided that body weight is 60 kg) in a form of injectable solution, it is advantageous that said compound of about 0.01 to about 30 mg, preferably about 0.1 to about 20 mg, more preferably about 0.1 to about 10 mg per day is administered by intravenous injection. In the case of other animal, amount which is converted based on 60 kg of body weight can be administered. In case of the other animal, the amount which is converted based on 60 kg of weight can be administered.

As described above, the DNA expression incomplete non-human mammal of the present disclosure is extremely useful for screening the compound promoting or inhibiting activity of the promoter to the DNA of the present disclosure, and can greatly contribute to investigate the cause of various diseases associated with the DNA expression incompleteness of the present disclosure or to develop a prophylactic / therapeutic agent.

In addition, by using DNA which contains the promoter region of the protein of the present disclosure, connecting a gene encoding various proteins at the down-stream thereof, and introducing to an egg cell of an animal to produce so-called transgenic animal (gene-transferred animal), it becomes possible to synthesize the protein specifically, and investigate the function in living body. Further, by binding an appropriate reporter gene to the part of the above-described promoter, and establishing a cell strain which can express this gene, this can be used as a searching system for a low-molecular weight of compound having the function which specifically promotes or inhibits the production capability of the protein of the present disclosure itself in the body.

In this specification, when a base or an amino acid is described as an abbreviation, the description is based on an abbreviation according to IUPAC-IUB Commission on Biochemical Nomenclature or a common abbreviation in the relevant field. Also, when an amino acid can include an optical isomer, the description represents L-isomer unless otherwise specified.

SEQ ID Nos in the sequence table in the specification of the present application show the following sequences:
[SEQ ID No: 1]
   Base sequence of the DNA (CDS) encoding EVI1 is shown.
[SEQ ID No: 2]
   Amino acid sequence of EVI1 is shown.
[SEQ ID No: 3]
   Base sequence of the DNA (CDS) encoding ITGA6 is shown.
[SEQ ID No: 4]
   Amino acid sequence of ITGA6 is shown.
[SEQ ID No: 5]
   Base sequence of DNA (CDS) encoding ITGB4 is shown.
[SEQ ID No: 6]
   Amino acid sequence of ITGB4 is shown.
[SEQ ID No: 7]
   Sequence of RNA forming shEVI1 together with SEQ ID No: 8 is shown.
[SEQ ID No: 8]
   Sequence of RNA forming shEVI1 together with SEQ ID No: 7 is shown.
[SEQ ID No: 9]
   Base sequence of the forward primer used in amplification of human EVI1 gene is shown.
[SEQ ID No: 10]
   Base sequence of the reverse primer used in amplification of human EVI1 gene is shown.
[SEQ ID No: 11]
   Base sequence of the forward primer used in amplification of human β-actin gene is shown.
[SEQ ID No: 12]
   Base sequence of the reverse primer used in amplification of human β-actin gene is shown.
[SEQ ID No: 13]
   Sequence of RNA forming shEVI1 together with SEQ ID No: 14 is shown.
[SEQ ID No: 14]
   Sequence of RNA forming shEVI1 together with SEQ ID No: 13 is shown.

### EXAMPLE

Hereinafter, the present invention is explained more specifically by means of Examples and Reference Examples, but the scope of the present invention is not limited thereto.

It should be noted that among the cell lines used in the following Examples, U937, K562, HNT34, HL60, 293T and MC3T3 were purchased from the cell bank of RIKEN. In addition, MOLM1 was purchased from Hayashibara Biochemical Labs., Inc. Furthermore, AML1 was supplied by University of California, San Diego (UCSD). Of these, U937, K562, HNT34, HL60, MOLM1 and AML1 were cultured in RPMI1640 (189-02025, produced by Wako Pure Chemical Industries, Ltd.) added with 10% fetal bovine serum (FCS) (produced by Nichirei Corp.). MC3T3 was cultured in αMEM (12561, produced by Invitrogen Corp.) added with 10% fetal bovine serum (FCS) (produced by Nichirei Corp.). 293T was cultured in DMEM/10% FCS.

### Example 1-1: Inhibition of expression of integrin gene in AML1 cell line by transduction of shRNA of EVI1 gene.

### (Establishment of EVIl-expression-inhibited AML1 cell)

EVI1 expression-inhibited AML1 cell line (hereinafter, also called "AML1 (shEVI1)") was established by the transfection of shEVI1, which is a short hairpin RNA (shRNA) having cleavage activity of mRNA of EVI1 gene, into the AML1 cell line, which was originated from human acute myelocytic leukemia (AML).

Specifically, at first, shEVI1 (sense strand: GAUCUCUCUAAGGCUGAACUAGCAGUCAAGAGACUGCUAGUUCAGCCUUAGAUUUUUUG (SEQ ID No: 7) and antisense strand: AAUUCAAAAAAUCUAAGGCUGAACUAGCAGUCUCUUGAACUGCUAGUUCAGCCUUAGAGA (SEQ ID No: 8), which had been disclosed in Oncogene, 2006 Jun 15; 25 (25) : 3565-75), were inserted into pSIREN-RetroQ-ZsGreen vector of RNAi-Ready pSIREN-RetroQ-ZsGreen (produced by Clonetech Laboratories, Inc.) at BamH1-EcoR1 site to prepare a shEVI1 retrovector. After that, 10 µg of the shEVI1 retrovector and 10 µg of p10A1 packaging vector (produced by Clonetech Laboratories, Inc.) were transduced to 293T cell using Hylimax liposome reagent (produced by DOJINDO Laboratories) to prepare a retrovirus containing shEVI1. After that, AML1 (shEVI1)was established by infecting the obtained retrovirus into 10⁵ of AML1 cells. It should be noted that as a control cell, the cell (hereinafter, also called "AML1 (shLuc) ") was established by transfecting a control vector (shLuc) attached to RNAi-Ready pSIREN-RetroQ-ZsGreen (produced by Clonetech Laboratories, Inc.) by the same method.

### (Confirmation of the inhibition of integrin expression)

Each cell cultured steadily of the established above mentioned AML1(shEVI1) and AML1(shLuc) was recovered by centrifugation, the medium was removed, and 1 ml of Trizol™ (produced by Invitrogen Corp.) was added. After that, 500 µL of chloroform was added, separated by centrifugation at 15000 rpm for 15 minutes, the upper layer was transferred to another tube, 500 µL of 2-propanol was added, separated by centrifugation at 15000 rpm for 10 minutes, and washed with 80% ethanol. The resultant RNA was dissolved in RNase-free water, the amount of RNA was measured by spectrophotometer at wavelength of 260nm, and 1 µg of the RNA was transformed to cDNA with reverse transcriptase (superscript 3, produced by Invitrogen Corp.).

Using each of the resultant cDNA as a template, the cDNA was amplified by PCR method, and expression level of the genes was confirmed by agarose gel electrophoresis. Specifically, TaKaRa Extaq (produced by TaKaRa Bio Inc.) was used as the polymerase, and PCR Thermal Cycler DICE (produced by TaKaRa Bio Inc.) was used as the PCR equipment. In addition, each cDNA was corrected by β-actin before the amplification by PCR. Base sequences of the primers used in the PCR were as follows; (Human EVI1)
Forward: CGAAAGCGAGAATGATCTCC (SEQ ID No: 9);
Reverse: GGAAGACGTAGTGCTGAACA (SEQ ID No: 10); (Human β-actin)
Forward: AAGAGATGGCCACGGCTGCT (SEQ ID No: 11);
Reverse: TCCTTCTGCATCCTGTCGGC (SEQ ID No: 12); (Integrin families)
The ones disclosed in Journal of Biomedical Science (2005)12:803-813.

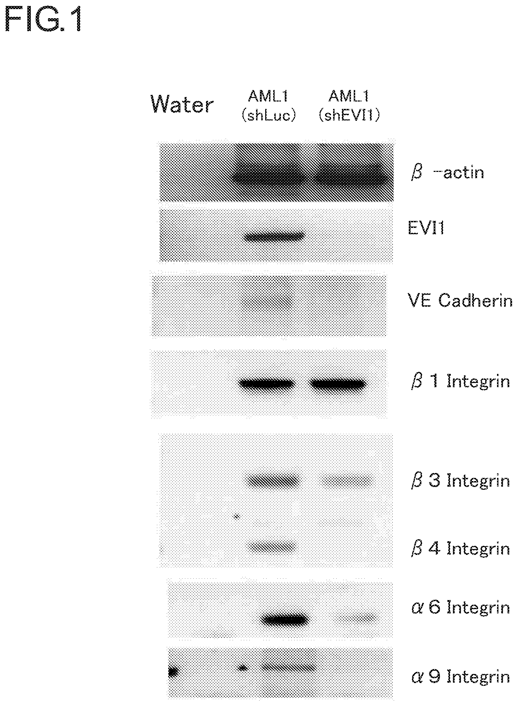

The result of the agarose gel electrophoresis is shown in Figure 1. As shown in the Figure 1, EVI1 gene expression was inhibited in AML1(shEVI1) compared with AML1(shluc). This is considered due to an action of RNA interference (RNAi) by the addition of shEVL1. In addition, as shown in Figure 1, inhibition of gene expressions of various integrin families including ITGA6 gene and ITGB4 gene was observed in AML1(shEVI1) compared with AML1(shLuc). Furthermore, expression of VE cadoherin gene, a kind of cadherin family, was revealed also to be inhibited. These were considered to be caused by the inhibition of the expression of the EVI1 gene.

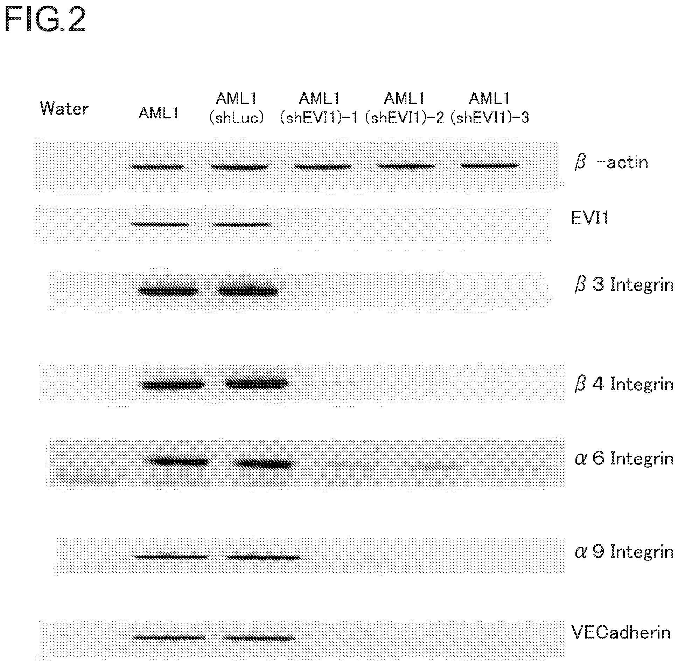

Further, the result of triplicated experiment for AML1(shEVI1) is shown in Figure 2, in which the AML1 cell not administered with shLuc or shEVI1 was added as a control. As shown in the Figure 2, the inhibition of the EVI1 gene expression, and the inhibition of the expression of various integrin families and VE cadherin genes associated therewith were confirmed.

### Example 1-2: Decrease in cellular adhesiveness of AML1 cell line by the administration of shRNA of EVI1 gene

Cellular adhesiveness was evaluated for the above-established AML1(shEVI1) (triplicate) and AML1(shLuc).

Specifically, 100 µL of 40 times-diluted growth factor reduced matrigel (produced by BD Biosciences) with RPMI1640/10% FCS was added to each well of a 96-well plate, incubated at 4°C for 6 hours, and washed twice with PBS. The above-mentioned cell was added to each of the wells at 10000cells/100 µL. After that, the cell was incubated at 37°C for 2 hours and washed three times with PBS. After that, 90 µL of RPMI1640 / 10%FCS and 10 µL of Cell counting kit 8 (produced by DOJINDO Laboratories) were added to each well, incubated at 37°C for 30 minutes and measured at wavelength of 450 nm by a spectrophotometer.

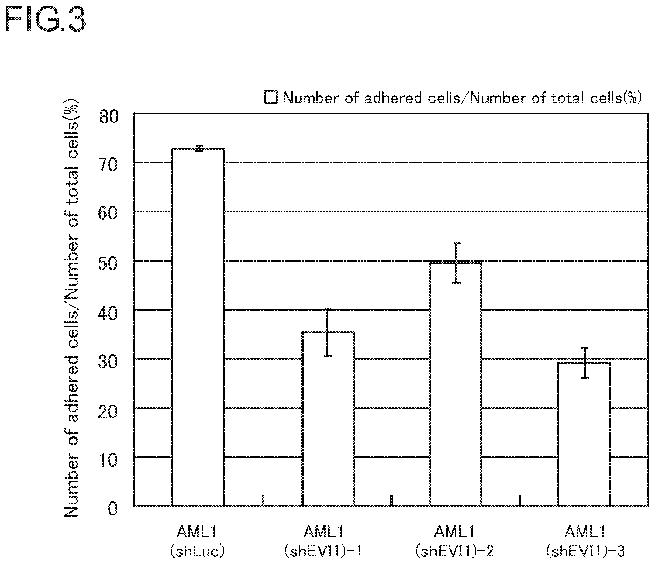

Figure 3 is a graph showing the result of the above-mentioned cellular adhesiveness assay. As shown in Figure 3, cellular adhesiveness (ratio of the number of cells adhered to the number of total cells) of AML1(shEVI1) was revealed to decrease by about 31 to 60% compared with that of AML1(shLuc). Based on that, the present inventors set a hypothesis that the EVI1 gene and protein encoded by it, genes belonging to various integrin families and proteins encoded by them may be involved in the exertion of cellular adhesiveness in acute myelocytic leukemia cell.

### Reference Example 1: Comparison of integrin family genes among various cells

As cell lines not expressing the EVI1 gene, steadily cultured HL60 cell line derived from human, U937 cell line derived from human, and K562 cell line derived from human were prepared. On the other hand, as cell lines expressing the EVI1 gene, steadily cultured AML1 cell line derived from human acute myelocytic leukemia (above-mentioned), MOLM1 cell line derived from human, and HNT34 cell line derived from human were prepared. For these cell lines, expression of the EVI1 gene and genes of various integrin families were confirmed by the same method as in the Example 1-1.

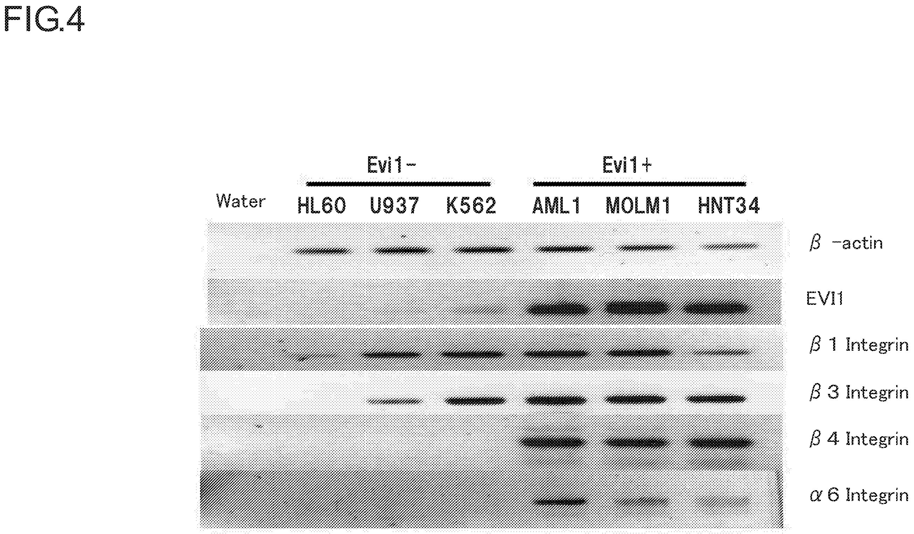

The results of the agarose gel electrophoresis are shown in Figure 4. As shown in Figure 4, ITGA6 gene and ITGB4 gene were revealed to express specifically in EVIl-positive cell lines (AML1, MOLM1 and HNT34). Based on the result, the present inventors set a hypothesis that, in addition to the EVI1 gene, in particular, ITGA6 gene and ITGB4 gene and proteins encoded by the genes among the integrin families may be involved in the expression of cellular adhesiveness in acute myelocytic leukemia.

### Example 2-1: Inhibition of expression of integrin genes in HNT34 cell line by transfection of shEVI1

EVI1 expression-inhibited HNT34 cell line (hereinafter, also called "HNT34(shEVI1)") was established by the transfection of shEVI1, which was used in the above-mentioned Example 1-1, using Nucleofector (produced by Amaxa Biosystems), a gene transfection system, into HNT34 cell line derived from human. In the same way, the cell line transfected with shLuc (hereinafter, also called "HNT34 (shLuc)") was established. For these cell lines, expression of EVI1 gene and genes of various integrin families were confirmed by the same method as in the Example 1.

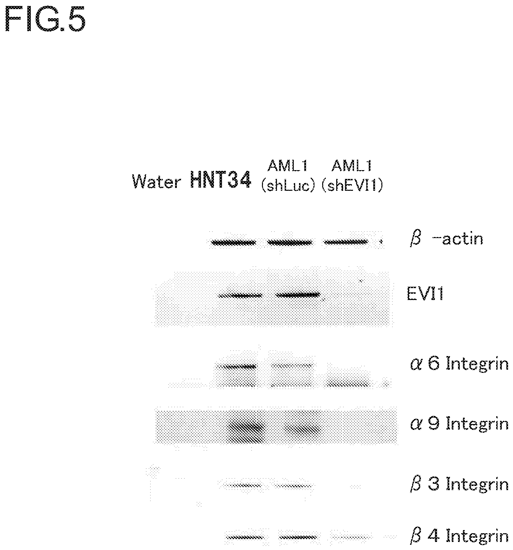

The results of the agarose gel electrophoresis are shown in Figure 5. As shown in Figure 5, expression of the EVI1 gene and genes of various integrin families were confirmed to be inhibited by the transfection of shEVI1 into HNT34 cell line with Nucleofector, too, similarly to those in the above-mentioned Example 1-1 and Example 1-2.

### Example 2-2: Decrease of cellular adhesiveness of HNT34 cell line by administration of shRNA of EVI1 gene

The cellular adhesiveness was evaluated for the above-mentioned established HNT34(shEVI1) and HNT34(shLuc) by the same method as the above-mentioned Example 1-2.

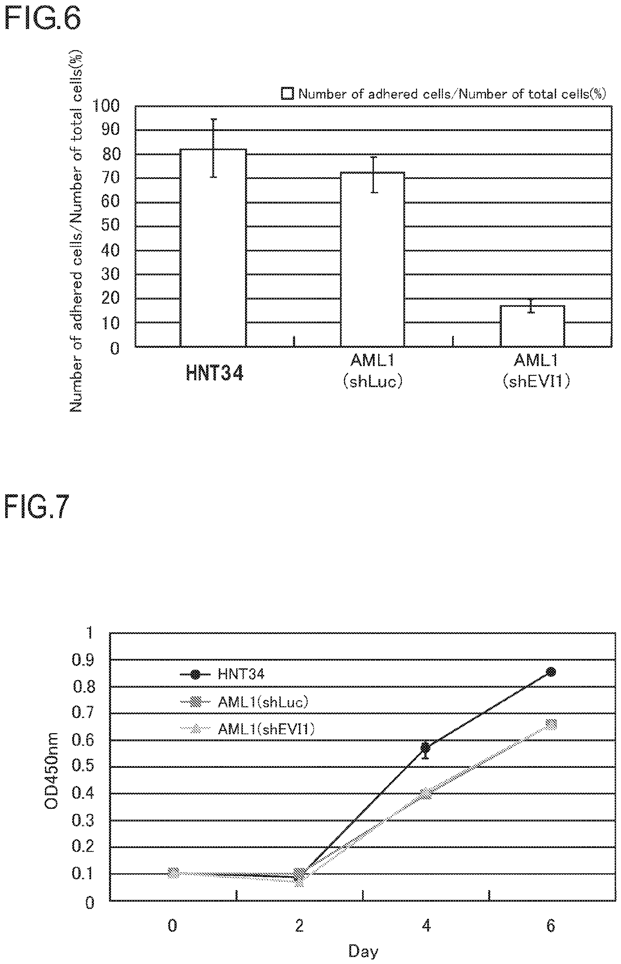

Figure 6 shows the results of the above-mentioned cellular adhesiveness assay. As shown in Figure 6, cellular adhesiveness of HNT34 (shEVI1) (ratio of the number of adhered cells to the number of total cells) was revealed to decrease by about 76% compared with that of HNT34(shLuc). As described above, similar results were obtained in HNT34 cell line to the above-mentioned Example 1-2.

### Reference Example 2: Effect of administration of shRNA of EVI1 gene on cellular proliferative ability of HNT34 cell line

Cellular proliferative ability was evaluated for the above-mentioned established HNT34(shEVI1) and HNT34(shLuc).

Specificaaly, cells diluted with RPMI1640/10% FCS were added to each well of a 96-well plate at 1000 cells/100 µL. After that, the cell suspension was incubated at 37°C for an appropriate time, each 10 µL of Cell counting kit 8 (produced by DOJINDO Laboratories) was added, incubated at 37°C for 30 minutes, and measurement was carried out at wavelength of 450 nm using a spectrophotometer.

Figure 7 is a graph showing the results of the above-mentioned cellular proliferation ability assay. As shown in Figure 7, no significant difference in cellular proliferation ability was observed between HNT34 (shLuc) as a control cell line and HNT34 (shEVI1) as a EVI1 expression-inhibited cell line. Based on the result, EVI1 gene is considered not to be involved in cell proliferation.

### Example 3-1: Decrease of cellular adhesiveness of AML1 cell line by administration of shRNA of ITGB4 gene

ITGB4 expression-inhibited AML1 cell line (hereinafter also called "AML1(shITGB4)") was established by the transfection of shRNA having an activity of cleaving mRNA of ITGB4 gene (hereinafter also called "shITGB4") into AML1 cell line by the same method as the above-mentioned Example 2-1. It should be noted that the base sequences of the used shITGB4 were as follows.
Sense strand: GAUCUCCGAGUUGCGUGGGGUGUGUCUUCAAGAGAGA CACACUCCGUGCAGCUCUUUUUG (SEQ ID No: 13);
Antisense strand: AAUUCAAAAAGAGCUGCACGGAGUGUGUCUCUCUUG AAGACACACCCCACGCAACUCGGA (SEQ ID No: 14).

As a control cell, AML1(shLuc) established in the above-mentioned Example 1-1 was used.

Cellular adhesiveness was evaluated for the established above-mentioned AML1(shITGB4) and AML1(shLuc) by the same method as the above-mentioned Example 1-2.

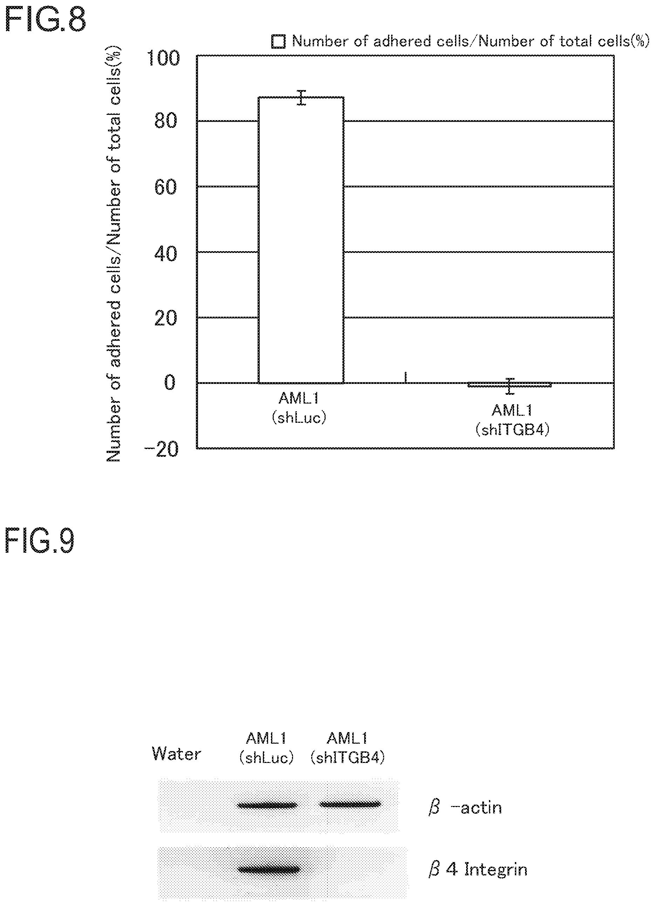

Figure 8 is a graph showing the results of the above-mentioned cellular adhesiveness assay. As shown in Figure 8, cellular adhesiveness of AML1(shITGB4) (ratio of the number of adhered cells to the number of total cells) was revealed to decrease nearly to zero to that of AML1 (shLuc) . Based on the result, the hypothesis that the ITGB4 gene is involved in the exertion of cellular adhesiveness in acute myelocytic leukemia cell was substantiated.

Further, expression of ITGB4 gene in AML1 (shITGB4) was confirmed by the same method as the above-mentioned Example 1-1. As a result, as shown in the results of PCR agarose gel electrophoresis in Figure 9, expression of ITGB4 gene was suppressed in AML1(shITGB4) compared with AML1(shLuc).

### Example 3-2: Decrease of cellular adhesiveness of AML1 cell line by β4 integrin neutralizing antibody

The effect of administration of β4 integrin neutralizing antibody to AML1 cell line, which was originated from human, was evaluated on the cellular adhesiveness of the cell line.

Specifically, β4 integrin clone ASC3 antibody (produced by Nihon Millipore KK, MAB2058) was added to 10000 cells/100 µL so as to be 20 µg/mL of the final concentration, incubated at 4°C for 30 minutes, and washed with RPMI1640/10% FCS. After that, the cell was suspended with 100 µL of RPM1640 / 10%FCS, and added to the wells of Matrigel™-coated 96-well plate, and incubated at 37°C for 2 hours, after that, washed with PBS was carried out three times. Each 90 µL of RPM1640 / 10%FCS and 10 µL of Cell counting kit 8 (produced by DOJINDO Laboratories) was added, and incubated at 37°C for 30 minutes, after that measured at wavelength of 450 nm using a spectrophotometer. It should be noted that, as a control, a cell line administered with anti-FLAG M2 antibody or anti-β1 integrin antibody was used in place of the β4 integrin clone ASC3 antibody.

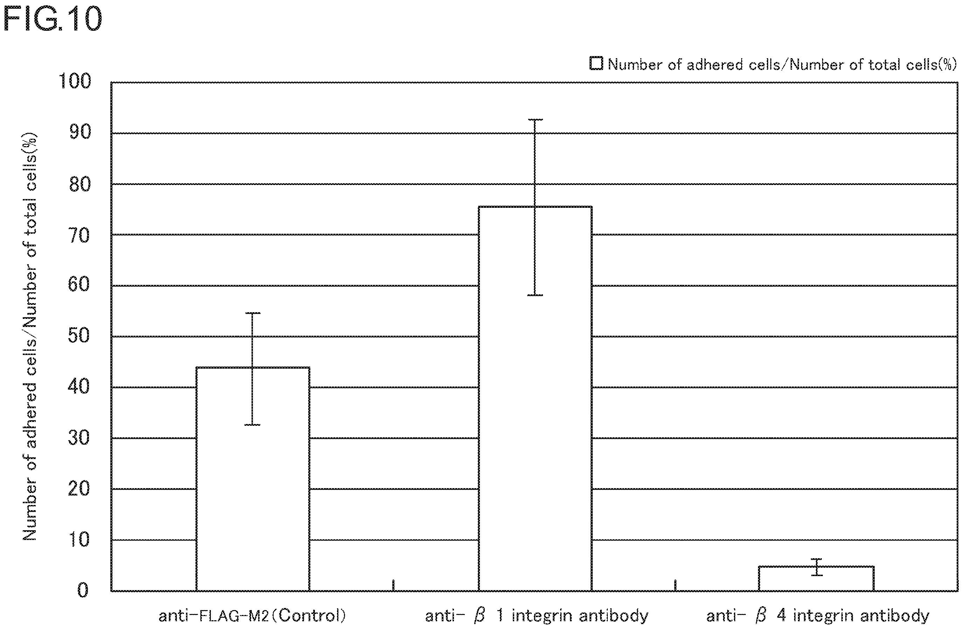

Figure 10 is a graph showing the results of the above-mentioned cellular adhesiveness assay. As shown in Figure 10, cellular adhesiveness of AML1 (ratio of the number of adhered cells to the number of total cells) was revealed to decrease by about 90% and about 94% to that of the group administered by anti-FLAG M2 and the group administered by anti-β1 integrin antibody, respectively. Based on this result, the hypothesis that ITGB4 protein is involved in the exertion of cellular adhesiveness of acute myelocytic leukemia cell was substantiated.

### Reference Example 3-1: Enhancement of integrin gene expression in U937 cell line by the transfection of EVI1 gene

The examination was carried out whether the expressions of various integrin family genes are enhanced by the transfection of EVI1 gene into U937 cell line, which did not express the EVI1 gene.

Specifically, EVI1 gene-expressing U937 cell line (hereinafter, also called "U937(EVI1)") was established by the transfection of EVI1 gene into U937 cell, and expressions of various integrin family genes in the U937(EVI1) cell line were examined. It should be noted that, as a control, U937 cell line transfeced with GFP gene (hereinafter also called "U937(GFP)") was used.

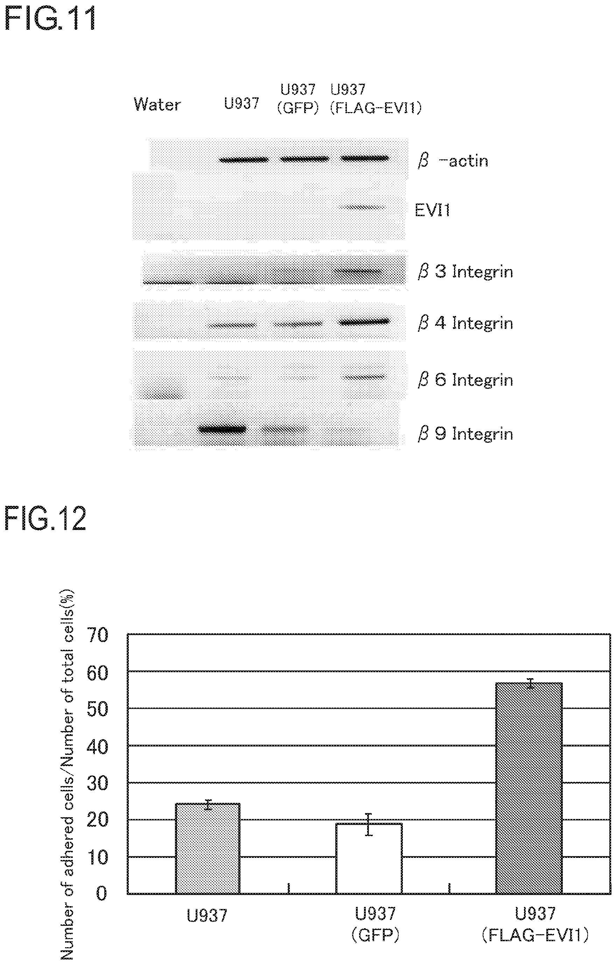

The results of agarose gel electrophoresis are shown in Figure 11. As shown in Figure 11, expressions of ITGA6 gene, ITGB3 gene and ITGB4 gene were revealed to be enhanced by the transfection of EVI1 gene into EVIl-negative U937 cell line.

### Reference Example 3-2: Improvement in cellular adhesiveness of U937 cell line by the transfection of EVI1 gene

Cellular adhesiveness was evaluated for U937 cell line, as well as U937(GFP), which was the above-established control, and U937(FLAG-EVI1), which was a cell line transfected with EVI1, by the same method as the above-mentioned Example 2-1.

Figure 12 is a graph showing the results of the above-mentioned cellular adhesiveness assay. As shown in Figure 12, cellular adhesiveness of U937 (FLAG-EVI1) (ratio of the number of adhered cells to the number of total cells) was revealed to improve by about 2.3 times and about 3.0 times to that of U937 cell line and U937(GFP), respectively. Based on the result, expression of EVI1 gene was shown to involve in the improvement of cellular adhesiveness through the expression of integrin.

### Reference Example3-3: Effect of the transfection of EVI1 gene on cellular proliferative ability of U937

Cellular proliferative ability of U937, as well as U937(GFP), which was the above-established control, and U937 (FLAG-EVI1), which was the EVIl-transfected cell line, was evaluated by the same method as the above-mentioned Reference Example 2.

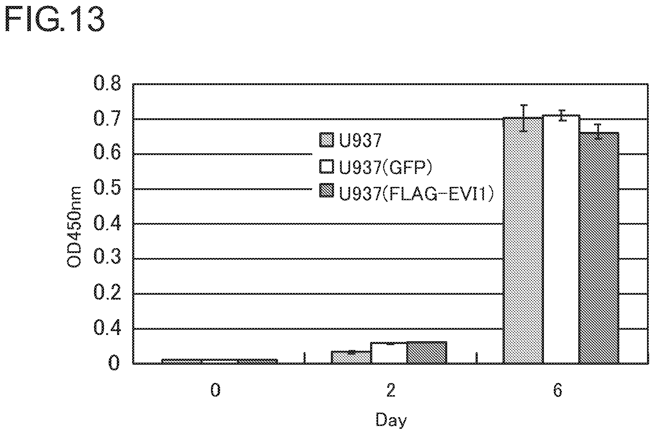

Figure 13 is a graph showing the results of the above-mentioned cellular proliferative ability assay. As shown in Figure 13, no significant difference in cellular proliferative ability was observed between U937 cell line or U937(GFP), which was a control cell line, and U937(FLAG-EVI1), an EVI1expression-enhanced cell line. Based on this result, there is a high possibility that EVI1 gene is not involved in the proliferation of a cell.

### Reference Example 4-1: Correlation between the expression of EVI1 gene, and ITGA6 gene and ITGB4 gene

As the cell lines absolutely or almost not expressing EVI1 gene, U937, K562, KG1, HEL, HL-60, K052, THP-1, FKH-1, K051, NH and OIH-1 were provided. On the other hand, as the cell lines highly expressing EVI1 gene, AML-1, HNT-34, Kasumi3 and MOLM-1were provided.

Expression of EVI1 gene and various integrin family genes were confirmed for the various cell lines provided.

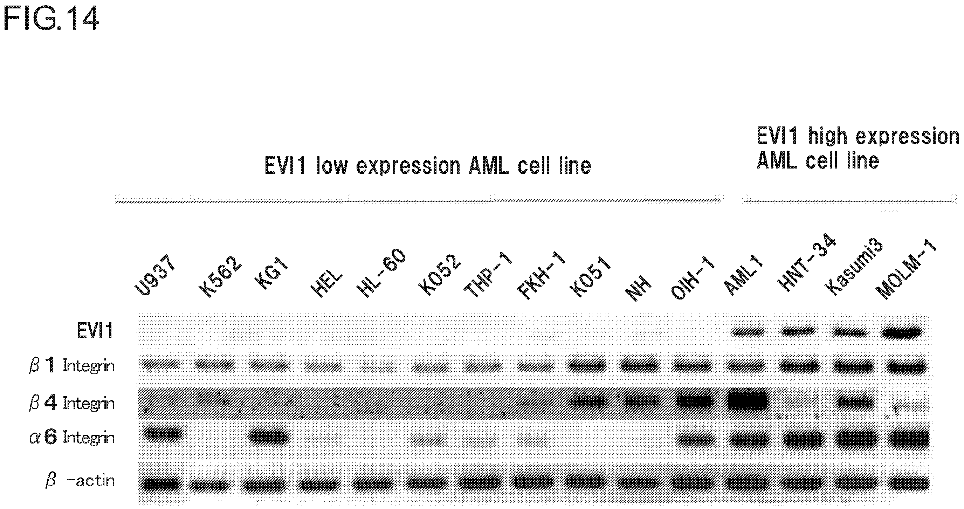

The results of agarose gel electrophoresis are shown in Figure 14. As shown in Figure 14, expression was observed both in ITGA6 gene and ITGB4 gene in the cell lines in which EVI1 gene was highly expressed (AML1, HNT34, kasumi3 and MOLM1), and showed that there was a correlation between these expressions.

### Reference Example 4-2: Correlation between the expressions of EVI1 gene, and ITGA6 gene or ITGB4 gene in clinical samples

As clinical samples, 3 samples of acute myelocytic leukemia (AML) cell expressing no EVI1 gene, 4 samples of t(3;7) AML cell, which is a kind of AML cell highly expressing EVI1 gene, and likewise, 3 samples of AML cell of 3q21q26 syndrome, a kind of AML cell highly expressing EVI1 gene, were prepared. It should be noted that as control cell lines, Bone Marrow (BM) cell expressing no EVI1 gene and CD34-positive cell line highly expressing EVI1 gene were prepared together with them.

Expression of EVI1 gene and each integrin family gene in the samples and control cell lines prepared above were confirmed.

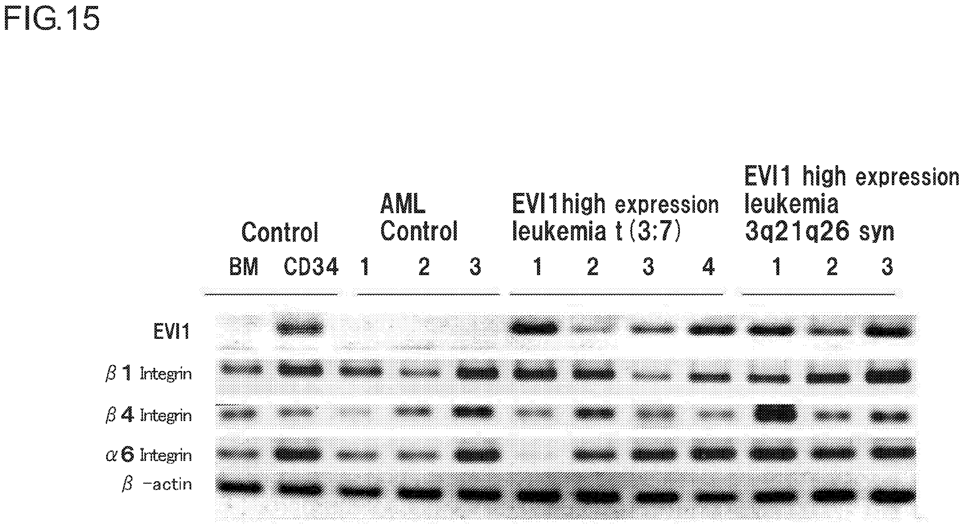

The results of agarose gel electrophoresis are shown in Figure 15. As shown in Figure 15, a tendency of the expression was observed both in ITGA6 gene and ITGB4 gene in the cell line of clinical samples in which EVI1 gene was highly expressed, and showed that there was a correlation between these expressions.

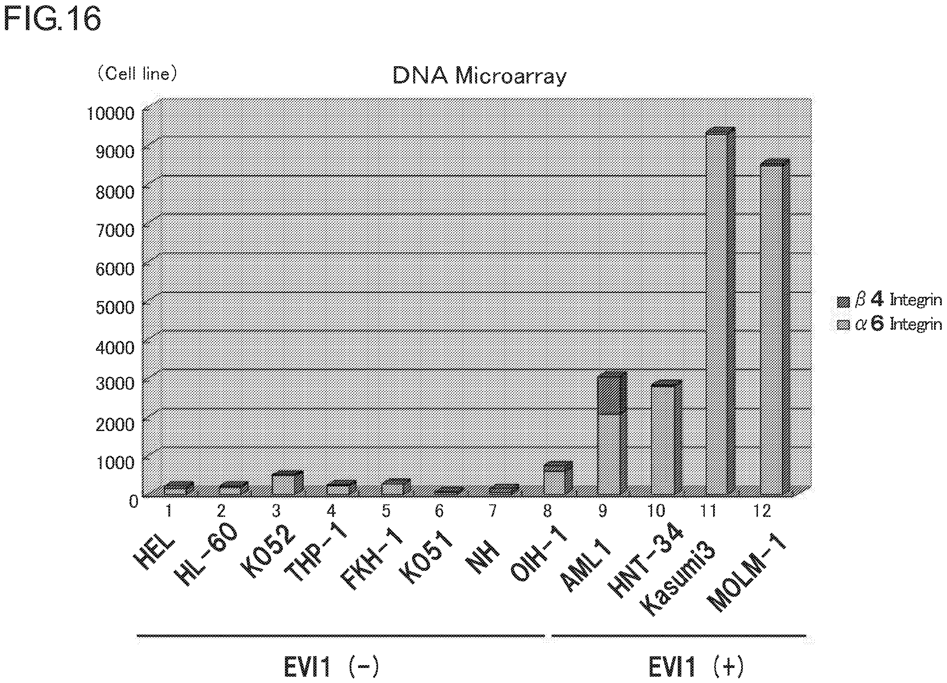

### Reference Example 4-3: Confirmation of expression of ITGA6 gene and ITGB4 gene in various cell lines using DNA microarray (Figure 16)

As cell lines expressing no EVI1 gene, HEL, HL60, K052, THP-1, FKH-1, K051, NH and OIH-1 were prepared. On the other hand, as cell lines highly expressing EVI1 gene, AML-1, HNT-34, MOLM-1 and Kasumi3 were prepared. Expression of ITGA6 gene and ITGB4 gene in these cell lines was detected by DNA microarray according to the manual of Gene-chip produced by Affymetrix, Inc.

Specifically, at first, cDNA was prepared from each of the cells and synthesis of labeled cRNA with biotin was carried out using One-cycle target label kit (produced by Affymetrix, Inc.). After that, 30 µL of the prepared cRNA (15 µg) was reacted at 94°C for 35 minutes for fragmentation, and added to the hybridization solution (300 µL). After that, the solution was reacted at 99°C for 5 minutes and at 45°C for 5 minutes, then centrifuged, and the supernatant was hybridized with human genome gene-chip (U133A). After the washing, the resultant solution was subjected to scanning using gene-chip scanner (produced by Affymetrix, Inc.) and the data was quantified. It should be noted that correction was made for the quantification of data using β-actin as a standard.

The results of the above-mentioned DNA microarray are shown in Figure 16. As shown in Figure 16, ITGA6 gene and ITGB4 gene were confirmed to be highly expressed in the group of cell lines expressing EVI1 gene, and showed that there was a correlation between these expressions.

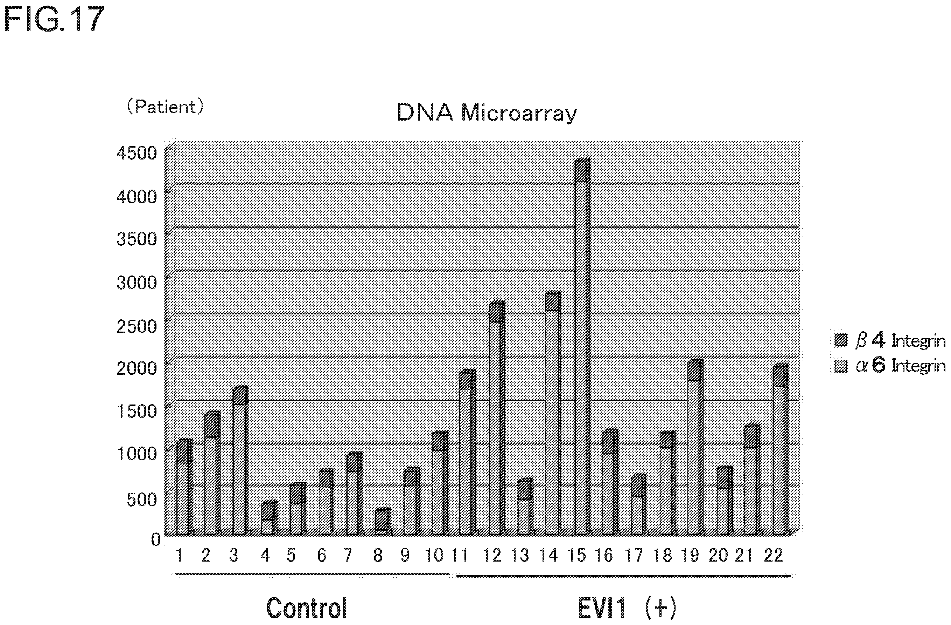

### Reference Example 4-4: Confirmation of expression of ITGA6 gene and ITGB4 gene in clinical samples using DNA microarray (Figure 17)

As clinical samples, 10 samples of acute myelocytic leukemia (AML) cell line expressing no EVI1 gene and 12 samples of AML cell line highly expressing EVI1 gene were prepared.

Expressions of ITGA6 gene and ITGB4 gene in the samples and control cell lines prepared above were confirmed using DNA microarray by the same method as the Reference Example 4-3.

The results of the above-mentioned DNA microarray are shown in Figure 17. As shown in Figure 17, expressions of ITGA6 gene and ITGB4 gene were detected to be enhanced in the clinical samples highly expressing EVI1 gene, and a correlation was shown to exist between them.

### Reference Example 5: Cellular adhesion and resistance against drugs (VP16 or Ara-C) of AML1 cell line

Effect of the presence of osteoblast cell on the drug resistance of AML1 cell line derived from human acute myelocytic leukemia (AML) was examined.

Specifically, by WST method, in which water-soluble formazan was produced, viability of AML1 cell line was evaluated under the presence of VP16 (5 µM), a kind of etoposide-based anticancer drug, between the cases of presence and absence of osteoblast cell in culture medium. It should be noted that AML1 cell line is fixed to medium plate through osteoblast cell when the osteoblast cell exists in the medium. On the other hand, AML1 cell line is not fixed to medium plate and proliferates in the suspended state when the osteoblast cell does not exist in the medium.

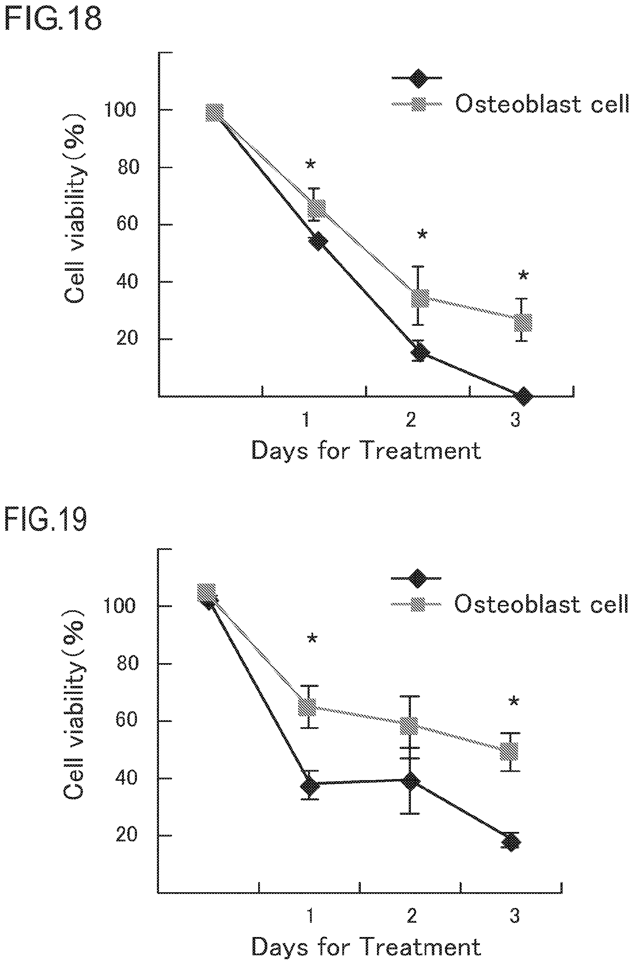

Figure 18 is a graph showing the results of the drug resistance assay by the above-mentioned WST method. As shown in Figure 18, the viability of AML1 cell line was revealed to increase significantly in the culture in the presence of VP16 when osteoblast cell existed in the medium compared with that when it did not exist. In other words, it is suggested that the presence of osteoblast (i.e. cellular adhesiveness) is essential for AML1 cell line to gain drug resistance against VP16.

In addition, effect of the presence of the osteoblast cell on the drug resistance of AML1 cell line was examined, when Ara-C, a kind of anticancer drug of differentiation-inducing type was used in place of VP16 as the anticancer drug. Specifically, the drug resistance assay was carried out by WST method using the same procedures as the above-mentioned method excepting that Ara-C (1 µg/mL) was used in place of VP16 as the anticancer drug.

Figure 19 is a graph showing the results of the drug resistance assay by the above-mentioned WST method. As shown in Figure 19, the viability of AML1 cell line was revealed to increase significantly in the culture in the presence of Ara-C, too, when osteoblast cell existed in the medium, compared with that when it did not exist. In other words, it is suggested that the presence of osteoblast (i.e. cellular adhesiveness) is essential for AML1 cell line to gain drug resistance against Ara-C, too.

### Example 4: Disappearance of osteoblast-mediated drug resistance by RNA interference of shEVI1

Drug resistance assay by WST method was carried out by the same procedures as the above-mentioned Reference Example 5 (in the presence of Ara-C) using each of the above-established AML1(shEVI1) cell and AML1(shLuc) cell in place of AML1 cell line.

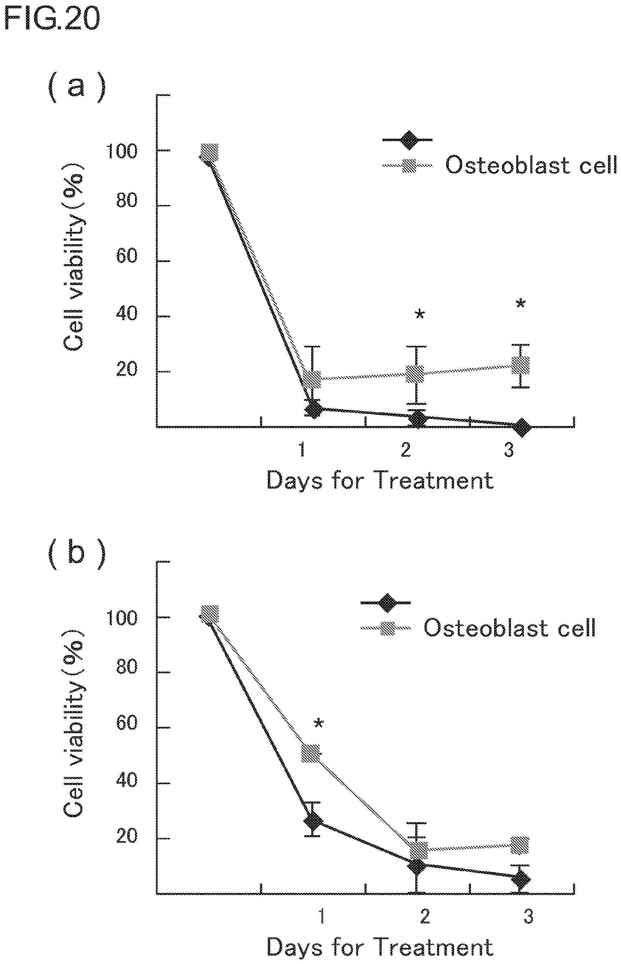

Figure 20 is a graph showing the results of the drug resistance assay by the above-mentioned WST method. Figure 20(a) is a graph showing the results of culture of AML1(shLuc), and Figure 20(b) is a graph showing the results of culture of AML1(shEVI1). Based on these results shown in Figures 20(a) and 20(b), it was revealed that drug resistance to Ara-C was gained by the existence of osteoblast in AML1(shLuc) as in the Reference Example 5 (Figure 20(a)), while the drug resistance disappeared in AML1(shEVI1). This result can be considered due to that the expression of EVI1 gene, which is regarded as a responsible gene of leukemia in AML1(shEVI1), is inhibited by the RNA interference of shEVI1, and consequently, the cellular adhesiveness through osteoblast was inhibited. Based on this result, it was shown that such antisense polynucleotide including a base sequence complementary to the base sequence of EVI1 gene like that in the present Example exerts as a cell adhesion inhibitor, thus, is useful for reducing drug resistance of a cancer cell.

### Reference Example 6: Expression of integrin gene in patients with AML

Fifty five cases of gene expression data in bone marrow cells of human AML patients were collected from Gene Expression Omnibus (GEO) of United States National Center for Biotechnology Information (NCBI). After that, these data were classified into a group of completely cured AML (30 cases) and a group of recurrent AML (25 cases), and it was evaluated statistically by unpaired t-test whether significant difference was observed between the two groups in the expression level of ITGA6 gene and ITGB4 gene.

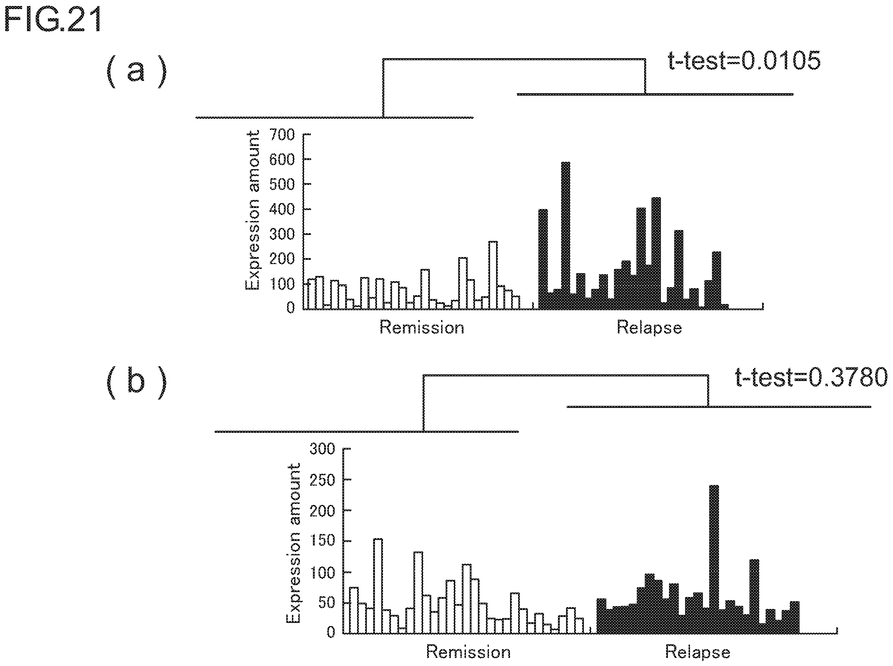

Figure 21 is a graph showing the data of expression levels collected. Figure 21(a) shows the data of expression level of ITGA6 gene, and Figure 21 (b) shows the data of expression level of ITGB4 gene. Based on the results shown in Figure 21, the expression level of ITGA6 gene was revealed to be higher significantly in the recurrent group.

### Reference Example 7: Expression of integrin gene in human myelocyte / peripheral blood cell

Thirty cases (15 kinds x 2) of the gene expression data in various human osteocyte / peripheral blood cells were collected from Gene Expression Omnibus (GEO) of NCBI. After that, the expression levels of ITGA6 gene and ITGB4 gene in these data were compared.

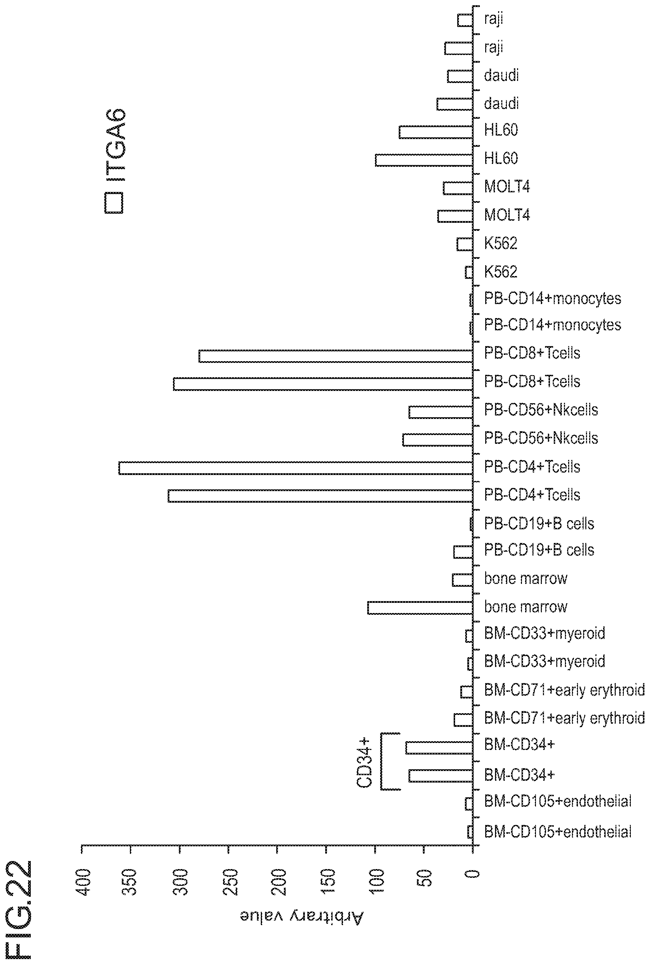

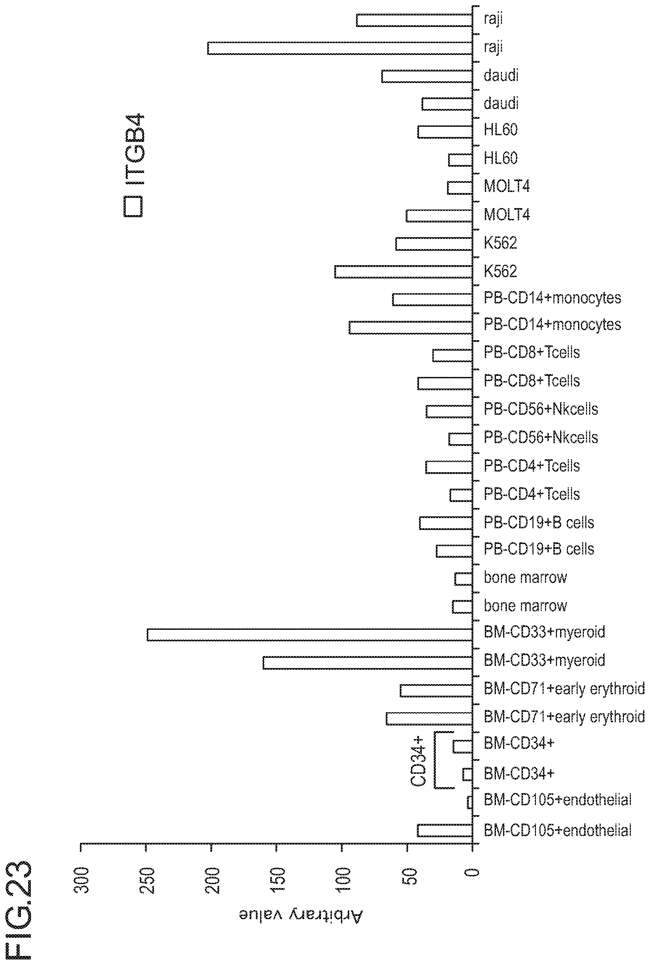

Figure 22 and Figure 23 are graphs showing the data of the expression levels collected. Figure 22 shows the data of expression level of ITGA6 gene and Figure 23 shows the data of expression level of ITGB4 gene. Based on the results shown in Figure 22 and Figure 23, both expression levels of ITGA6 gene and ITGB4 gene were revealed to be low in CD34 positive hematopoietic stem cell. Based on the results, it is expected that the cell adhesion inhibitor of the present disclosure does not act against normal hematopoietic stem cell, but can inhibit the adhesion into bone marrow by acting specifically only against AML cells.

### Reference Example 8: Expression of integrin gene in human CD34 positive cell/leukemia cell

Following the result shown in the above-mentioned Reference Example 7, expressions of integrin family genes were compared between CD34-positive cell (hematopoietic stem cell) derived from human umbilical cord blood and AML1 cell. Here, CD34-positive cell (also called "CD34+") was separated from human umbilical cord blood and cultured using Methoculture (produced by Stem Cell Institute) as a culture medium. It should be noted that comparison of expression levels of integrin genes was carried out by the PCR method that was the same as in the above-mentioned Example 1-1.

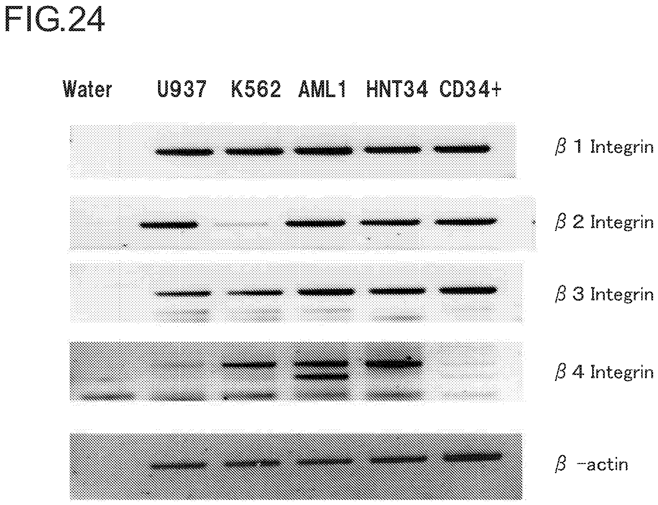

The results of agarose gel electrophoresis in the present Reference Example is shown in Figure 24. It should be noted that as mentioned above, U937 and K562 in Figure 24 were EVIl-non-expressing cell line derived from human and HNT34 is EVIl-expressing cell line derived from human. As shown in Figure 24, expression level of ITGB4 gene was confirmed to be low in CD34-positive cell (hematopoietic stem cell), and the result was consistent with the result of the above-mentioned Reference Example 7.

### Example 5: Accumulation of AML1 cell in AML1 cell-transplanted NOG mouse thighbone

The above-established AML1(shEVI1) cell was transplanted into NOG mouse™, and accumulation of the AML1 (shEVI1) cell into the murine thighbone was examined after the transplantation. Here, NOG mouse is a severe immunodeficient mouse suitable for the preparation of humanized model mouse that is available from the Central Institute for Experimental Animals.

Specifically, 6 million of each of the AML1(shLuc) cell and the AML1(shEVI1) cell was injected into NOG mouse through venous artery, and bone marrow of the thighbone was recovered 1 month after. After that, a graph representing the fluorescence intensity in the horizontal axis was prepared by FACS, and analysis was made how frequently cells were distributed from low intensity (peak near to 0) to high fluorescence intensity.

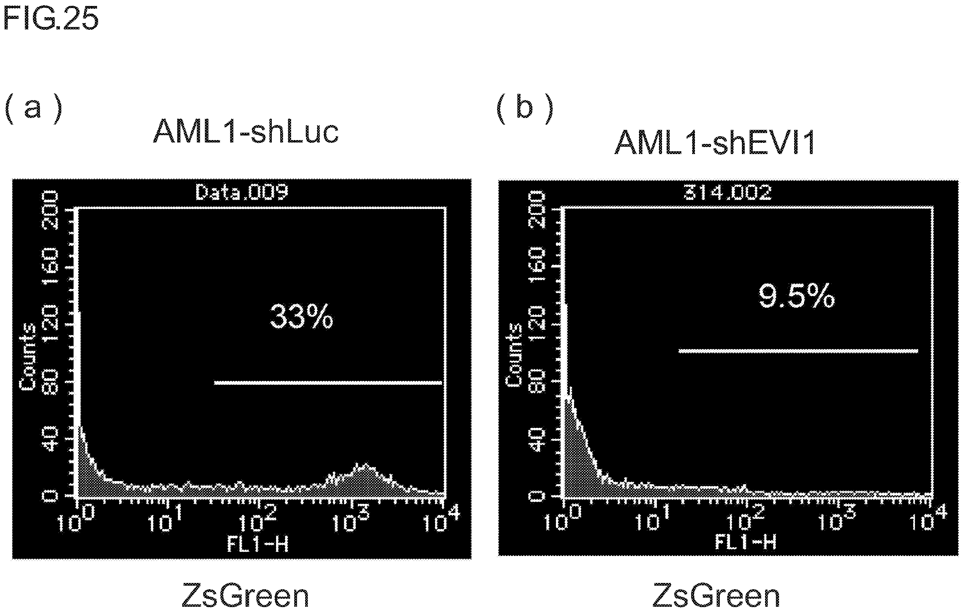

Figure 25 is a graph showing the results of the measurement by FACS method. Figure 25(a) shows the results of FACS using AML1 (shLuc) cell, and Figure 25(b) shows the results of FACS using AML1(shEVI1) cell. As shown in Figure 25, the abundance rate of AML1 (shEVI1) cell (9.5%) was revealed to be lower than that of AML1(shLuc) cell (33%). The result can be interpreted that the expression of EVI1 gene was inhibited in AML1(shEVI1) cell by RNA interference of shEVI1, consequently, expression of ITGA6/B4 was lowered, and engraftment into murine thighbone marrow was lowered. Based on the result, such an antisense polynucleotide including a base sequence complementary to the base sequence of EVI1 gene like present Example exerts an action as a cell adhesion inhibitor, thus, is shown to be useful for the prevention of accumulation of leukemia cell to bone marrow.

It should be noted that the present application is based on the Japanese Patent Application No. 2009-041204 filed on February 24, 2009.

## Claims

1. A cell adhesion inhibitor that inhibits the adhesion of leukaemia cells, for use in the prevention or treatment of acute myelocytic leukaemia, wherein the inhibitor is used in combination with an anticancer drug to enhance sensitivity of a cancer cell to the anticancer drug, wherein the anticancer drug is Ara-C, the inhibitor comprising:
(i) an antisense polynucleotide comprising a base sequence that is complementary to a target region of a base sequence of a polynucleotide encoding the amino acid sequence represented by SEQ ID NO: 2, or
(ii) an antisense polynucleotide comprising a base sequence that has at least 90% homology to the antisense polynucleotide of (i) for the over-lapped region thereof,
wherein the antisense polynucleotide is capable of inhibiting expression of said polynucleotide encoding the amino acid sequence represented by SEQ ID NO: 2.

2. A method for screening a substance to inhibit cell adhesion of a cancer cell, the method comprising using a protein comprising an amino acid sequence represented by SEQ ID No: 2 to identify a substance that inhibits the activity of said protein thereby inhibiting cell adhesion of a cancer cell.

3. The screening method according to claim 2, wherein the protein is provided in a form of a cell producing the same.

4. The screening method according to claim 2, wherein said cancer is leukaemia.

5. The screening method according to any one of claims 2 to 4, to be used for screening a substance for prevention or treatment of a cancer.

## Patentansprüche

1. Zelladhäsionshemmer, der die Adhäsion von Leukämiezellen hemmt, zur Verwendung bei der Vorbeugung oder Behandlung akuter myeloischer Leukämie, wobei der Hemmer in Kombination mit einem Antikrebsmedikament verwendet wird, um die Empfindlichkeit einer Krebszelle gegenüber dem Antikrebsmedikament zu erhöhen, wobei es sich bei dem Antikrebsmedikament um Ara-C handelt, wobei der Hemmer umfasst:
(i) ein Antisense-Polynukleotid, das eine Basensequenz umfasst, die komplementär zu einer Zielregion einer Basensequenz eines Polynukleotids ist, das die durch SEQ ID NO: 2 dargestellte Aminosäuresequenz codiert, oder
(ii) ein Antisense-Polynukleotid, das eine Basensequenz umfasst, die wenigstens 90 % Homologie zu dem Antisense-Polynukleotid von (i) aufweist, für die überlappte Region desselben,
wobei das Antisense-Polynukleotid in der Lage ist, die Expression des Polynukleotids, das die durch SEQ ID NO: 2 dargestellte Aminosäuresequenz codiert, zu hemmen.

2. Verfahren zum Screening eines Stoffes zum Hemmen der Zelladhäsion einer Krebszelle, welches die Verwendung eines Proteins umfasst, das eine durch SEQ ID NO: 2 dargestellte Aminosäuresequenz umfasst, um einen Stoff zu identifizieren, der die Aktivität des Proteins hemmt und dadurch Zelladhäsion einer Krebszelle hemmt.

3. Screening-Verfahren nach Anspruch 2, wobei das Protein in Form einer Zelle bereitgestellt wird, die dieses herstellt.

4. Screening-Verfahren nach Anspruch 2, wobei es sich bei dem Krebs um Leukämie handelt.

5. Screening-Verfahren nach einem der Ansprüche 2 bis 4, zur Verwendung zum Screening eines Stoffes zur Vorbeugung oder Behandlung eines Krebses.

## Revendications

1. Inhibiteur d'adhésion cellulaire qui inhibe l'adhésion des cellules leucémiques, pour utilisation dans le traitement prophylactique ou thérapeutique de la leucémie myéloïde aiguë, où l'inhibiteur est utilisé en combinaison avec un médicament anticancéreux pour améliorer la sensibilité d'une cellule cancéreuse au médicament anticancéreux, où le médicament anticancéreux est Ara-C, l'inhibiteur comprenant :
(i) un polynucléotide antisens comprenant une séquence de bases qui est complémentaire d'une région cible d'une séquence de bases d'un polynucléotide codant la séquence d'acides aminés répondant à SEQ ID NO: 2, ou
(ii) un polynucléotide antisens comprenant une séquence de bases qui présente une homologie d'au moins 90 % vis-à-vis du polynucléotide antisens de (i) pour la région superposée de celui-ci,
où le polynucléotide antisens est capable d'inhiber l'expression dudit polynucléotide codant la séquence d'acides aminés répondant à la SEQ ID NO: 2.

2. Méthode de recherche par criblage d'une substance destinée à inhiber l'adhésion cellulaire d'une cellule cancéreuse, la méthode comprenant l'utilisation d'une protéine comprenant une séquence d'acides aminés répondant à SEQ ID No: 2 pour identifier une substance qui inhibe l'activité de ladite protéine, inhibant ainsi l'adhésion cellulaire d'une cellule cancéreuse.

3. Méthode de recherche par criblage selon la revendication 2, où la protéine se présente sous la forme d'une cellule la produisant.

4. Méthode de recherche par criblage selon la revendication 2, où ledit cancer est une leucémie.

5. Méthode de recherche par criblage selon l'une quelconque des revendications 2 à 4, à utiliser dans le criblage d'une substance destinée au traitement prophylactique ou thérapeutique d'un cancer.
